(19) Europäisches Patentamt
European Patent Office
Office européen des brevets

(11) EP 4 759 230 A2

(12) **EUROPEAN PATENT APPLICATION**

(43) Date of publication:
17.06.2026 Bulletin 2026/25

(21) Application number: 26170733.5

(22) Date of filing: 22.02.2023

(51) International Patent Classification (IPC):
**A61B 5/107** *(2006.01)*

(52) Cooperative Patent Classification (CPC):
**G06T 7/0012; A61B 5/1075; A61B 5/4566;
G06T 7/11; G06T 7/62;** A61B 5/7264;
G06T 2207/10088; G06T 2207/20084;
G06T 2207/30012

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: 22.02.2022 US 202263312678 P

(62) Document number(s) of the earlier application(s) in
accordance with Art. 76 EPC:
**23712722.0 / 4 483 322**

(71) Applicant: **AUGMEDICS, INC.
Arlington Heights, IL 60005 (US)**

(72) Inventor: **Gawel, Dominik
Deerfield, Illinois 60015 (US)**

(74) Representative: **Kancelaria Eupatent.pl Sp. z o.o.
ul. Jana Kilinskiego 193 lok. 2.13
93-106 Lodz (PL)**

Remarks:
This application was filed on 03-04-2026 as a
divisional application to the application mentioned
under INID code 62.

(54) **SYSTEMS, DEVICES, AND METHODS FOR SPINE ANALYSIS**

(57) Embodiments include example systems, methods, and computer-accessible mediums for analysis of anatomical images for assessment of stenosis and disc degeneration. In some embodiments, systems, devices, and methods described herein include selecting a region of interest (ROI) in a three-dimensional (3D) volume of image data of a plurality of vertebra of a spine, identifying one or more anatomical parts in the ROI, determining one or more parameters of the one or more anatomical parts, and assessing a severity of a spinal deformity based on the one or more parameters.

EP 4 759 230 A2

## Description

CROSS-REFERENCE TO RELATED APPLICATIONS

This application claims priority to U.S. Provisional Application No. 63/312,678, entitled "SYSTEMS, DEVICES, AND METHODS FOR SPINE ANALYSIS," filed February 22, 2022, the entire disclosure of which is incorporated herein by reference.

TECHNICAL FIELD

The present disclosure relates generally to systems, devices, and methods for spine analysis, and specifically relates to analyzing anatomical image data for spinal deformity assessment.

BACKGROUND

A person's spinal column is a complex system of bones and soft tissue structures. The spine, which forms part of the spinal column, functions as the body's central support structure, and is composed of many individual bones known as vertebrae. Intervertebral discs are positioned between adjacent vertebrae to provide support and cushioning between the vertebrae. The vertebrae and intervertebral discs, together with other soft tissue structures (e.g., ligaments, nervous systems structures, etc.) in their vicinity, form the spinal column. Each intervertebral disc is made up of a dense series of collagen layers called annulus fibrosis and a hydrogel comprising proteoglycan and water called the nucleus. Dehydration of the intervertebral disc (e.g., dehydration of annulus fibrosis and/or dehydration of nucleus) due to age and other factors can lead to loss of structural and functional integrity of the intervertebral disc referred to as "disc degeneration." Nerve roots exit the spine through an opening between two adjacent vertebrae. The opening is called the intervertebral foramen. An abnormal narrowing of the intervertebral foramen can lead to spinal stenosis. Changes to tissues and/or bones can lead to other spinal deformities such as disc herniation, osteophyte formation, spondylolisthesis, etc.

Spinal deformities or diseases can take many forms. Two common ones are spinal stenosis and degenerative disc disease. Spinal stenosis involves a narrowing of the central canal of the spine. One example of spinal stenosis is degenerative lumbar spinal stenosis (DLSS). DLSS is a major cause of low back pain and is one of the most common indications for spinal surgery. In the working-age population, e.g., particularly in males between 40 and 50 years of age, DLSS has a high prevalence rate and can cause high medical and/or societal cost. Disc degeneration occurs as a result of changes in an individual's intervertebral discs. Degenerative disc disease can also lead to lower back pain, which affects young to middle-aged persons with peak incidence at approximately 40 years old. Disc degeneration can also lead to sciatica.

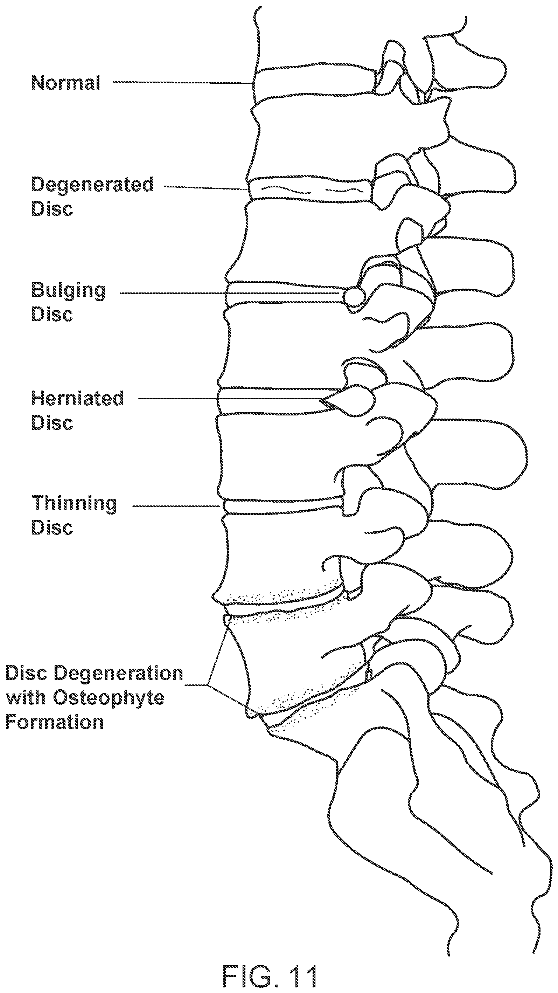

FIG. 11 depicts the progression of degenerative disc disease. Typically disc degeneration is a cascade of events originating from loss of hydration of the disc, which can lead to altered mechanical strains on the nucleus and annulus, and can result in a variety of consequences including: loss of disc height (e.g., disc narrowing or thinning), which can narrow the intervertebral foramen where the spinal nerve exits the spine; disc bulging or herniation, which also narrows the space for the spinal nerve; and other instabilities or deformities. When such instability occurs, the body may react to it by growing bone across the joint, in a process known as "autofusing." In particular, the body can grow bone spurs or osteophytes around the disc and/or facet joints. The growth of osteophytes can further complicate the negative effects of disc degeneration.

Spinal assessment can enable detection of various spinal conditions and/or spinal deformities. Detecting and treating spinal conditions and/or spinal deformities at an early stage (e.g., when the condition is mild) can prevent the conditions from becoming severe. When conducting spinal assessment, it can be important to identify the specific location, geometric limits, and other parameters within the spine. These parameters can be indicative of the type of spinal condition and/or spinal deformity and its location within the spine. Analyzing image data of a person's anatomy can help identify existing spinal conditions and/or spinal deformities. Some existing imaging systems include, for example, computed tomography (CT), magnetic resonance imaging (MRI), X-ray, ultrasound, and fluoroscopy systems. Such imaging is widely utilized for both initial diagnosis and follow up evaluation in both conservative and surgical treatment pathways.

Traditional X-ray and CT are common methods for acquiring information of patient anatomy, including, for example, a spine of the patient. Traditional X-rays involve directing high-energy electromagnetic radiation at a patient's body, and capturing a resulting two-dimensional (2D) X-ray profile on a film or plate. X-ray imaging, however, can subject patients to high levels of radiation. Analysis of X-rays can also be subjective based on physician training and experience. Currently, there is no autonomous way to objectively analyze X-rays. Accordingly, performing necessary measurement on X-rays requires time and can be subject to user error. Lack of autonomous methods of analyzing X-rays also makes it difficult to objectively compare a patient's X-rays over time, e.g., to track a patient's progress. Due to these limitations, it is not presently possible to reliably predict certain outcomes based on X-ray imaging. It is also not presently possible to obtain necessary measurements in an autonomous and/or consistent fashion that ensures reliability and reproducibility of such measurements.

CT involves using controlled amounts of X-ray radiation to obtain 3D image data of patient anatomy. Existing CT systems can include a rotating gantry that has an X-ray tube mounted on one side and an arc-shaped detector mounted on an opposite side. An X-ray beam can be emitted in a fan shape as the rotating frame spins the X-ray tube and detector around a patient. Each time the X-ray tube and detector make a 360° rotation and the X-ray passes through the patient's body, an image of a thin section of the patient anatomy can be acquired. During each rotation, the detector can record about 1,000 images or profiles of the expanded X-ray beam. Each profile can then be reconstructed by a dedicated computer into a 3D image of the section that was scanned. Accordingly, CT systems use a collection of multiple 2D CT scans or X-rays to construct a 3D image of the patient anatomy. The speed of gantry rotation, along with slice thickness, contributes to the accuracy and/or usefulness of the final image. Commonly used intraoperative CT imaging systems have a variety of settings that allow for control of the radiation dose. In certain scenarios, high dose settings may be chosen to ensure adequate visualization of the anatomical structures. The downside is increased radiation exposure to the patient. The effective doses from diagnostic CT procedures are typically estimated to be in the range of 1 to 10 millisieverts (mSv). Such high doses can lead to increased risk of cancer and other health conditions. Low dose settings are therefore selected for CT scans whenever possible to minimize radiation exposure and associated risk of cancer development. Low dose settings, however, may have an impact on the quality of the image data available for the surgeon.

MRI imaging systems operate by forming a strong magnetic field around an area to be imaged. In most medical applications, protons (e.g., hydrogen atoms) in tissues containing water molecules produce a signal that is processed to form an image of the body. First, energy from an oscillating magnetic field is temporarily applied to the patient at an appropriate resonance frequency. The excited hydrogen atoms emit a radio frequency (RF) signal, which is measured by a RF system. The RF signal may be made to encode position information by varying the main magnetic field using gradient coils. As these coils are rapidly switched on and off, they produce the characteristic repetitive noise of an MRI scan. Contrast between different tissues can be determined by the rate at which excited atoms return to their equilibrium state. In some instances, exogenous contrast agents may be given intravenously, orally, or intra-articularly, to further facilitate differentiation between different tissues. The major components of an MRI imaging system are the main magnet that polarizes tissue, the shim coils for correcting inhomogeneities in the main magnetic field, the gradient system for localizing the magnetic resonance (MR) signal, and the RF system that excites the tissue and detects the resulting signal. With MRI imaging, different magnetic field strengths can be used. The most common strengths are 0.3T, 1.5T and 3T. The higher the strength, the higher the image quality. For example, a 0.3T magnetic field strength will result in lower quality imaging then a 1.5T magnetic field strength.

Currently, there is also no autonomous way of objectively analyzing MRI images, with analysis of such images being reliant on physician training and experience. Moreover, due to technical limitations, diagnostic MRI protocols provide a limited number of slices of a target region, which leaves a physician to piece together anatomical information from available axial, sagittal, and/or coronal scans of the patient anatomy. Existing systems also lack a reliable way to easily and autonomously compare a patient's MRI images against a larger database of MRI images. Such comparison can allow a physician to obtain additional information about the severity of a patient's condition. Existing systems also lack the ability to autonomously compare a patient's MRI images at a present time against past images of that patient. In addition, it is not currently possible to screen a patient's MRI images for spinal cord compression, fracture, tumor, infection, among other conditions. Such limitations make it difficult if not impossible to make treatment recommendations based on patient MRI images that would result in a high degree of confidence in treatment outcome.

Even with these limitations, MRI is typically the imaging of choice for assessing spinal stenosis due to its ability to image soft tissue details. MRI also provides anatomical information that enables radiologists to identify the location, etiology and severity of the nerve root compression and report their findings. However, interpretation of MRIs of spinal anatomy can be time consuming, especially when advanced multi-level degeneration is present. While there exists imaging-based grading systems such as the Lee grading or Pfirrmann grading systems, the inter-reader variability, even among specialists, is high and degrades the perceived value of a reader's reporting. Therefore, a solution that provides an accurate and consistent interpretation of imaging data for stenosis, that can be applied at scale, has high clinical utility.

Similarly, for assessing disc pathology and degeneration, MRI is commonly used due to its lack of radiation, multiplanar imaging capability, high spinal soft tissue contrast, and precise localization of intervertebral discs changes. In some cases, antero-posterior (AP) and lateral views of X-rays can also be helpful in visualizing gross anatomic changes in the intervertebral disc. However, MRI is the standard imaging modality for detecting disc pathology because its aforementioned advantages present high clinical value in providing a clinician with information about disk degeneration.

With low quality images and lack of reliable and/or reproducible image analysis, existing systems pose a diagnostic challenge for physicians. Such limitations can make it difficult to adequately identify key landmarks and conduct measurements. This in turn can make it difficult to accurately conduct spinal assessment to detect spinal conditions and/or spinal deformities. Accordingly, additional systems, devices, and methods for spinal analysis to detect spinal conditions and/or spinal deformities may be desirable. In particular, a solution that provides an accurate and consistent interpretation of imaging data, especially MRI imaging data, is desirable.

SUMMARY OF DISCLOSURE

Systems, devices, and methods described herein relate to analysis of anatomical images and identification of anatomical components and/or structures. In some embodiments, systems, devices, and methods described herein relate to identification of spinal conditions and/or spinal deformities.

In some embodiments, a method includes: selecting a region of interest (ROI) in a three-dimensional (3D) volume of image data of a plurality of vertebra of a spine, the ROI including image data of one or more vertebra from the plurality of vertebra and tissue structures surrounding the one or more vertebra; identifying a spinal cord or thecal sac in the ROI; determining one or more parameters associated with the spinal cord or thecal sac in the ROI; and determining a severity of spinal stenosis in the ROI based on the one or more parameters associated with the spinal cord or thecal sac.

In some embodiments, an apparatus includes: a memory; and a processor operatively coupled to the memory, the processor configured to: select a region of interest (ROI) in a three-dimensional (3D) volume of image data of a plurality of vertebra of a spine, the ROI including image data of one or more vertebra from the plurality of vertebra and tissue structures surrounding the one or more vertebra; identify a spinal cord or thecal sac in the ROI; determine one or more parameters associated with the spinal cord or thecal sac in the ROI; and determine a severity of spinal stenosis in the ROI based on the one or more parameters associated with the spinal cord or thecal sac.

In some embodiments, a non-transitory processor-readable medium storing code representing instructions to be executed by a processor, the code comprising code to cause the processor to: select a region of interest (ROI) in a three-dimensional (3D) volume of image data of a plurality of vertebra of a spine, the ROI including image data of one or more vertebra from the plurality of vertebra and tissue structures surrounding the one or more vertebra; identify a spinal cord or thecal sac in the ROI; determine one or more parameters associated with the spinal cord or thecal sac in the ROI; and determine a severity of spinal stenosis in the ROI based on the one or more parameters associated with the spinal cord or thecal sac.

In some embodiments, a method includes: selecting a region of interest (ROI) in a three-dimensional (3D) volume of image data of a plurality of vertebra of a spine, the ROI including image data of one or more vertebra from the plurality of vertebra and tissue structures surrounding the one or more vertebra; identifying one or more nerve roots in the ROI; tracking the one or more nerve roots from a lateral recess to a vertebral foramen of the one or more vertebra to identify one or more regions including a discontinuity in a nerve root or a narrowed section of a nerve root; and determining a severity or a location of spinal stenosis in the ROI based on the one or more regions.

In some embodiments, an apparatus includes: a memory; and a processor operatively coupled to the memory, the process configured to: select a region of interest (ROI) in a three-dimensional (3D) volume of image data of a plurality of vertebra of a spine, the ROI including image data of one or more vertebra from the plurality of vertebra and tissue structures surrounding the one or more vertebra; identify one or more nerve roots in the ROI; track the one or more nerve roots from a lateral recess to a vertebral foramen of the one or more vertebra to identify one or more regions including a discontinuity in a nerve root or a narrowed section of a nerve root; and determine a severity or a location of spinal stenosis in the ROI based on the one or more regions.

In some embodiments, a non-transitory processor-readable medium storing code representing instructions to be executed by a processor, the code comprising code to cause the processor to: select a region of interest (ROI) in a three-dimensional (3D) volume of image data of a plurality of vertebra of a spine, the ROI including image data of one or more vertebra from the plurality of vertebra and tissue structures surrounding the one or more vertebra; identify one or more nerve roots in the ROI; track the one or more nerve roots from a lateral recess to a vertebral foramen of the one or more vertebra to identify one or more regions including a discontinuity in a nerve root or a narrowed section of a nerve root; and determine a severity or a location of spinal stenosis in the ROI based on the one or more regions.

In some embodiments, a method includes: selecting a region of interest (ROI) in a three-dimensional (3D) volume of image data of a plurality of vertebra of a spine, the ROI including image data of one or more vertebra from the plurality of vertebra and tissue structures surrounding the one or more vertebra; identifying an intervertebral disc in the ROI; determining one or more parameters of an annulus and a nucleus of the intervertebral disc; and determining a disc degeneration ratio based on the one or more parameters of the annulus and the nucleus of the intervertebral disc.

In some embodiments, an apparatus includes: a memory; and a processor operatively coupled to the memory, the process configured to: select a region of interest (ROI) in a three-dimensional (3D) volume of image data of a plurality of vertebra of a spine, the ROI including image data of one or more vertebra from the plurality of vertebra and tissue structures surrounding the one or more vertebra; identify an intervertebral disc in the ROI; determine one or more parameters of an annulus and a nucleus of the intervertebral disc; and determine a disc degeneration ratio based on the one or more parameters of the annulus and the nucleus of the intervertebral disc.

In some embodiments, a non-transitory processor-readable medium storing code representing instructions to be executed by a processor, the code comprising code to cause the processor to: select a region of interest (ROI) in a three-dimensional (3D) volume of image data of a plurality of vertebra of a spine, the ROI including image data of one or more vertebra from the plurality of vertebra and tissue structures surrounding the one or more vertebra; identify an intervertebral disc in the ROI; determine one or more parameters of an annulus and a nucleus of the intervertebral disc; and determine a disc degeneration ratio based on the one or more parameters of the annulus and the nucleus of the intervertebral disc.

BRIEF DESCRIPTION OF THE DRAWINGS

FIG. 1 is a block diagram illustrating a configuration of a system for collecting and analyzing anatomical images, according to some embodiments.

FIG. 2 schematically illustrates an example compute device for spinal deformity diagnosis and/or spinal deformity assessment, according to some embodiments.

FIG. 3A is a schematic illustrating a segmentation convolutional neural network, according to some embodiments.

FIG. 3B is a schematic illustrating an example model for performing level identification of spinal anatomy, according to some embodiments.

FIG. 4A is a flow chart illustrating a method of training and validating a model for performing spinal deformity assessment, according to some embodiments.

FIG. 4B is a flow chart illustrating training of a neural network training process for performing spinal deformity assessment, according to some embodiments.

FIG. 5 is a flow chart illustrating spinal deformity assessment, according to some embodiments.

FIG. 6A is a flow chart of a level identification process, according to some embodiments.

FIG. 6B is a flow chart of a spinal deformity assessment process, according to some embodiments.

FIG. 7A is a flow chart of a stenosis assessment process, according to some embodiments.

FIG. 7B is a flow chart of a disc degeneration assessment process, according to some embodiments.

FIG. 8 illustrates an example report generated by systems and methods described herein, according to some embodiments.

FIGS. 9A-9E depict an example report generated for a patient with mild stenosis.

FIG. 10A illustrates an example diagnosis of severe stenosis in a patient.

FIG. 10B illustrates an example diagnosis of no stenosis in a patient.

FIG. 11 is an illustration of the progression of disc degeneration of the spine.

FIG. 12 is a flow chart of a level identification process, according to some embodiments.

FIG. 13A visually depicts the flow of identifying and assigning levels to different MRI imaging sets, according to some embodiments.

FIG. 13B is a schematic visualization of combining MRI sagittal data with axial data, according to embodiments.

FIG. 14A is an illustration of the spine under normal conditions and with spinal stenosis.

FIG. 14B is an illustration of the different classifications of lumbar central canal stenosis based on the grading system developed by Lee et al. ("Lee grading system").

FIG. 15 is a flow chart of a stenosis assessment process, according to some embodiments.

FIG. 16 schematically illustrates a spinal stenosis analysis report, according to some embodiments.

FIGS. 17A-17D provide an example of a spinal stenosis analysis report, according to embodiments.

FIGS. 18A and 18B provide detailed views of graphs that correlate thecal sac surface area with different classifications or grades of spinal stenosis, according to embodiments.

FIG. 19 is an illustration of an intervertebral disc under normal conditions and with disc degeneration.

FIG. 20A is an illustration of the different classifications of intervertebral disc degeneration based on the grading system developed by Pfirrmann et al. ("Pfirrmann grading system").

FIG. 20B is a flow depicting how the different classifications of the Pfirrmann grading system are assigned.

FIG. 21 is a flow chart of a disc degeneration assessment process, according to some embodiments.

FIG. 22 schematically illustrates a disc degeneration analysis report, according to some embodiments.

FIG. 23 provides an example of a disc degeneration analysis report, according to embodiments.

DETAILED DESCRIPTION

1. Overview of System

Systems, devices, and methods described herein relate to processing of patient anatomical structures, including a spine. While certain examples presented herein may generally relate to processing of image data of a spine, it can be appreciated by one of ordinary skill in the art that such systems, devices, and methods can be used to process image data of other portions of patient anatomy, including, for example, vessels, nerves, bone, and other soft and hard tissues near the brain, heart, or other regions of a patient's anatomy.

Systems, devices, and methods described herein can be suited for processing several different types of image data, including X-ray, computer tomography (CT), magnetic resonance imaging (MRI), fluoroscopic, ultrasound, etc. In some embodiments, such systems, devices, and methods can process a single image type and/or view, while in other embodiments, such systems, devices, and methods can process multiple image types and/or views. In some embodiments, multiple image types and/or views can be combined to provide richer data regarding a patient's anatomy.

Systems, devices, and methods described herein can implement machine learning models to process and/or analyze image data regarding a patient's anatomy. Such machine learning models can be configured to identify and differentiate between different anatomical parts within anatomical structures. In some embodiments, machine learning models described herein can include neural networks, including deep neural networks with multiple layers between input and output layers. For example, one or more convolutional neural networks (CNNs) can be used to process patient image data and produce segmentation outputs that classify different objects within the image data and/or identify different levels of the spine in the image data. Suitable examples of segmentation models and the use thereof are described in U.S. Patent Application Publication No. 2019/0105009, published November 11, 2019, titled "Automated Segmentation Of Three Dimensional Bony Structure Images," U.S. Patent Application Publication No. 2020/0151507, published May 14, 2020, titled "Autonomous Segmentation Of Three-Dimensional Nervous System Structures From Medical Images," U.S. Patent Application Publication No. 2020/0410687, published December 31, 2020, titled "Autonomous Multidimensional Segmentation Of Anatomical Structures On Three-Dimensional Medical Imaging," U.S. Provisional Patent Application No. 63/187,777, filed May 12, 2021, titled "Systems, Devices, and Methods for Segmentation of Anatomical Image Data," and PCT Patent Application No. PCT/US22/29000, filed May 12, 2022, titled "Systems, Devices, and Methods for Segmentation of Anatomical Image Data," the disclosures of each of which are incorporated herein by reference. Suitable examples of methods of level identification are described in U.S. Patent Publication No. 2020/0327721, published October 15, 2020, titled "Autonomous Level Identification of Anatomical Bony Structures on 3D Medical Imagery," and U.S. Provisional Patent Application No. 63/256,306, filed October 15, 2021, titled "Level Identification of Three-Dimensional Anatomical Images," the disclosures of each of which are incorporated herein by reference. While certain examples described herein employ CNNs, it can be appreciated that other types of machine learning algorithms can be used to process patient image data, including, for example, support vector machines (SVMs), decision trees, k-nearest neighbor, and artificial neural networks (ANNs).

In some embodiments, systems, devices, and methods described herein can implement machine learning models (e.g., spinal analysis model(s) as further described herein) to perform anatomical feature analysis and/or to perform spinal deformity diagnosis or classification. For example, systems, devices, and methods described herein can identify and differentiate between different anatomical parts within anatomical structures, identify different levels of the spine, analyze anatomical features (e.g., vertebral disc, shape and/or size of vertebral disc, distance between adjacent vertebrae, etc.), perform spinal deformity assessment based on the analysis of anatomical features, associate the assessment with identified level of the spine, and/or provide report(s) relating to anatomical feature analyses or spinal deformity diagnoses.

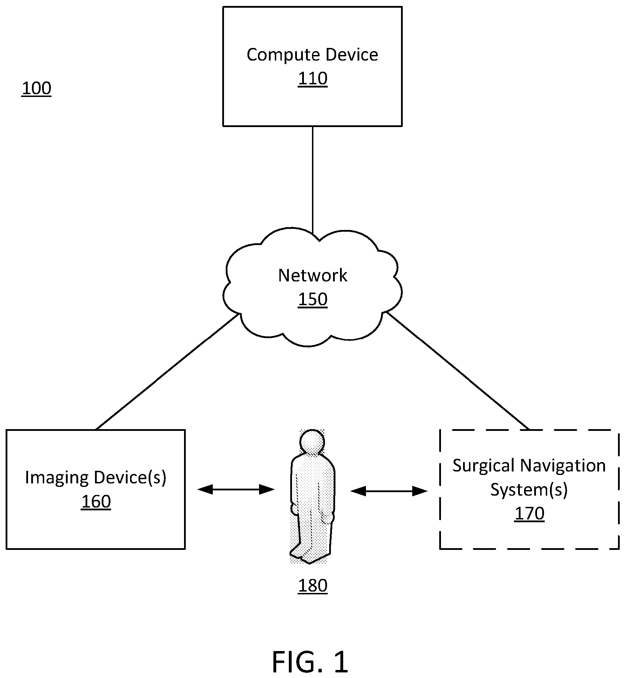

FIG. 1 is a high-level block diagram that illustrates a system 100 for processing image data of patient anatomy and/or providing image guidance to physicians during a surgical procedure, according to some embodiments. The system 100 can include a compute device 110, an imaging device(s) 160, and, optionally, surgical navigation system(s) 170. In some embodiments, the compute device 110 can communicate with one or more imaging device(s) 160 and optionally one or more surgical navigation system(s) 170, e.g., to perform segmentation of patient anatomical structures, to perform level identification of patient anatomical structures, to perform spine analysis, to detect spinal conditions and/or other spinal deformities, and/or to provide digital guidance to surgeons during surgical procedures.

In some embodiments, the compute device 110 may be configured to perform segmentation of anatomical image data to identify anatomical parts of interest. For example, the compute device 110 can be configured to generate segmentation outputs that identify different anatomical parts of interest. Additionally or alternatively, the compute device 110 may be configured to perform level identification of different regions of the spine. The compute device 110 can be configured to generate level identification outputs, such as, for example, a level type (e.g., sacrum, thoracic, lumbar, cervical), a vertebral level (ordinal identifier), or a pair or range of vertebral levels associated with a vertebrae (and/or other nearby anatomical part(s)). Additionally or alternatively, the compute device 110 can be configured to perform anatomical feature analysis and/or spinal deformity assessment for the various identified levels. For example, the compute device 110 can be configured to analyze the anatomical parts of interest to determine spinal conditions and/or spinal deformities. In some embodiments, the compute device 110 can be configured to associate the determined spinal conditions and/or spinal deformities to one or more levels of the spine, e.g., based on the level identification outputs. In some embodiments, the compute device can be configured to generate visual representations of the patient's anatomy, and associate level identification information, anatomical feature information, and/or spinal deformity diagnoses to different parts of the patient's anatomy. In some embodiments, the compute device 110 can be configured to generate virtual representations of patient anatomy and/or surgical instruments, e.g., to provide image guides to surgeons before and/or during surgical procedures.

The compute device 110 may be implemented as a single compute device, or be implemented across multiple compute devices that are connected to each other and/or the network 150. For example, the compute device 110 may include one or more compute devices such as servers, desktop computers, laptop computers, portable devices, databases, etc. Different compute device may include component(s) that are remotely situated from other compute devices, located on premises near other compute devices, and/or integrated together with other compute devices.

In some embodiments, the compute device 110 can be located on a server that is remotely situated from one or more imaging device(s) 160 and/or surgical navigation system(s) 170. For example, an imaging device 160 and a surgical navigation system 170 can be located in a surgical operating room with a patient 180, while the compute device 110 can be located at a remote location but be operatively coupled (e.g., via network 150) to the imaging device 160 and the surgical navigation system 170. In some embodiments, the compute device 110 can be integrated into one or both of the imaging device 160 and the surgical navigation system 170. In some embodiments, system 100 includes a single device that includes the functionality of the compute device 110, one or more imaging device(s) 160, and one or more surgical navigation system(s) 170, as further described herein.

In some embodiments, the compute device 110 can be located within a hospital or medical facility. The compute device 110 can be operatively coupled to one or more databases associated with the hospital, e.g., a hospital database for storing patient information, etc. In some embodiments, the compute device 110 can be available to physicians (e.g., surgeons) for performing evaluation of patient anatomical data (including, for example, level data as described herein), visualization of patient anatomical data, diagnoses, and/or planning of surgical procedures. In some embodiments, the compute device 110 can be operatively coupled to one or more other compute devices within a hospital (e.g., a physician workstation), and can send level outputs and/or other image processing outputs to such compute devices (e.g., via network 150) for performing evaluation of patient anatomical data, visualization of patient anatomical data, diagnoses, and/or planning of surgical procedures.

Network 150 may be any type of network (e.g., a local area network (LAN), a wide area network (WAN), a virtual network, a telecommunications network) implemented as a wired network and/or wireless network and used to operatively couple compute devices, including system 100. As shown in FIG. 1, a connection may be defined between compute device 110 and any one of imaging device(s) 160, surgical navigation system(s) 170, and/or other compute devices (e.g., databases, servers, etc.). In some embodiments, the compute device 110 may communicate with imaging device(s) 160 and/or surgical navigation system(s) 170 (e.g., send data to and/or receive data from such devices) and with the network 150 via intermediate networks and/or alternate networks (not shown in FIG. 1). Such intermediate networks and/or alternate networks may be of a same type and/or a different type of network as network 150. Each of the compute device 110, imaging device(s) 160, and surgical navigation system(s) 170 may be any type of device configured to send data over the network 150 to send and/or receive data from one or more of the other devices.

In some embodiments, an imaging device 160 may refer to any device configured to image anatomical structures of a patient 180. In some embodiments, the imaging device 160 may include one or more sensors for measuring signals produced by various imaging technologies. The imaging device 160 can employ a non-invasive technology to image a patient's anatomy. Nonlimiting examples of imaging devices include CT scanners, MRI scanners, X-ray devices, ultrasound devices, and combinations thereof, and the like. The image data generated by the imaging device 160 may be transmitted to any of the devices connected to network 150, including, for example, compute device 110. In some embodiments, the image data generated by the imaging device 160 can include a 2D image of an anatomical structure. In some embodiments, the image data generated by the imaging device 160 can include a plurality of 2D image scans that together provide image data for a 3D volume. The imaging device 160 can transmit the image data to the compute device 110 such that the compute device 110 can perform segmentation of the patient anatomy, perform level identification of the patient anatomy, label different anatomical parts of interest in the patient anatomy, perform spinal assessment (e.g., spinal deformity diagnosis), detect spinal conditions and/or spinal deformities, and/or associate spinal deformity diagnosis to level identification information. Optionally, the imaging device 160 can provide the image data to a surgical navigation system 170 such that the surgical navigation system can generate one or more virtual representations of the patient anatomy, e.g., for use in image-guided surgery.

The surgical navigation system 170 can be configured to provide image-guided surgery, e.g., during a surgical operation. For example, the surgical navigation system 170 may facilitate one or more of planning, visualization, and guidance during a surgical procedure. In some embodiments, the surgical navigation system 170 can include a tracking system for tracking patient anatomy, surgical tool(s), implant(s), or other objects within a surgical field. In some embodiments, the surgical navigation system 170 can include an image generator for generating one or more virtual representations of patient anatomy and/or surgical tool(s), implant(s), or other objects within a surgical field and to display these to a physician or other healthcare provider (e.g., a surgeon). In some embodiments, the surgical navigation system 170 can be configured to present a 3D display, e.g., via a 3D wearable device and/or a 3D projector or screen. In some embodiments, the surgical navigation system 170 can be configured to display a position and/or orientation of one or more surgical instrument(s) and implant(s) with respect to presurgical or intraoperative medical image data of the patient anatomy. The image data can be provided, for example, by an imaging device 160, and the surgical navigation system 170 can use the image data to generate a virtual representation of one or more anatomical parts of interest along with position and/or orientation data associated with a surgical device. Suitable examples of surgical navigation systems are described in U.S. Patent Application Publication No. 2019/0053851, published February 21, 2019, and incorporated herein by reference.

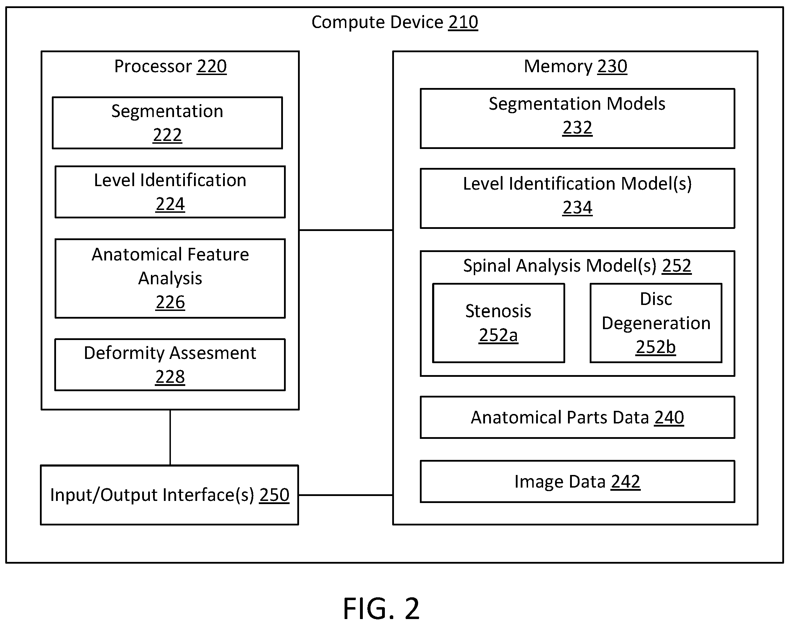

FIG. 2 schematically illustrates an example compute device 210 for surgical planning and/or navigation, according to some embodiments. Compute device 210 can be structurally and/or functionally similar to compute device 110. While a single compute device 210 is schematically depicted, it can be appreciated that the compute device 210 can be implemented as one or more compute devices. In some embodiments, compute device 210 may be configured to segment patient anatomy, identify levels of spinal anatomy of a patient (e.g., patient 180), and/or perform anatomical feature analysis and/or spinal deformity analysis. Compute device 210 includes a processor 220, a memory 230, and one or more input/output interface(s) 250.

Memory 230 may be, for example, a random access memory (RAM), a memory buffer, a hard drive, a database, an erasable programmable read-only memory (EPROM), an electrically erasable read-only memory (EEPROM), a read-only memory (ROM), and/or so forth. In some embodiments, memory 230 stores instructions that cause processor 220 to execute modules, processes, and/or functions associated with segmentation 222, level identification 224, anatomical feature analysis 226, and deformity assessment 228. Memory 230 can store one or more segmentation models 232, level identification model(s) 234, spinal analysis model(s) 252 (e.g., on stenosis assessment 252a, on disc degeneration assessment 252b), anatomical parts data 240, and/or image data 242.

The segmentation models 232 can be models or algorithms for performing image-based segmentation, whereby different portions of anatomical image data can be classified or labeled. In some embodiments, the segmentation models 232 can include machine learning models, such as, for example, a CNN model, a SVM model, etc. The segmentation models 232 can be implemented by the processor 220 to perform segmentation 222. In some embodiments, the segmentation models 232 can be unique to particular anatomical regions, e.g., spinal anatomy, cardiac anatomy, etc. In some embodiments, the segmentation models 232 can be unique to particular image types, e.g., X-ray, CT, MRI, etc.

The level identification models 234 can be models or algorithms for identifying and/or labeling different levels of the vertebrae of the spine and/or other anatomical parts associated with those levels (e.g., nerves, intervertebral discs, etc.). In some embodiments, the level identification models 234 can include machine learning models, such as, for example, a CNN model, a SVM model, etc. The level identification models 234 can be implemented by the processor 220 to perform level identification 224. In some embodiments, the level identification models 234 can be unique to particular image types (e.g., X-ray, CT, MRI) and/or image views. For example, the level identification models 234 can include different models for identifying levels in axial image data, sagittal image data, and/or coronal image data. Alternatively, the level identification models 234 can include models for evaluating volumetric image data and/or combined image data, such as, for example, image data from multiple imaging systems and/or combined axial, sagittal, and/or coronal image data.

The spinal analysis model 252 can be models or algorithms for analyzing anatomical features and/or performing spinal deformity assessment or diagnoses. In some embodiments, the spinal analysis model 252 can associate spinal deformity assessment information (e.g., output of the spinal analysis model 252) with particular levels of the spine (e.g., output of the level identification models 234) and/or particular anatomical parts or structures (e.g., output of the segmentation models 232). In some embodiments, the spinal analysis model(s) 252 can include machine learning models, such as, for example, a CNN model, a SVM model, etc. The spinal analysis model(s) 252 can be implemented by the processor 220 to perform anatomical feature analysis 226 and/or deformity assessment 228. In some embodiments, the spinal analysis model(s) 252 can be unique to the type of spinal condition and/or spinal deformity being assessed. For example, the spinal analysis model(s) 252 can include a stenosis model 252a for stenosis assessment and/or a disc degeneration model 252b for disc degeneration assessment.

The anatomical parts data 240 can include information relating to anatomical parts of a patient. For example, the anatomical parts data 240 can include information identifying, characterizing, and/or quantifying different features of one or more anatomical part(s), such as, for example, a location, color, shape, geometry, or other aspect of an anatomical part. The anatomical parts data 240 can provide general or patient-specific information on different anatomical parts to enable processor 220 to perform segmentation 222, level identification 224, anatomical feature analysis 226, and/or deformity assessment 228 based on patient image data. The image data 242 can include image data associated with one or more patient(s) and/or information about different image devices, e.g., different settings of different image devices (e.g., image device(s) 160) and how those settings may impact images captured using those devices.

The processor 220 may be any suitable processing device configured to run and/or execute any of the functions described herein. In some embodiments, processor 220 may be a general purpose processor, a Field Programmable Gate Array (FPGA), an Application Specific Integrated Circuit (ASIC), a Digital Signal Processor (DSP), a Dedicated Graphics Processing Unit (GPU), and/or the like. In some embodiments, the processor 220 can be configured to perform one or more of segmentation 222, level identification 224, anatomical feature analysis 226, and/or deformity assessment 228. Segmentation 222, level identification 224, anatomical feature analysis 226, and deformity assessment 228 can be implemented as one or more programs and/or applications that are tied to hardware components (e.g., processor 220, memory 230, input/output interface(s) 250). In some embodiments, a system bus (not shown) may be configured to enable processor 220, memory 230, input/output interface(s) 250, and/or other components of the compute device 210 to communicate with each other.

While a single processor 220 that is located on a single compute device 210 is depicted in FIG. 2, it can be appreciated that processor 220 can be one or more processors that are located on the same compute device and/or different compute devices. In some embodiments, systems, devices, and methods described herein, including, for example, anatomical feature analyses and/or deformity assessments, can be implemented in a cloud platform (e.g., using one or more remote compute devices). The cloud platform can be connected to one or more databases, such as, for example, hospital databases, via a network (e.g., network 150). As such, systems, devices, and methods described herein can receive information from those databases (e.g., patient information, imaging data, etc.) and/or send information to those databases (e.g., spinal analysis reports, visualization of patient anatomy, etc.).

The input/output interface(s) 250 may include one or more components that are configured to receive inputs and send outputs to other devices (e.g., imaging device(s) 160, surgical navigation system(s) 170, etc.). In some embodiments, the input/output interface(s) 250 can include a user interface, which can include one or more components that are configured to receive input and/or present output to a user. For example, input/output interface 250 may include a display device (e.g., a display, a touch screen, etc.), an audio device (e.g., a microphone, a speaker), a keypad, and/or other interfaces for receiving information from and/or presenting information to users. In some embodiments, the input/output interface 250 can include a communications interface for communicating with other devices, and can include conventional electronics for data communication using a standard communication protocol, e.g., Wi-Fi, Bluetooth®, etc.

2. Overview of Methods

Systems, devices, and methods described herein can perform spinal deformity analyses (e.g., stenosis assessment, disc degeneration assessment, and/or other deformities assessment). In some embodiments, systems, devices, and methods can perform such analyses in combination with segmentation and/or level identification. As described above, a compute device (e.g., compute devices 110, 210) for performing segmentation, level identification, and/or spinal analysis can implement one or more algorithms or models. In some embodiments, the algorithms or models can include machine learning models, which can be trained using labeled training datasets. The machine learning models can use the training datasets to learn relationships between different features in the image data and the output spinal deformity.

In some embodiments, systems, devices, and methods described herein can perform pre-processing of image data, e.g., prior to performing segmentation, level identification, spinal feature analysis, and/or spinal deformity assessments. In many instances, image data collected using conventional imaging techniques can have low quality. For example, to avoid the risks of exposing patients to high levels of radiation, a CT imaging device may be used on a lower dose setting to capture images of patient anatomy. Similarly, MRI imaging devices using lower power may be used to capture images of patient anatomy. Such low dose or low power images can have images that have a higher amount of noise. A compute device (e.g., compute devices 110, 210) as described herein can optionally pre-process the image to remove such noise prior to performing segmentation, spinal level identification, anatomical feature analysis, and/or spinal deformity assessment.

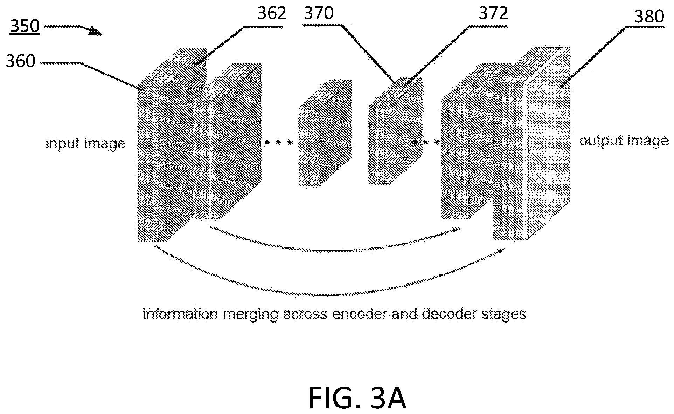

FIG. 3A schematically depicts an architecture for a CNN model 350, which can be utilized for performing segmentation (e.g., both semantic and binary). The CNN model 350 can be an example of a level identification model, such as, for example, segmentation model 232 described with reference to FIG. 2. The architecture of the CNN model 350 can be fully convolutional and have layer skip connections. The CNN model 350 can perform pixel-wise class assignment using an encoder-decoder architecture. The CNN model 350 can take as the input the raw images generated by an imaging device (e.g., imaging device 160) and/or the images generated by the imaging device after denoising for example, with another CNN model for denoising. The left side of the CNN model 350 is a contracting path (encoder), which includes one or more convolution layers 360 and/or pooling layers 362. One or more images (e.g., raw images or denoised images) can be presented to the input layer of the CNN model 350, and the CNN model 350 via the series of convolution layers 360 and/or pooling layers 362 can extract features from the image data. The right side of the CNN model 350 is an expanding path (decoder), which includes upsampling or transpose convolution layers 370 and convolution layers 372, which results in an output layer 380.

In some embodiments, the CNN model 350 can be used to perform segmentation of patient spinal anatomy. For example, the CNN model 350 can be configured to classify portions of images (e.g., each pixel or groupings of pixels) into different classes, e.g., bone and not bone, or bone, nerve, vertebral body, pedicles, processes, etc. In some embodiments, a first CNN model can be configured to perform a first classification (e.g., bone and not bone), and the output of that first CNN model 350 can be combined and inputted into one or more additional CNN models 350 that are configured to perform one or more additional classifications (e.g., nerve or not nerve, intervertebral disc or not intervertebral disc, etc.). In some embodiments, the CNN model 350 can be trained to segment patient anatomy using a training dataset including images with labeled anatomical parts.

Further details of CNN models 350 configured for performing segmentation are described in U.S. Patent Application Publication No. 2019/0105009, U.S. Patent Application Publication No. 2020/0151507, U.S. Patent Application Publication No. 2020/0410687, U.S. Provisional Patent Application No. 63/187,777, and PCT Patent Application No. PCT/US22/29000, incorporated above by reference.

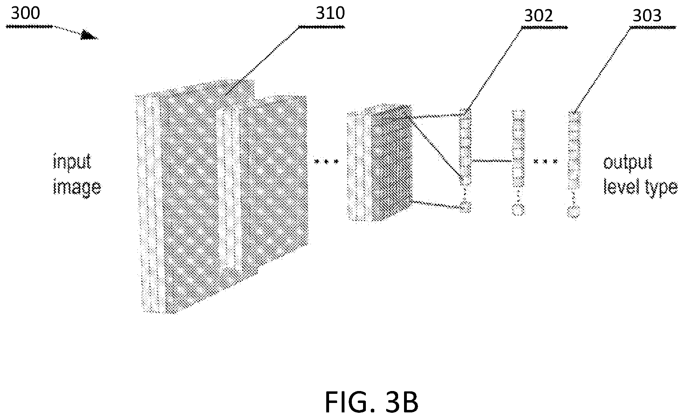

FIG. 3B is a schematic illustrating a CNN model 300 for performing level identification of spinal anatomy, according to embodiments. The CNN model 300 can be an example of a level identification model, such as, for example, level identification model 234 described with reference to FIG. 2. The CNN model 300 may be configured to perform vertebral level prediction on image data, including, for example, 2D scans (e.g., DICOM slices) of anatomical structures. In some embodiments, the CNN 300 can be configured to return one or more probability maps that identify for each vertebral level class (e.g., thoracic, sacrum, lumbar, cervical), a probability that an anatomical image (or portion of the image, e.g., pixel or group of pixels) belongs to that particular class. The probabilities associated with the different classes can sum to one, such that a class having the largest probability is indicative of the most likely class to which the image (or portion of the image) belongs.

In some embodiments, the input to the CNN model 300 may be a contracting path (encoder) and includes a plurality of stacked convolution blocks 310 including one or more convolution layers and/or pooling layers. One or more images (e.g., raw images or denoised images) can be presented to the input layer of the CNN model 300, and the CNN model 300 via the series of convolution layers and/or pooling layers can extract features from the image data. The image data can include a single image (e.g., an X-ray image or a single image scan) or a set of images of 2D scans that together form a local volume representation. In some embodiments, the last convolution block 310 may be directly connected to a plurality of dense, fully-connected layers 302 that are stacked together. The last fully-connected layer 302 may be considered a network output layer that corresponds to all possible outputs. For example, the possible outputs can include all vertebral type classes (e.g., cervical, thoracic, lumbar, sacrum).

As described above, the CNN model 300 can be used to perform level identification of spinal anatomy. For example, the CNN model 300 can be configured to classify portions of images (e.g., each pixel or groupings of pixels) into different level type classes, e.g., thoracic, sacrum, lumbar, and/or cervical. In some embodiments, the CNN model 300 can be configured to classify portions of images into different vertebral level (ordinal identifier) classes, e.g., thoracic levels 1-12 (TH1-TH12), lumbar levels 1-5 (L1-L5), sacral levels 1-5 (S1-S5), and/or cervical levels 1-8 (C1-C8). In some embodiments, a first CNN model can be configured to perform a first classification (e.g., vertebral level type), and the output of that first CNN model can be combined and inputted into one or more additional CNN models that are configured to perform one or more additional classifications (e.g., ordinal identifier). In some embodiments, the CNN model 300 can be configured to classify images by identifying a pair of spine levels (e.g., L1/L2, C6/C7, etc.) or a range of spine levels (e.g., C5-T7, L1-L4, etc.). As described above, the CNN model 300 can be trained to identify patient anatomy using a training dataset including images with labeled anatomical parts.

Further details of CNN models 300 configured for performing level identification are described in U.S. Patent Publication No. 2020/0327721 and U.S. Provisional Patent Application No. 63/256,306, incorporated above by reference.

A spinal analysis model can be used to measure or determine information about one or more anatomical features and/or to perform a spinal deformity assessment, such as described with reference to FIG. 2. In some embodiments, a spinal analysis model can be implemented as a CNN model and/or other machine learning algorithm. Put differently, a CNN model can be an example of spinal analysis model, such as for example, spinal analysis model 252 described with reference to FIG. 2. In some embodiments, the CNN model can perform anatomical feature analysis in response to receiving image data, including, for example, 2D scans and/or 3D volumetric image data of anatomical structures. In some embodiments, the CNN model can be configured to receive and/or extract from image data information relating to a region of interest (e.g., parameters, characteristics, or other information of anatomical features such as vertebrae, intervertebral disc, intervertebral foramen, nerves, etc.). For example, the CNN model can be configured to receive or extract information including shape of an anatomical feature, surface area of an anatomical feature, distance between two adjacent anatomical features, a combination thereof, and/or the like. The CNN model can be configured to classify one or more anatomical parts and/or structures based at least in part on the analysis of the anatomical feature information (e.g., shape, surface area, distance, etc.). In some embodiments, the CNN model can be configured to assess a state or condition of one or more anatomical parts and/or structures based on the anatomical feature information. For example, the CNN can be configured to classify one or more vertebral levels as having mild spinal deformity, medium spinal deformity, severe spinal deformity, no spinal deformity, etc. based on the anatomical feature information.

While segmentation models, level identification models, and spinal analysis models are described separately in this section, it can be appreciated that one or more models for performing segmentation, level identification, and/or spinal analysis can be used together, e.g., in a model or algorithm that combines multiple such processes (segmentation, level identification, and/or spinal analysis) together. For example, in some embodiments, a model can be configured to receive image data, to perform segmentation on that image data, to identify different levels of vertebrae in the image data, to perform analysis of different spinal deformities, and to output information on one or more anatomical parts and/or structures.

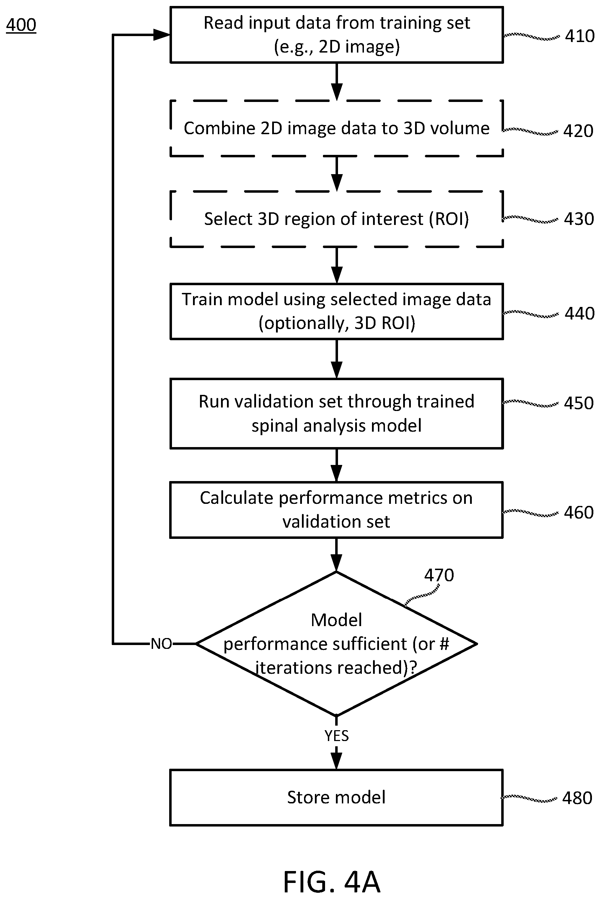

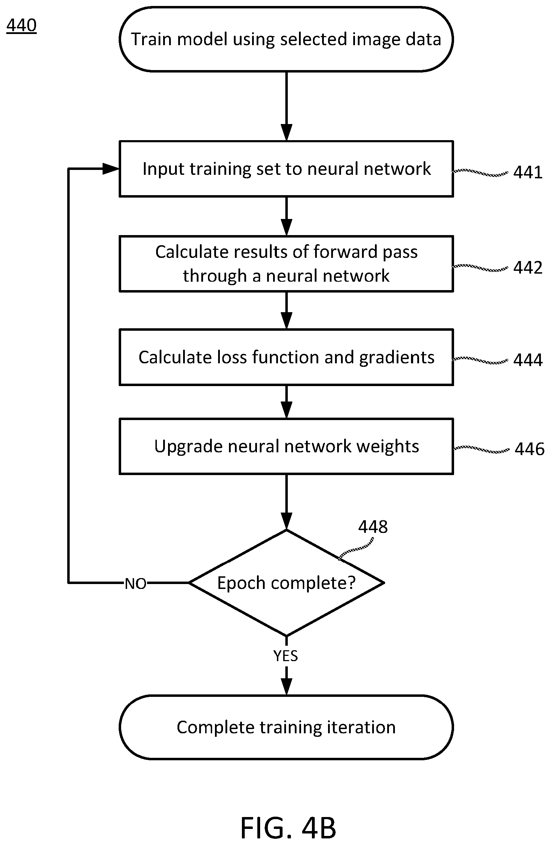

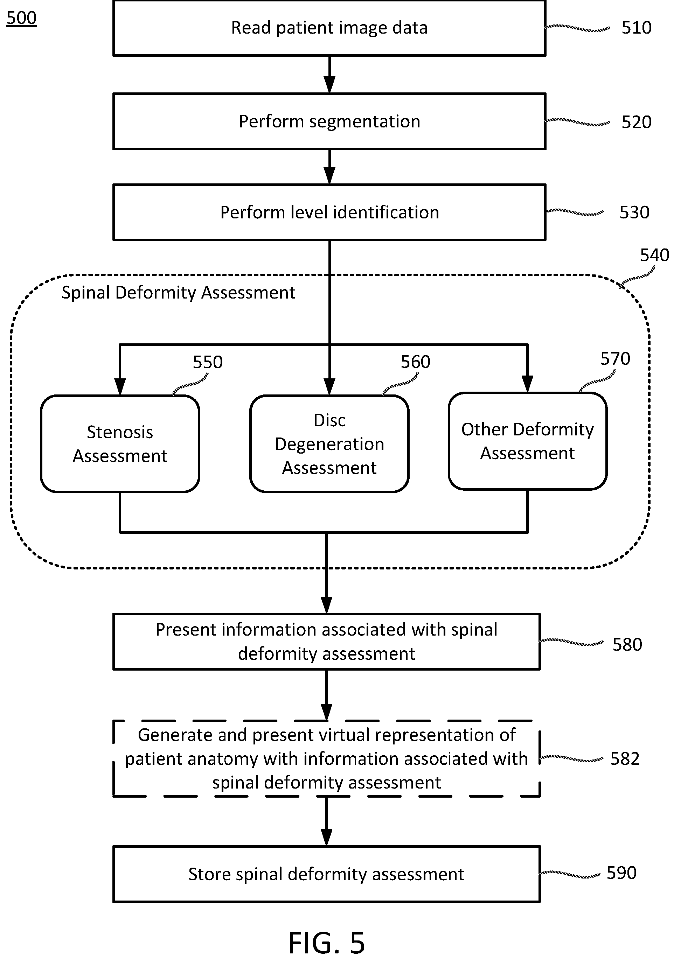

Further details of training various models are discussed with reference to flow diagrams depicted in FIGS. 4A and 4B. Further details of implementing models for performing anatomical feature analysis and/or spinal deformity assessments are discussed with reference to flow diagrams depicted in FIGS. 5-7B. The methods depicted in these flow diagrams can be implemented by one or more devices as described with reference to FIGS. 1 and 2, including, for example, compute devices 110, 210.

### 3. Training of Models for Spinal Analysis

In some embodiments, systems, devices, and methods described herein can perform spinal analysis using a model trained to analyze and/or diagnose a condition or deformity associated with a patient's spinal column. For example, systems, devices, and methods described herein can employ a model that is trained to extract one or more features from anatomical image data and to assess a spinal deformity based on the extracted features. Examples of spinal deformities can include spinal stenosis, dis degeneration analysis, vertebral fracture, spondylolisthesis, scoliosis, tumors, and/or disc herniation.

FIG. 4A is a flow chart of a method 400 of training a spinal analysis model (e.g., spinal analysis model 252 in FIG. 2), according to some embodiments. The spinal analysis model can be used to perform spinal deformity assessment and/or spinal deformity diagnosis. The method 400 can include reading image data and/or other input data from a training dataset, at 410. The training dataset can include input images of anatomical structures (e.g., spine, nerves, intervertebral discs, etc.) and corresponding output images of anatomical structures with labeling applied to the anatomical structure. In some embodiments, the training dataset can optionally include other input data, including, for example, segmentation data identifying different anatomical structures within the images and/or level identification data identifying different levels of the spine. In some embodiments, the input images can be merged or combined with the segmentation data and/or level identification data to provide a combined image, including the image data showing the tissue appearance and its classification (e.g., assignment of anatomical parts and/or structures based on the segmentation data and/or assignment of levels based on the level identification data). Alternatively, the image data, segmentation data, and/or level identification data can be separately provided as input into the model.

The output images can include labels associated with different output classifications. In some embodiments, the output images can include labels that provide information on a state or condition of one or more anatomical parts of structures. For example, the output images can include labels that identify whether a particular vertebral level or range of vertebral levels has or does not have a spinal deformity. Alternatively or additionally, the output images can include labels that identify a type of spinal deformity and/or a degree or grade of the deformity (e.g., Grade I-Grade IV, or none, mild, or severe). In some embodiments, the output images can include labels that identify one or more anatomical features of interest (e.g., anatomical features associated with a spinal deformity assessment), including a portion of one or more anatomical parts and/or structures or a visual or geometric feature that characterizes a portion of one or more anatomical parts and/or structures (e.g., a distance between two anatomical parts; a length, width, perimeter, or other geometric parameter of an anatomical part; a curvature of the spine or a portion thereof). In some embodiments, the output images can include labels that identify the vertebral level type and/or the vertebral levels associated with a spinal condition and/or deformity. In an example implementation, an output image can include one or more labels indicating the vertebral level, information corresponding to the anatomical feature analysis (e.g., shape, surface area, distance, etc.), and/or information corresponding to the spinal deformity assessment. In some embodiments, the images (e.g., input images and/or output images in the training dataset) can be 2D images of the anatomical structure depicting one or more views such as axial view, sagittal view, coronal view, etc. of the anatomical structure. The images can be grouped into multiple batches for training the spinal analysis model. Each image within a batch can include images representative of a series of slices of a 2D area and/or a 3D volume of an anatomical structure. A compute device (e.g., compute device 110, 210) can read the image data by loading one or more batches of images for further processing.

Optionally, several 2D images of the anatomical structure(s) can be combined to generate a 3D volume of the anatomical structure(s), at 420. For example, as discussed above, 2D images can depict a specific view of the anatomical structure such as axial view, sagittal view, coronal view, etc. The 2D images of different views of the anatomical structure can be combined to generate a 3D volume of the anatomical structure. For instance, images representing an axial view of the anatomical structure can be combined with images representing a sagittal view of the anatomical structure to generate a 3D volume of the anatomical structure. The 3D volumetric image data can then be used to train the model.

In some embodiments, the image data (e.g., 2D image data, 3D image data, etc.) can be augmented. Data augmentation can be performed on the image data to create a more diverse set of images. Each input image and its corresponding output image can be subjected to the same data augmentation, and the resulting input and output images can be stored as new images within the training dataset. The data augmentation can include applying one or more transformations or other data processing techniques to the images. These transformations or processing techniques can include: rotation, scaling, movement, horizontal flip, additive noise of Gaussian and/or Poisson distribution and Gaussian blur, etc. Data augmentation can be performed on any image type, including, for example, X-ray, CT scans, and/or MRI scans, as well as any image view (e.g., axial, sagittal, coronal).

Optionally, at 430, multi-dimensional regions of interest (ROI) can be selected and/or defined in the image data. In some embodiments, the ROIs can be defined based on predefined parameters (e.g., size of the region or the multidimensional stride). In some embodiments, overlapping ROIs can be defined, while in other embodiments, non-overlapping ROIs can be defined. In some embodiments, the predefined parameters can be adjusted based on the size or type of the image data and/or on the type of spinal deformity assessment (e.g., stenosis assessment, disc degeneration assessment, other deformity assessment, etc.). In some embodiments, image data from a 3D scan volume can be combined with segmentation data, level identification data, other image data (e.g., CT or X-ray being combined with MRI), and/or other data to provide input data having higher dimensionality. For example, 3D volumetric image data (e.g., 3D volume generated from 2D images and/or 3D information from medical imaging source such as imaging device 160) can be combined with other 3D information (e.g., manual and/or autonomous segmentation data, image data from another imaging source) to produce higher dimensional image data or ROIs. In an example implementation, each ROI or 3D region of image data can include (1) information about the voxel distribution along the multi-dimensional axes (e.g., X, Y, and Z axes), (2) appearance information of the anatomical parts captured by the imaging system for each voxel, and (3) segmentation data for each voxel indicative of the classification of the anatomical parts.

In some embodiments, the image data can be processed by one or more trained segmentation models (e.g., segmentation model in FIG. 3A) to segment the anatomical structure(s) of interest in the image data. For example, the compute device can be configured to input the image data into a trained segmentation model such that the segmentation model produces an output that classifies or labels one or more anatomical part(s) or structure(s) of interest. This segmentation output can then be used together with the image data to train the spinal analysis model (and optionally combined with the image data to produce multi-dimensional data, as described above).

In some embodiments, the image data can be processed by one or more level identification models (e.g., level identification model in FIG. 3B) to identify one or more vertebral levels of interest. For example, the compute device can be configured to input the image data into a trained level identification model such that the level identification model produces an output that classifies or labels the vertebra or range of vertebrae in the image data or ROI. This level identification output can then be used together with the image data to train the spinal analysis model (and optionally combined with the image data to produce multi-dimensional data, as described above).

At 440, a spinal analysis model can be trained using the training dataset. In some embodiments, the spinal analysis model can be trained using the image data, including the original image data and/or augmented image data, as well as other input data (e.g., segmentation data, level identification data, etc.). In some embodiments, the training can be supervised. The training can include inputting the input images into the spinal analysis model, and minimizing differences between an output of the spinal analysis model and the output images in the training dataset (i.e., the images including the relevant labels) corresponding to the input images. In some embodiments, the spinal analysis model can be a CNN model, whereby one or more weights of a function can be adjusted to better approximate a relationship between the input images and the output images. Further details of training a CNN model is described with reference to FIG. 4B. In some embodiments, the training can be unsupervised. For example, the spinal analysis model can rely on distance between feature vectors to classify known data points.

A validation dataset can be used to assess one or more performance metrics of the trained spinal analysis model. Similar to the training dataset, the validation dataset can include input images of anatomical structures (e.g., spine, nerves, intervertebral discs, etc.) and output images including labels associated with the anatomical structures and/or assessment of spinal deformities. The validation dataset can be used to check whether the trained spinal analysis model has met certain performance metrics or whether further training of the spinal analysis model may be necessary. At 450, input images of a validation dataset can run through the trained spinal analysis model to obtain outputs. At 460, one or more performance metrics can be calculated based on the outputs of processing the validation dataset. For example, the outputs of the validation dataset can be compared to the output images that correspond to the input images, and differences between the outputs of the model and the output images can be evaluated on a qualitative and/or quantitative scale. Different performance metrics can be calculated based on the differences between the outputs of the model and the output images corresponding to the input images. For example, a number or percentage of pixels (or groupings of pixels) that are classified correctly or incorrectly can be determined, and/or a Sørensen-Dice coefficient can be calculated.

At 470, the compute device can determine whether training is completed (e.g., performance of the trained spinal analysis model is sufficient and/or a certain number of training iterations has been met) or whether further training is necessary. In some embodiments, the compute device can continue to cycle through training iterations (i.e., proceed back to 410-460) until the performance of the trained model no longer improves by a predetermined amount (i.e., the performance metrics of a later training iteration 410-460 do not differ from the performance metrics of an earlier training iteration 410-460 by a predefined threshold value or percentage). If the model is not improving, the spinal analysis model may be overfitting the training data. In some embodiments, the compute device can continue to cycle through training iterations (i.e., proceed back to 410-460) until the performance metrics of a training iteration 410-460 reaches a certain predefined threshold indicative of sufficient performance. In some embodiments, the compute device can continue to cycle through training iterations (i.e., proceed back to 410-460) until a predefined number of iterations has been met (i.e., the spinal analysis model has been trained a predefined number of times).

Once the spinal analysis model has been sufficiently trained (470: YES), the spinal analysis model can be stored, e.g., in a memory (e.g., memory 230), at 480. The stored spinal analysis model can be used by the compute device in an inference or prediction process, e.g., to perform spinal deformity assessment and/or spinal deformity diagnosis on new image data of a patient.

FIG. 4B is a flow chart that provides a more detailed account of training 440 a spinal analysis model implemented as a neural network such as, for example, a CNN, according to embodiments. The neural network model can be trained by adjusting or tuning the parameters of the neural network model to be able to classify different portions of the image data based on features extracted from those portions. The neural network after being trained can be used to perform spinal deformity assessment and/or spinal deformity diagnosis (e.g., stenosis assessment, disc degeneration assessment, other deformity assessment, etc.) on a plurality of images (e.g., 2D scans of patient anatomy and/or 3D volume generated from 2D scans).

The method 440 can include inputting a batch of image data from a training dataset to a neural network, at 441. As described above, the training dataset can include input images of patient anatomy and corresponding output images of labeled patient anatomy (e.g., anatomical components such as vertebrae being labeled with information relating to anatomical feature analysis and/or spinal deformity assessment). Batches of images can be read from the training dataset one at a time, and processed using the neural network. In some embodiments, the batches of images can include different views of the anatomical structure (e.g., axial, sagittal, etc.). The different images can be combined to generate a 3D volume of the anatomical structure.

The batch of images can be passed through the layers of the neural network in a standard forward pass, at 442. The forward pass can return outputs or results, which can be used to calculate a value of a loss function, at 444. The loss function or objective function represents the function that is used to evaluate a difference between the desired output (as reflected in the output images that correspond to the input images) and the output of the neural network. The value of the loss function can indicate a measure of that difference between the desired output and the output of the neural network. In some embodiments, the difference can be expressed using a similarity metric, including, for example, a mean squared error, mean average error, or categorical cross-entropy. The value of the loss function can be used to calculate the error gradients, which in turn can be used to update one or more weights or parameters of the neural network, at 446. The weights and parameters, can be updated to reduce the value of the loss function in a subsequent pass through the neural network.

At 448, the compute device can determine whether the training has cycled through the full training dataset, i.e., whether the epoch is complete. If the epoch has been completed, then the process can continue to 450, where a validation dataset is used to evaluate the performance metrics of the trained spinal analysis model. Otherwise, the process may return to 441, where a next batch of images is passed to the neural network.

While not described with reference to FIGS. 4A and 4B, method 400 can also be used to train segmentation and/or level identification models. Detailed examples of such training are described in U.S. Patent Application Publication No. 2019/0105009, U.S. Patent Application Publication No. 2020/0151507, U.S. Patent Application Publication No. 2020/0410687, U.S. Provisional Patent Application No. 63/187,777, PCT Patent Application No. PCT/US22/29000, U.S. Patent Publication No. 2020/0327721, and U.S. Provisional Patent Application No. 63/256,306, incorporated by reference above.

4. Spinal Analysis

FIG. 5 is a flow chart of a method 500 of performing spinal analysis, according to some embodiments. The method 500 can be performed by a compute device, such as, for example, compute device 110, 210. In some embodiments, systems, devices, and methods described herein can use one or more spinal analysis models, e.g., trained per method 400 described with reference to FIGS. 4A and 4B. Alternatively or additionally, in some embodiments, systems, devices, and methods described herein can analyze certain anatomical features and assess one or more spinal conditions or deformities based on such analysis.

The method 500 can include reading a batch of images from patient image data, at 510. The images can be new images that are acquired of a patient's anatomy of interest. The images can be, for example, 2D scans of a 3D volume of one or more anatomical structures. In some embodiments, the images can include CT images, MRI images, and/or X-ray images. In some embodiments, the images can include axial, sagittal, and/or coronal views. In some embodiments, the images can be preprocessed. For example, the one or more images can be denoised using a model for denoising image data. Alternatively or additionally, the images can be processed using other techniques, such as, for example, filtering, smoothing, cropping, normalizing, resizing, etc. In some embodiments, inference-time distortions can be applied to one or more images, with a predefined number of distorted images being created for each input image. These distorted images can create inference results that are robust as to small variations in brightness, contract, orientation, etc.

At 520, segmentation can be performed on the patient image data. For example, the patient image data can processed by one or more segmentation models (e.g., trained segmentation models 232 in FIG. 2 or CNN 350 in FIG. 3A). In some embodiments, the patient image data can be segmented based on a predefined and/or selected ROI (e.g., such as ROI selected in step 430 in FIG. 4A). For instance, one or more segmentation models can process the ROIs (e.g., selected ROIs and/or predefined ROIs) to segment the patient image data so as to define or indicate a size and shape of a ROI within the anatomical structure. The segmentation models can be trained to identify one or more anatomical parts of interest from the image data. For example, the trained segmentation model can be used to identify bony structure (e.g., vertebral bodies, pedicles, transverse processes, lamina, and/or spinous processes) and/or one or more soft tissue structures of interest (e.g., nerves, intervertebral discs, etc.). Suitable examples of performing segmentation are described in U.S. Patent Application Publication No. 2019/0105009, U.S. Patent Application Publication No. 2020/0151507, U.S. Patent Application Publication No. 2020/0410687, U.S. Provisional Patent Application No. 63/187,777, and PCT Patent Application No. PCT/US22/29000, incorporated above by reference.

The image data can be processed using a level identification model (e.g., level identification model 234), at 530. Some example level identification models include vertebrae-based level identification process, disc-based level identification process, axial image-based level identification process, sagittal or coronal image-based level identification process, etc. Further details of such models include are described with in U.S. Provisional Patent Application No. 63/256,306, incorporated above by reference. For example, systems, devices, and methods can assign a level type (i.e., cervical (C), thoracic (TH), lumbar (L), and/or sacrum(S)) or a vertebral level (e.g., C1-S5, or C1-C7, TH1-TH12, L1-L5(L6), and/or S1-S5) to an image or portions of an image based on morphological and spatial relationships determined using vertebrae-based level identification and/or disc-based level identification, predictions of vertebral level types and/or vertebral levels determined using axial image-based level identification, and/or predictions of vertebral levels or ranges of vertebral levels determined using sagittal or coronal image-based level identification. In some embodiments, a vertebral type can be assigned to one or more sub-volumes or groups of vertebrae (e.g., using a level identification model) and then a vertebral level or ordinal identifier can be assigned to the one or more sub-volumes or groups of vertebrae based on morphological and spatial relationships between the vertebrae, the orientation of the patient anatomy in the image data, overall distribution of level types, etc. In some embodiments, indices can be assigned and counting can be used to assign the ordinal identifiers. For example, counting of lumbar vertebrae may start from L5 (or L6) if the sacrum is included in the image data or from L1 if the thoracic spine is included in the image data. Similar counting can be employed for each of the other vertebrae (e.g., cervical, sacrum, and thoracic). An ordinal identifier can be assigned to each group of anatomical components belonging to a level type (e.g., C, TH, L, S) and based on the anatomical structure and distribution of all the other levels.

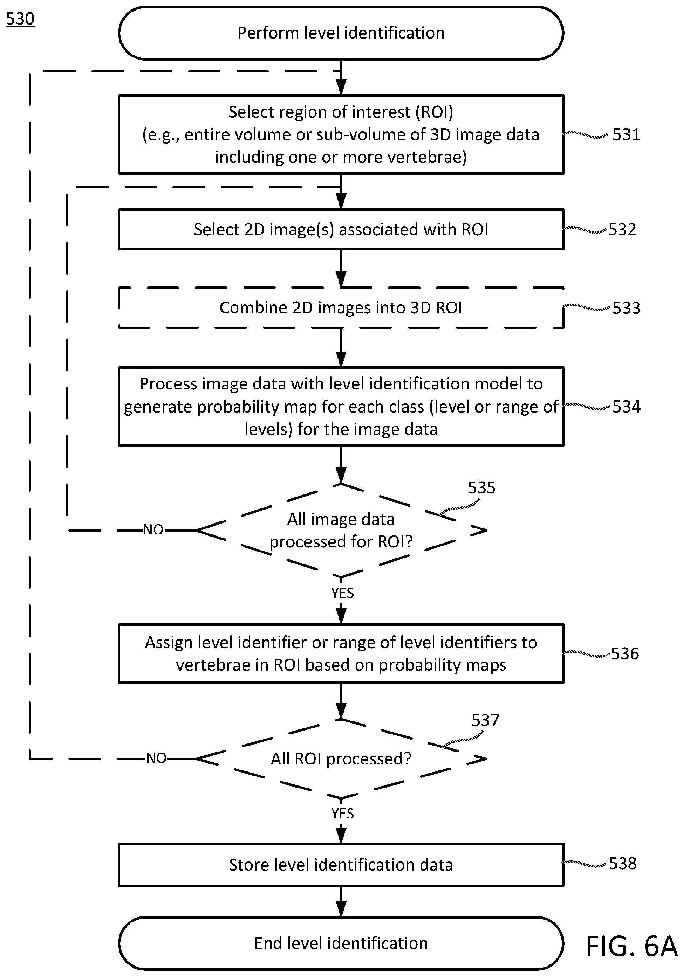

Alternatively or additionally, in some embodiments, a vertebral level or ordinal identifier (e.g., C1-S5, or C1-C7, TH1-TH12, L1-L5(L6), and/or S1-S5) can be assigned to one or more portions of the image data using a level identification model trained to assign vertebral levels to one or more vertebrae. For example, the level identification model can be trained using input images of one or more vertebrae and corresponding output images including labels identifying the vertebral level of the one or more vertebrae. Further details of using a trained level identification model are described with reference to FIG. 6A.

The patient image data can be further processed via one or more spinal analysis models to perform spinal deformity assessment, at 540. In some embodiments, the image data can be combined with the segmentation data and/or level identification data before being processed by the spinal analysis model(s). In some embodiments, the image data, the segmentation data, and/or the level identification data can be separately provided as input into a spinal analysis model for assessing the spinal deformity.

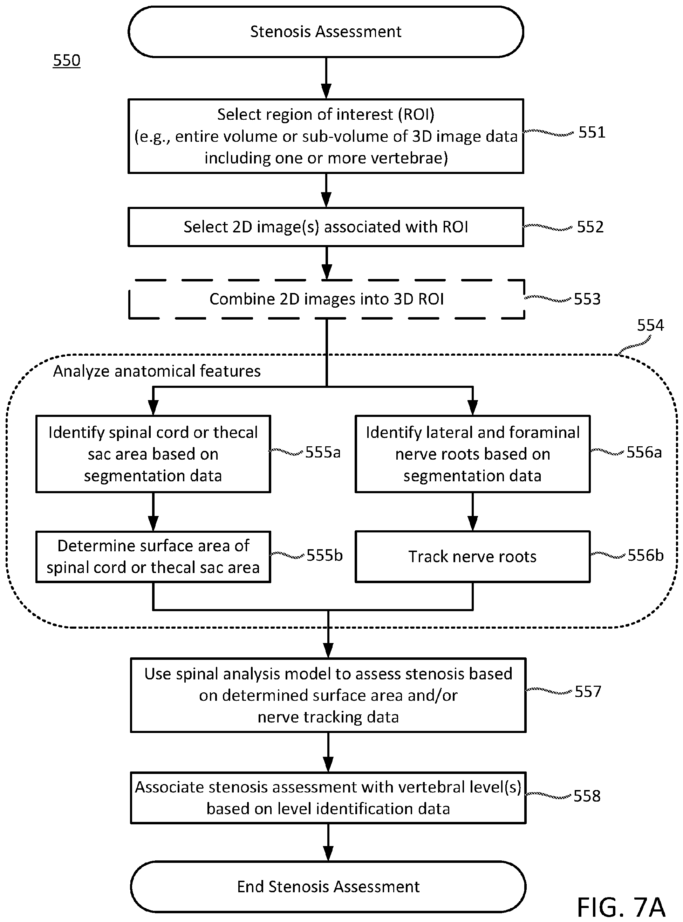

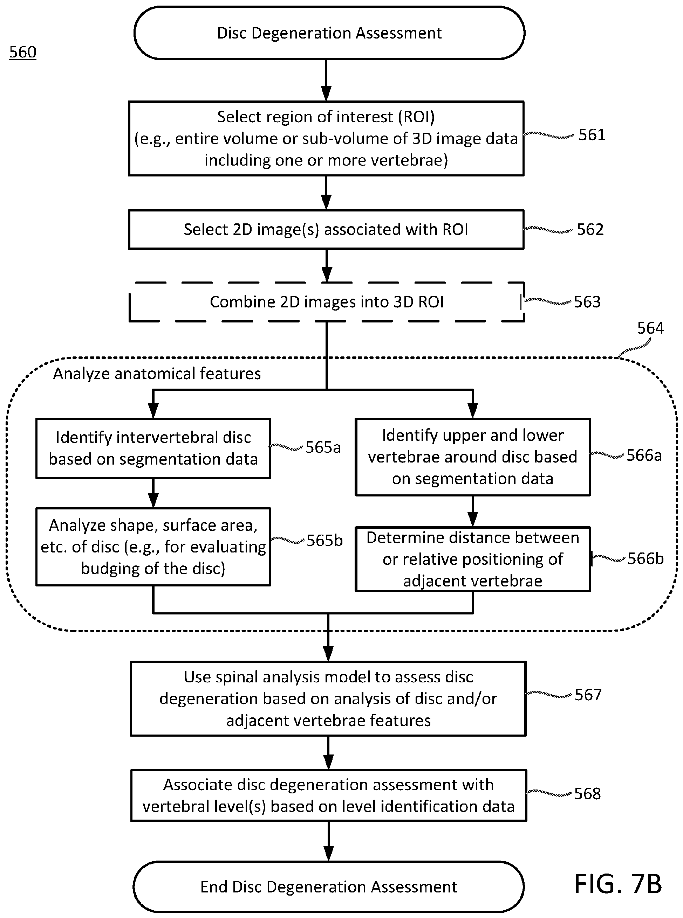

At 550, the image data (or a portion of the image data) can be input to a stenosis assessment process. For instance, the patient image data (or a portion of the image data) can be processed to analyze anatomical features such as spinal cord, nerve roots, thecal sac, etc. In particular, stenosis assessment can be performed based on the analysis of the anatomical features such as determining the surface area of the thecal sac and/or area for the spinal cord, or tracking of the nerve roots, etc. Further details of an example stenosis assessment process are described with reference to FIG. 7A. At 560, the image data (or a portion of the image data) can be input to a disc degeneration assessment process. For instance, the patient image data (or a portion of the image data) can be processed to analyze anatomical features such as intervertebral discs, etc. In particular, disc degeneration assessment can be performed based on the analysis of anatomical features such as the intensity, shape and/or surface area of the intervertebral disc, or analysis of the distance between two adjacent vertebrae, etc. Further details of an example disc degeneration assessment process are described with reference to FIG. 7B. At 570, the image data (or a portion of the image data) can be input to other suitable deformity assessment process (e.g., other deformity such as disc herniation, osteophyte formation, spondylolisthesis, etc.) to identify other deformities. For example, the patient image data (or a portion of the image data) can be processed to analyze any suitable anatomical feature (e.g., vertebral bodies, pedicles, lamina, nerves, intervertebral discs, etc.) to assess for other deformities (e.g., as depicted with reference to FIG. 11).

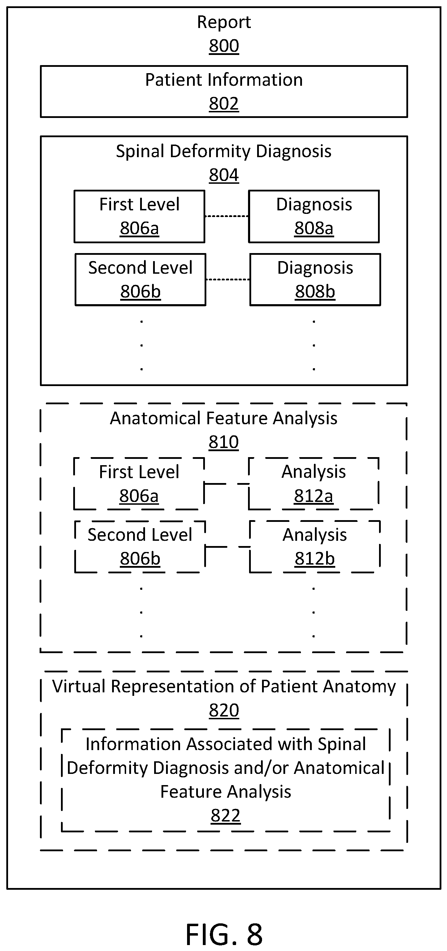

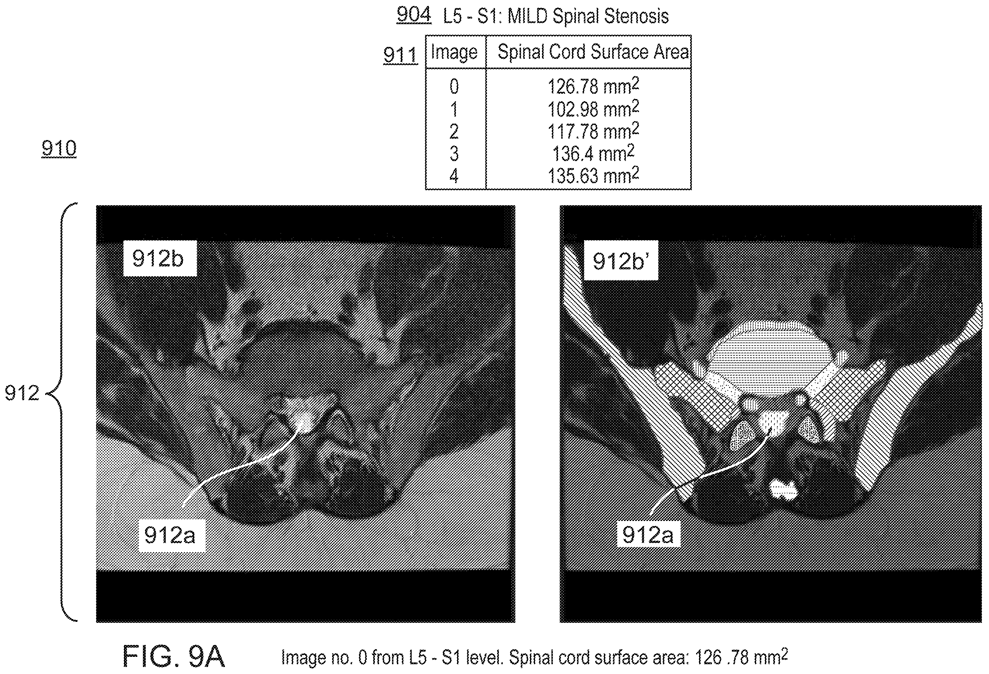

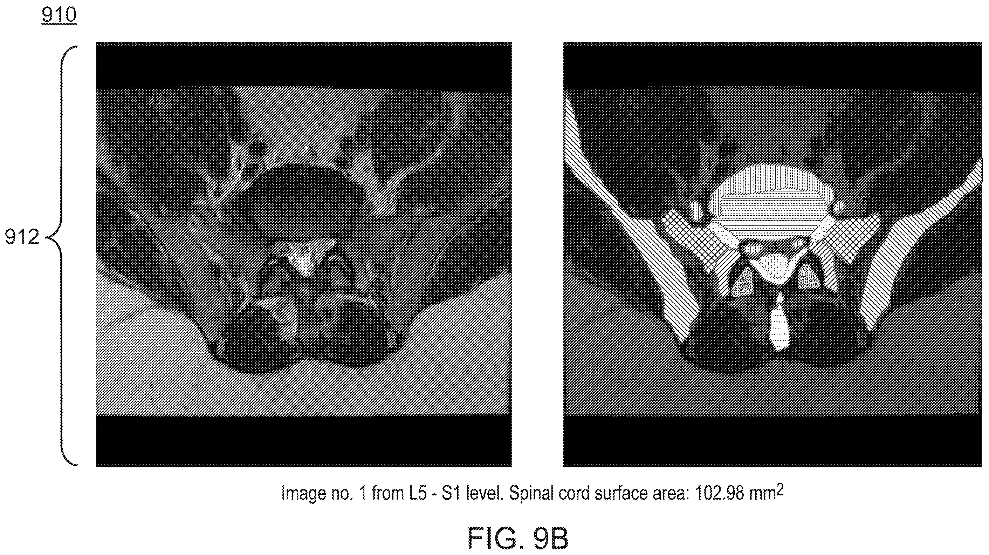

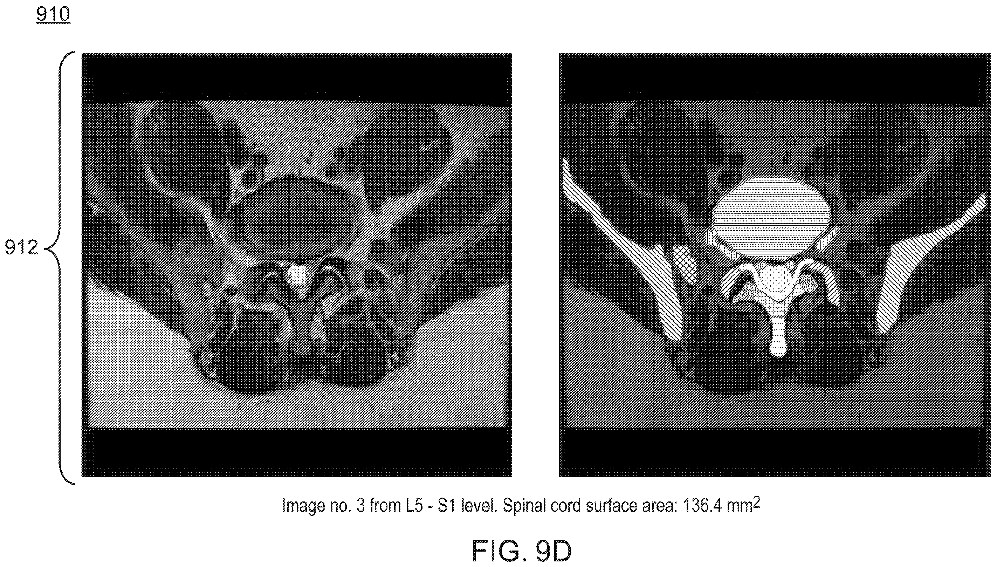

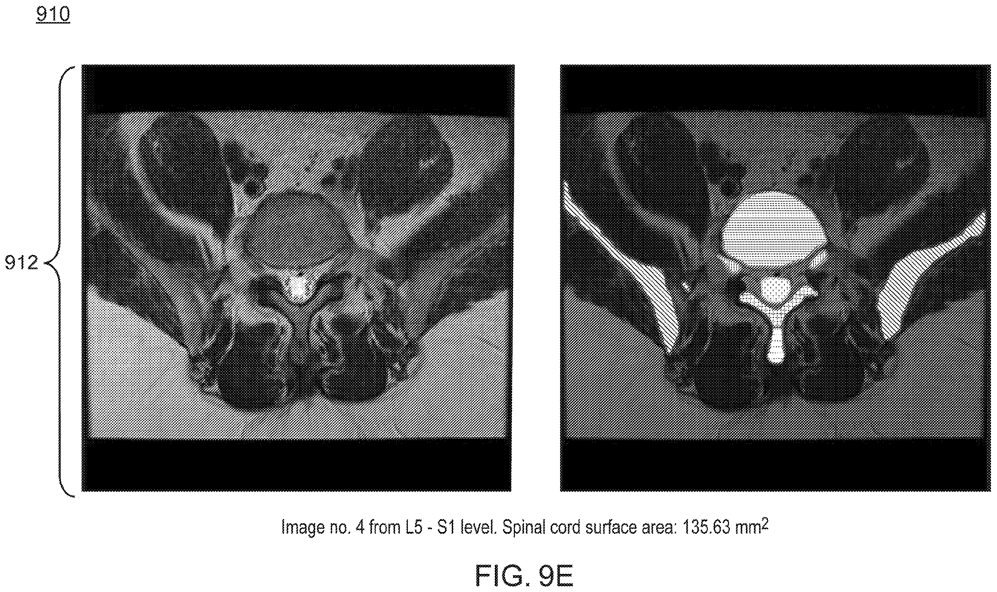

At 580, the spinal deformity assessment can be presented to a user (e.g., a surgeon). The spinal deformity assessment can include the type of deformity (e.g., stenosis, disc degeneration, and/or other deformity) and/or the severity or grade of the deformity (e.g., mild, medium, severe, or Grade I-Grade IV, etc.). In some embodiments, the presentation of the spinal deformity assessment can include the vertebral level associated with the spinal deformity assessment, e.g., based on the output of the level identification at 530, along with information indicative of the spinal deformity assessment. As an example, the assessment "L5-S1: MILD Spinal Stenosis" as shown in FIG. 9A can be presented to the user. In particular, the assessment includes the ordinal identifiers L5-S1 indicating that the anatomical parts in the patient image data includes Lumbar vertebrae starting from L5 until the Sacrum S1. The assessment also includes the output from the stenosis assessment process 550, indicating mild spinal stenosis for the patient. In some embodiments, if the spinal deformity assessment is conducted on multiple sets of patient image data (e.g., image data with different views such as axial view and sagittal view, image data captured at different imaging sessions, image data captured using different imaging devices), the assessment for the different sets of patent image data can be merged or presented together. For example, in cases where the different sets have produced different spinal deformity assessments (e.g., one assessment being mild and another being severe), the different assessments can be merged, e.g., according to predetermined schemes (e.g., averaging with or without weighting factors, majority rules, etc.), or be presented together to a user (e.g., surgeon). As another example, if stenosis assessment is conducted on axial image view and sagittal image view for a patient, the assessment from both these views can be merged and presented to the user or presented together to the user. In some embodiments, the outputs from processing different views can supplement one another. For example, the output from analyzing an axial view can include spinal deformity assessment which can be confirmed with the assessment obtained from analyzing sagittal view images. Further examples of presenting spinal deformity assessments are described below with reference to FIGS. 8-10C.

Optionally, one or more virtual representations of the patient anatomy can be generated, at 582, e.g., for visualization in pre-operative planning (e.g., via compute device 110, 210) and/or image-guided surgery (e.g., via a surgical navigation system 170). In some embodiments, a 3D anatomical model can be generated based on the image data, which can be used to generate virtual representations of the patient anatomy for visualization. In some embodiments, the 3D anatomical model can be converted or used to generate a polygonal mesh representation of the patient's anatomy (or portion thereof). The parameters of the virtual representation (e.g., volume and/or mesh representation) can be adjusted in terms of color, opacity, mesh decimation, etc. to provide different views of the patient anatomical structure to a user (e.g., a surgeon). In some embodiments, the virtual representations can be 2D views, e.g., a sagittal or coronal view of the patient's anatomy, with labelled vertebral level types or levels and/or information relating to analysis of an anatomical feature (e.g., surface area of a spinal cord or thecal sac, shape of an intervertebral disc, etc.). In some embodiments, the virtual representation can be 3D views, e.g., a 3D construction or model of the patient's spine and/or neighboring anatomy (e.g., nerves, discs, etc.). In some embodiments, anatomical models of the patient anatomy can be used to provide a virtual or augmented reality image for display by a computer-assisted surgical system, such as, for example, surgical navigation system(s) 170. In such systems, a virtual 2D or 3D view of the patient's anatomy can be displayed over a real portion of the patient anatomy (e.g., a surgical site).

At 590, the spinal deformity assessment and/or the outputs of the spinal analysis models can be stored in memory (e.g., memory 230).

i. Anatomical Segmentation

As described above with reference to 520 of FIG. 5, systems, devices, and methods described herein can be configured to perform autonomous spine anatomical segmentation. In some embodiments, segmentation can be performed using pre-trained models, including those described in U.S. Patent Application Publication No. 2019/0105009, U.S. Patent Application Publication No. 2020/0151507, U.S. Patent Application Publication No. 2020/0410687, U.S. Provisional Patent Application No. 63/187,777, and PCT Patent Application No. PCT/US22/29000, incorporated above by reference. For example, the segmentation can be performed using a pre-trained modified 2D U-Net neural network.

In some embodiments, segmentation can be performed on MRI images, including, for example, T2-weighted scans. Systems and devices described herein (e.g., compute devices 110, 210) can be configured to load patient image data and make an automatic selection of T2-weighted sequences that provides better visualization of one or more anatomical parts of interest (e.g., thecal sac, nerve roots, intervertebral discs, etc.). T2 hyperintensity can be associated with fluid heavy tissue and T2 hypointensity can reflect hypercellularity. Segmentation can be performed in multiple planes, such as, for example, in the sagittal and the axial planes. In some embodiments, a first model (e.g., a neural network) can be trained and used to perform segmentation of axial images, and a second model (e.g., a neural network) can be trained and used to perform segmentation of sagittal images. Alternatively, a single model can be trained and used to perform segmentation of images in multiple planes (e.g., axial and sagittal).

In some embodiments, pre-processing of imaging data can be performed prior to processing that imaging data by a segmentation model to segment the imaging data. For example, such pre-processing can include one or more algorithms or models that analyze imaging data quality, rotation, sequence, and/or other like parameters to ensure that the imaging data is correct and can be inputted into a segmentation model.

The segmentation model can be configured to segment the imaging data into a plurality of classes, e.g., between two and about 20 classes, including, for example, 16 classes. For example, when used to segment MRI image data, the segmentation model can be configured to segment the imaging data into more than two classes and up to about 20 classes, including, for example, 16 classes. In some embodiments, the segmentation model can be configured to segment MRI imaging data into classes that correspond to one or more anatomical parts of interest without segmenting surrounding anatomical parts that are not of interest. For example, when evaluating spinal stenosis and/or disc degeneration, a segmentation model can be trained and used to segment the MRI imaging data into two or more classes, including one or more of the thecal sac, the annulus, and the nucleus. Alternatively, when evaluating stenosis, a segmentation model can be trained and used to segment the MRI imaging data into (1) two classes, such as, for example, thecal sac and not thecal sac, or (2) three classes, such as, for example, thecal sac, nerve roots, and not thecal sac or nerve roots. Still alternatively, when evaluating disc degeneration, a segmentation model can be trained and used to segment the MRI imaging data into three classes, such as, for example, the annulus, the nucleus, and all other anatomy. Limiting the segmentation to specific anatomical parts of interest can reduce computational requirements (e.g., by having a smaller neural network), provide for easier and/or faster validation, and require less training and/or manual marking.

ii. Level Identification

FIG. 6A is a flow chart of an example level identification process 530, according to some embodiments. As described above, one or more level identification models can be used to identify the vertebral levels of different vertebrae depicted in the image data.

At 531, a ROI can be selected in the image data. The ROI can be the entire volume of image data or a portion of the image data (e.g., sub-volume, one or more 2D images). The ROI can be selected to include a specific anatomical component or structure such as one or more vertebrae, intervertebral discs, nerves, etc. Alternatively, ROIs can be selected to encompass a specific area, volume, and/or sub-groups of pixels in an image. In some embodiments, ROIs can be selected by a user (e.g., a surgeon). For example, a surgeon may want to assess one or more specific anatomical components or structures such as one or more vertebrae, intervertebral discs, nerves, etc. for deformity and may select a ROI to identify the specific anatomical components or structures in the image data. In some embodiments, ROIs can be autonomously selected (e.g., by a compute device) based on the type of image data, size of the image data, and/or type of spinal deformity assessment.

At 532, the method can include selecting 2D image(s) associated with the ROI. For example, consider that the ROI selected at 531 identifies a volume of interest in the anatomical structure. At 532, 2D images of that volume can be selected. These images can be 2D axial views of the volume of interest, 2D sagittal views of the volume of interest, 2D coronal views of the volume of interest, and/or a combination thereof. In a specific implementation, consider that the ROI selected at 531 includes an anatomical part of interest such as a specific vertebrae. At 532, 2D images of that vertebrae can be selected. These images can be 2D axial views of the vertebrae, 2D sagittal views of the vertebrae, 2D coronal views of the vertebrae, and/or a combination thereof.

Optionally, at 533, these 2D images of the ROI can be combined into a 3D ROI. For instance, if the ROI identifies a specific vertebrae and, at 532, 2D axial views, 2D sagittal views, and/or 2D coronal views of that vertebrae are selected, then at 533, these axial, sagittal, and/or coronal images can be combined to generate a 3D volume of that vertebrae. In some embodiments, the 2D images can be used to generate a 3D model or representation of one or more anatomical parts in the ROI.

At 534, for each selected ROI (e.g., 2D images associated with a ROI and/or 3D ROI), the image data associated with that ROI can be processed with one or more level identification model(s) (e.g., level identification CNN 300 in FIG. 3B). The image data can include axial image scans, sagittal image scans, and/or coronal image scans including one or more vertebrae. The level identification model(s) can be trained to predict a vertebral level for each vertebra based on axial image scans, sagittal image scans, and/or coronal image scans associated with the ROI. In some embodiments, multiple scans associated with each vertebra in a ROI can be processed separately to each provide a level identification prediction for that vertebra. Alternatively, multiple scans (e.g., axial, sagittal, and/or coronal) associated with each vertebra can be processed together to provide a single level identification prediction for that vertebra (or be processed in batches that each provides a level identification prediction for that vertebra).

The level identification model(s) can be trained to generate a probability map representing the probability of assigning an anatomical component (e.g., each vertebra) to a class (e.g., vertebral level or ordinal identifier). Put differently, the output of the level identification model(s) can include a probability map for each class (e.g., each vertebral level) for each anatomical component. For example, the output of the level identification model(s) can include the per-class probabilities for each pixel (or group of pixels) of the image data. More specifically, the level identification model(s) can be configured to classify the image data into one of a plurality of classes (e.g., vertebral levels). Accordingly, the level identification model(s) can be configured to generate, for each pixel or group of pixels in the images, the probability that that pixel or group of pixels belongs to any one of the classes from the plurality of classes. The plurality of classes can correspond to a plurality of vertebral levels or ordinal identifiers (e.g., TH1-TH12, L1-L5, S1-S5, and/or C1-C7).

If more image data is associated with the selected ROI (535: NO), then the process 530 can return to 532 and iterate through the process with additional image data (e.g., one or more additional 2D scans or 3D sub-volumes). If not, the process 530 proceeds with assigning level identifier or range of level identifiers for that ROI, at 536.

At 536, a vertebral level or ordinal identifier (e.g., C1-S5, or C1-C7, TH1-TH12, L1-L5(L6), and/or S1-S5) may be assigned to one or more vertebrae in the ROI based on the output of the level identification model (e.g., the probability maps). For example, one or more vertebrae can be associated with different portions of the image data (e.g., different portions of 2D axial, sagittal, and/or coronal scans or different portions of 3D sub-volumes). When processing the image data with the level segmentation model, the model can return an output that can assign a particular class (e.g., vertebral level) to those portions of the image data that correspond to each of the vertebrae. In some embodiments, multiple images (e.g., 2D axial, sagittal, and/or coronal images) can be associated with a particular vertebrae, and the level identification model may predict different vertebral levels for different images. For example, a set of axial images associated with a selected vertebra may include 80% that are assigned a first class (e.g., L1) and 20% that are assigned a second class (e.g., L2). In some embodiments, the class for the selected vertebra can be selected to be the class that has the greatest number of axial images assigned to it. As such, for a set of axial images of a vertebra where 80% are labeled "L1" and 20% are labeled "L2," the vertebra can be assigned the level "L1." Alternatively, other criteria (e.g., predefined number or percentage of axial images being associated with the class) can be used to determine the class (vertebral level) that is assigned to the vertebra.

If not all of the ROI have been processed (537: NO), then the process 530 can return to 531 and another ROI can be selected. At 538, the level identification data (e.g., vertebral level or ordinal identifier) assigned to the ROIs can be stored in memory (e.g., memory 230).

While an example method of performing level identification is described with reference to FIG. 6A, it should be readily understood that other suitable methods can be used for level identification, including those methods described in U.S. Provisional Patent Application No. 63/256,306, incorporated by reference above, and the intervertebral disc level identification process described below.

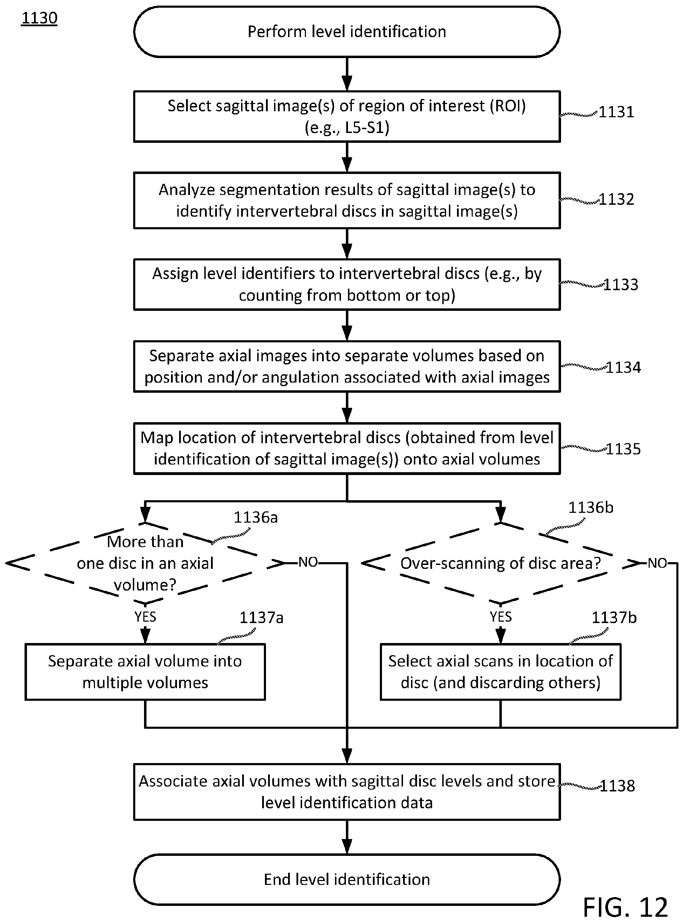

In some embodiments, systems, devices, and methods described herein can perform level identification of intervertebral discs. For example, FIG. 12 depicts an example process 1130 for performing autonomous intervertebral level identification, according to embodiments. Level identification process 1130 can be particularly suitable for grouping and assigning levels to MRI axial scans. With MRI imaging, axial scans can be taken continuously like with CT but can also be taken in groups or blocks around intervertebral discs. Typically, each group of MRI axial images around an intervertebral disc includes a set number of images, e.g., at least several images such as about 5 or more images, where the set number of images represent a scanning volume or axial volume. Each of the axial volumes has different scanning angulation, parallel to or substantially parallel to the intervertebral disc angulation. In practice, most axial scan volumes have problems, e.g., associated with the volume itself, the number of images in the volume, etc. As such, to separate and identify the intervertebral discs properly, it can beneficial to start with sagittal image analysis, where the intervertebral discs are more readily identified and distinguished from one another.

At 1131, one or more sagittal images (e.g., a sagittal image or a sagittal volume) of a section or portion of the spine can be selected. At 1132, the segmentation results or segmentation data of the sagittal image(s) can be analyzed to identify and separate the intervertebral discs. As described above, the segmentation data can be generated by a segmentation model that has been trained, e.g., to segment the intervertebral discs starting from a specific intervertebral level. After separating the intervertebral discs in the sagittal image(s), at 1133, level identifiers can be assigned to the intervertebral discs, e.g., by counting from the bottom level and assigning a level identifier to each level.

With the axial scans, the position (e.g., real world position) of the intervertebral discs can be mapped onto the axial images or volumes. The axial images may have been previously separated into axial volumes (i.e., sets of adjacent axial scans) based on their position and angulation. While position and angulation of the axial scans can enable some degree of separation between the different volumes, problems arise when the volumes are wrongly scanned, e.g., with additional images within a volume or images for two levels or discs being connected. As such, the location of the intervertebral discs obtained from the sagittal image(s) can be mapped into the axial volumes, at 1135, to improve the separation of the axial volumes. Stated differently, the separated axial volumes can be analyzed with information obtained from the segmentation and level identification of the sagittal image(s), to be able to determine the real world location of the intervertebral discs. Such analysis can enable determination of which axial volume corresponds to which intervertebral disc and whether there are any problems with the axial volumes. For example, at 1136a, if it is determined that there are two discs from the sagittal image located in a single axial volume, then at 1137a, the axial volume can be separated into two parts or volumes, with each part or volume being for a separate disc. Alternatively or additionally, at 1136b, if it is determined that an axial volume was over-scanned (e.g., has more than the set number of images), then at 1137b, a subset of the axial images in that volume such as the set number of images around or closest to an intervertebral disc can be selected as the volume, with the other images being discarded (i.e., not used in the spinal deformity analysis). After performing such analysis and cleaning, at 1138, the cleaned axial volumes can be bundled with or associated with the sagittal levels, e.g., to have a visualization, separation, and identification of each level in the axial and sagittal planes.

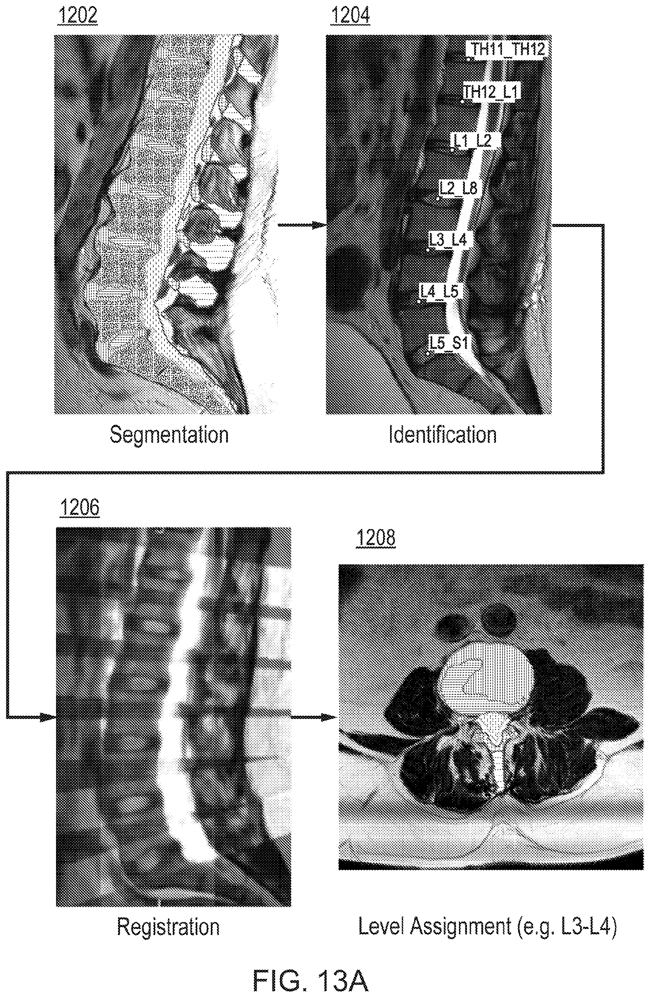

FIG. 13A visually depicts the flow of identifying and assigning levels to different MRI imaging sets, according to embodiments. The flow of FIG. 13A can be similar to the segmentation and level identification processes described above. The process can start with a sagittal image or images, and segmentation can be performed in the sagittal plane, at 1202. Based on the segmentation data, the intervertebral discs can be separated and identified, as shown at 1204. The axial volumes, depicted as light bands at 1206, can be registered with each of the levels in the sagittal images. In some embodiments, the axial volumes can be cleaned as described with reference to the level identification process 1130. Then each axial volume can be assigned to a different level, at 1208.

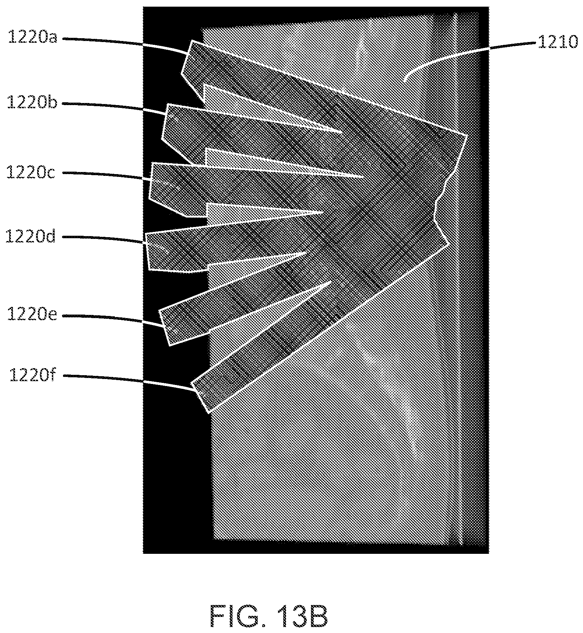

FIG. 13B provides a visualization of a set of axial volumes 1220a-1220f that has been combined with a sagittal image or volume 1210, according to embodiments. In some embodiments, such visualization or similar visualization can be provided, e.g., to a surgeon, to facilitate surgical planning and/or a surgical operation. For example, visualization of sagittal and axial image data can be provided on a surgical navigation system (e.g., surgical navigation system 170) or other compute device.

iii. Spinal Deformity Assessment

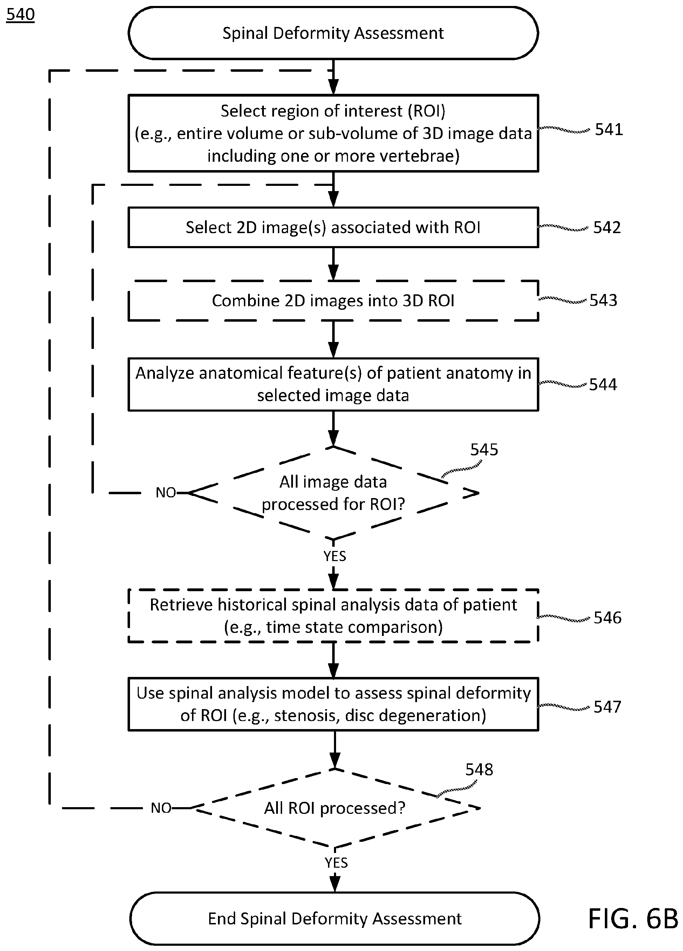

FIG. 6B is a flow chart of an example spinal deformity assessment process 540, according to some embodiments. The spinal deformity assessment process 540 can be generally applicable in analyzing a variety of different spinal deformities, including, for example, spinal stenosis, disc degeneration, vertebral fracture, etc. At 541, a ROI can be selected in the image data (e.g., similar to 531 in FIG. 6A). The ROI can be the entire volume of image data or a portion of the image data (e.g., sub-volume, one or more 2D images). The ROI can include one or more anatomical structures or portions thereof.

At 542, 2D images associated with the ROIs can be selected (e.g., similar to 532 in FIG. 6A). These images can include axial views of the patient anatomy, sagittal view of the patient anatomy, coronal views of the patient anatomy, and/or a combination thereof. Optionally, at 543, these 2D images can be combined to generate a 3D ROI (e.g., similar to 533 in FIG. 6A).

At 544, one or more anatomical features of a patient's anatomy can be analyzed in the selected image data (e.g., 2D image data, or 3D volume). For example, the selected image data may include one or more anatomical parts of interest such as the spinal cord, thecal sac, intervertebral disc, nerve roots, etc. The selected image data can be analyzed to assess and/or determine one or more anatomical features or characteristics associated with the anatomical parts of interest. These features or characteristics can include shape, surface area, volume of the anatomical parts, and/or distance between two or more anatomical parts, etc. For example, surface area of the spinal cord or a portion of the spinal cord, surface area of the thecal sac or a portion of the thecal sac, nerve roots (e.g., lateral and foraminal nerve roots), shape and/or surface area of intervertebral discs, distance between two adjacent vertebrae, etc. in the image data can be determined and/or analyzed for assessing different types of deformities.

If more image data is associated with the selected ROI (545: NO), then the process 540 can return to 542 and iterate through the process with additional image data (e.g., one or more additional 2D scans or 3D sub-volumes). If not, the process 540 proceeds to 546.

At 546, the compute device can optionally retrieve historical spinal analysis data of the patient. This historical spinal analysis data can include data associated with previous assessments of prior collected image data for the patient. For example, patient image data collected at a previous time (e.g., one or more previous years, previous months, previous days, etc.) may have been evaluated by a physician and/or processed using the spinal deformity assessment process 540. The physician's assessment and/or output of the spinal deformity assessment process 540 on the previous image data can be stored as historical spinal analysis data. The historical spinal analysis data can then be retrieved in a later spinal analysis process 540, e.g., to inform a change and/or a rate of change of one or more features or characteristics of one or more anatomical parts over time. For instance, the historical spinal analysis data can include information about the rate of change of surface area of the spinal cord, surface area of the thecal sac, shape and/or surface area of intervertebral discs, distance between two adjacent vertebrae, etc.

At 547, the image data (e.g., 2D images associated with a ROI and/or 3D ROI) can be processed by one or more spinal analysis models to assess a spinal deformity (e.g., stenosis, disc degeneration, disc herniation, etc.) of one or more anatomical parts and/or structures in the image data. For example, the image data can be input into a stenosis assessment process, as described in more detail herein with reference to FIG. 7A. Similarly, the image data can be input into a disc degeneration assessment process, as described in more detail herein with reference to FIG. 7B. As noted above, in some embodiments, the image data can be combined with the segmentation data and/or level identification data before being processed by the spinal analysis model(s). In some embodiments, the image data, the segmentation data, and/or the level identification data can be separately provided as input into a spinal analysis model for assessing the spinal deformity. The spinal analysis model(s) can process the image data (optionally with segmentation and/or level identification data) to predict or determine the presence or absence of the deformity. Additionally or alternatively, the spinal analysis model(s) can process the image data (optionally with segmentation and/or level identification data) to grade the severity of the deformity (e.g., no deformity, mild deformity, medium deformity, severe deformity, etc.).

In some embodiments, the spinal analysis model(s) can be a machine learning model (e.g., CNN) that can predict or determine a state or condition of the anatomical structures and/or parts thereof in the image data based on segmentation data generated at 520, level identification data generated at 530, the anatomical feature analysis performed at 544 and/or other features extracted from the image data, and/or historical spinal analysis data retrieved at 546. In some embodiments, the spinal analysis model(s) can be apply one or more criteria to evaluating a state or condition. For instance, threshold values and/or threshold ranges can be associated with features or characteristics of anatomical parts to indicate the severity of the spinal deformity. In some embodiments, the threshold values and/or threshold ranges can be determined based on the patient groups. For example, threshold values and/or threshold ranges can be an average across image data obtained for various patient groups such as an average across different age, gender, etc. In some embodiments, the spinal deformity can be classified (e.g., no deformity, mild deformity, medium deformity, severe deformity, etc.) based on evaluating whether one or more features or characteristics fall within certain threshold ranges and/or values. For example, the spinal analysis model(s) can include a first range of surface area values for the spinal cord that would indicate no deformity, a second range of surface area values for the spinal cord that would indicate mild deformity, and a third range of surface area values for the spinal cord that would indicate severe deformity. If the analysis of the spinal cord surface area performed at 544 returns a surface area within the first range, then the spinal analysis model(s) can output "no deformity." However, if the analysis of the spinal cord surface area performed at 544 returns a surface area within the second or third range, then the spinal analysis model(s) can output "mild deformity" or "severe deformity," respectively. As another example, the spinal analysis model(s) can include a first threshold value for a distance between adjacent vertebrae (e.g., an anterior distance, posterior distance, maximum distance, or minimum distance) that would indicate no deformity, a second threshold value for a distance between adjacent vertebrae that would indicate mild deformity, and a third threshold value for distance between adjacent vertebrae that would indicate severe deformity. If the analysis of the vertebrae performed at 544 returns a distance lower than the first threshold value, then the spinal analysis model(s) can output "no deformity." However, if the analysis of the vertebrae performed at 544 returns a distance greater than the second threshold value or a distance greater than the third threshold value, then the spinal analysis model(s) can output "mild deformity" or "severe deformity," respectively.

In some embodiments, the spinal analysis model(s) can compare current anatomical feature analyses with historical spinal analysis data retrieved at 546 to predict a spinal deformity assessment for the ROI. For example, if a comparison of current anatomical feature values to historical data retrieved at 546 indicates a change in a height of an intervertebral disc in the ROI (e.g., decreasing height of intervertebral disc) that is greater than a predetermined threshold value or percentage, then the spinal analysis model(s) may predict a possible spinal deformity.

Optionally, at 548, if all the ROIs for the image data have not been processed (548: NO), the process 540 may return to 541 and iterate from 541-547. Once all the ROI have been processed, then the process 540 can end and proceed to 580, as described with reference to FIG. 5.

FIGS. 7A and 7B, described below, provide examples of spinal stenosis assessment 550 and intervertebral disc degeneration assessment 560.

a) Stenosis Assessment

Spinal stenosis is characterized by a narrowing of the spinal canal and/or the intervertebral foramen. Spinal stenosis can be caused by soft tissue changes such as disc herniation (e.g., displacement of intervertebral disc tissue such as displacement of nucleus pulpous in the intervertebral disc), fibrous scar or tumor, etc., or it can be caused by changes to bony structure(s) (e.g., vertebral bodies, pedicles, transverse processes, lamina, and/or spinous processes) such as intervertebral disc collapse, osteophyte formation, spondylolisthesis (e.g., forward slip of vertebrae), etc. Therefore, spinal stenosis can lead to reduced volume and/or altered shape of the spinal canal.

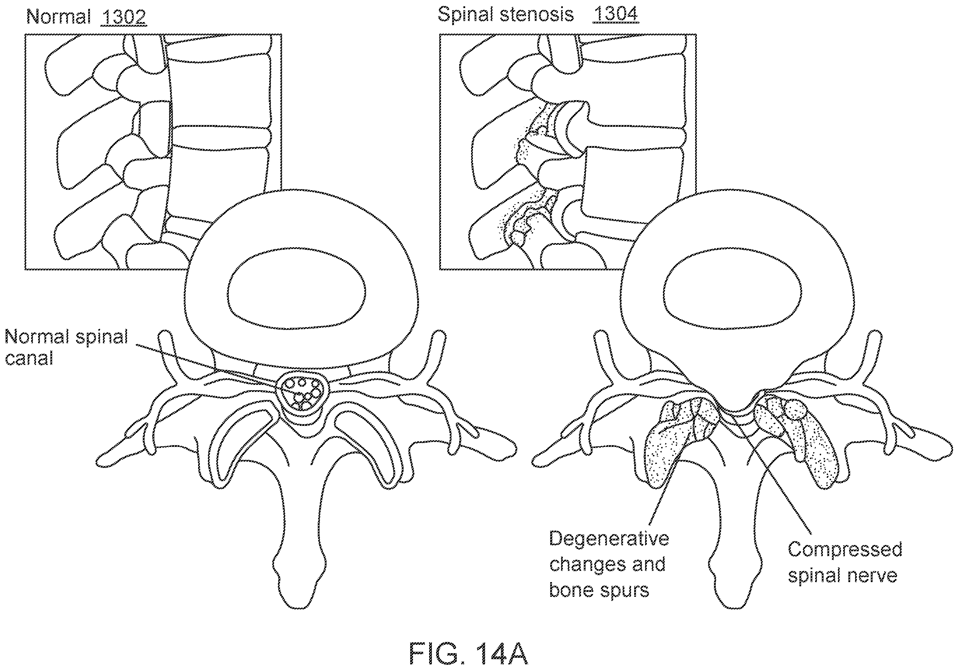

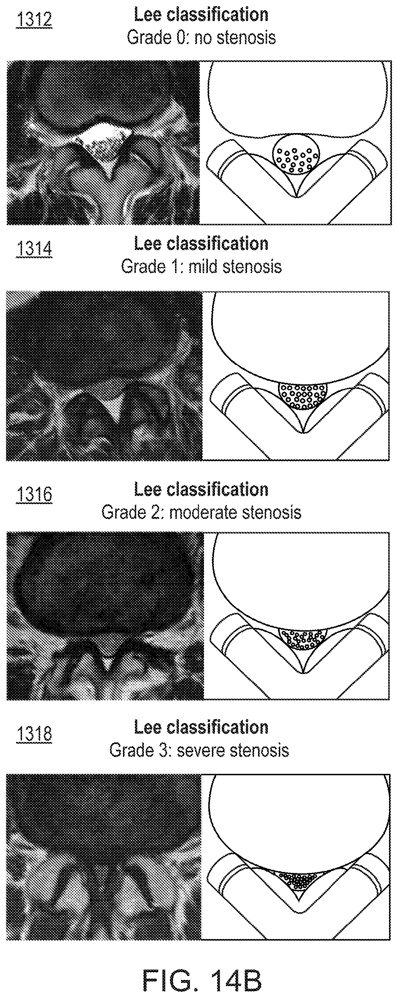

FIG. 14A provides an illustration of spinal anatomy, under normal conditions 1302 and with spinal stenosis 1304. As shown, with spinal stenosis, the volume and shape of the thecal sac or sheath that surrounds the spinal cord (and therefore the space for the spinal cord) has been reduced. FIG. 14B depicts a grading system for classification of spinal stenosis developed by Lee et al. ("Lee grading system"). The Lee grading system defines various qualitative metrics for classifying different cases of spinal stenosis. The Lee grading system is based on the obliteration of cerebrospinal fluid (CSF) space and distribution of the nerve roots in the thecal sac, as viewed in MRI images (e.g., T2-weighted images). Under the Lee grading system, a case is classified Grade 0 (no stenosis, 1312) when the anterior CFS space is not obliterated. A case is classified Grade 1 (mild stenosis, 1314) when the anterior CSF space is mildly obliterated, but all nerve roots can be clearly separated from one another. A case is classified Grade 2 (moderate stenosis, 1316) when the anterior CSF space is moderately obliterated and some of the nerve roots are aggregated (e.g., impossible to visually separate). And a case is classified Grade 3 (severe stenosis, 1318) when the anterior CSF space is severely obliterated showing marked compression of the thecal sac and the nerve roots appear as one bundle (e.g., none can be visually separated from one another).

As noted previously, identification of spinal stenosis by surgeons is subjective and can be inconsistent. Even with grading systems such as the Lee grading system, different surgeons viewing the same MRI scans and using the same grading system may arrive at different conclusions and therefore recommend different clinical interventions. As such, systems, devices, and methods described herein, by taking a more quantitative approach (e.g., in evaluating the thecal sac and/or other anatomy around the spinal canal), can provide a more reliable assessment of spinal stenosis.

FIG. 7A is a flow chart of a stenosis assessment process 550, according to some embodiments. At 551, ROIs can be selected in the image data (e.g., similar to 531 in FIG. 6A and 541 in FIG. 6B). The ROIs can include one or more anatomical parts of interest, e.g., one or more vertebrae and surrounding soft tissue (e.g., nerve roots) and/or other structures. At 552, 2D images associated with the ROIs can be selected (e.g., similar to 532 in FIG. 6A and 542 in FIG. 6B). These images can include axial views of the patient anatomy, sagittal view of the patient anatomy, coronal views of the patient anatomy, and/or a combination thereof. Optionally, at 553, these 2D images can be combined to generate a 3D volumes or sub-volumes for further analysis (e.g., similar to 533 in FIG. 6A and 543 in FIG. 6B).

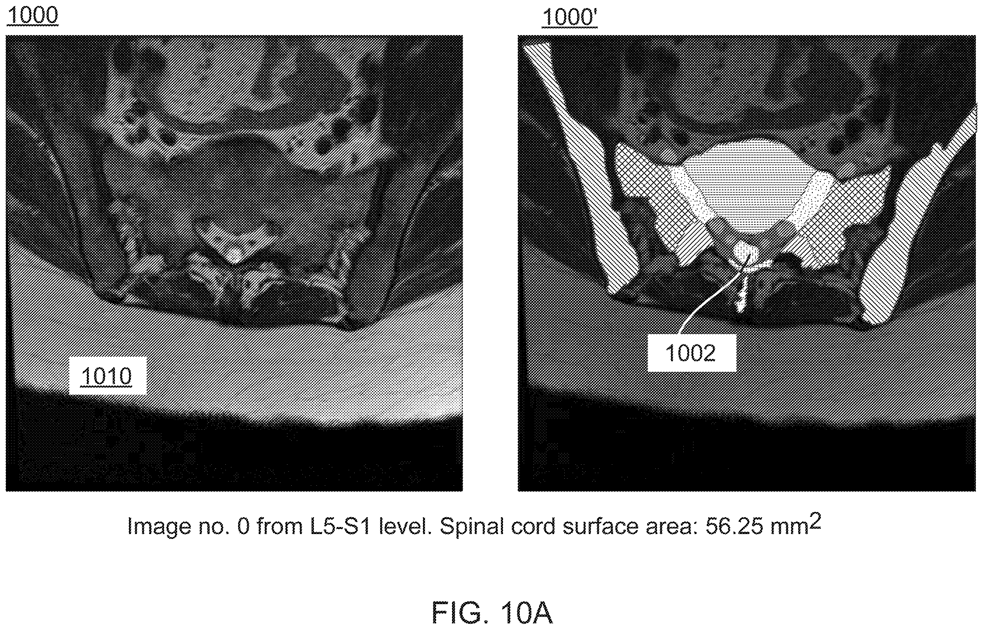

At 554, one or more anatomical features of a patient's anatomy in the selected image data can be analyzed (e.g., similar to 544 in FIG. 6B). In some embodiments, at 555a, spinal cord and/or thecal sac can be identified in the selected image data. The selected image data can include axial scans of a portion of the spine (e.g.,

axial scans of C1-C2, ... C6-C7, C7-TH1, TH1-TH2, TH2-TH3, .... TH11-TH12, TH12-L1, L2-L3, L4-L5, L5-S1, ... S6-S7, etc.). The identification of the spinal cord and/or thecal sac can be based on the output of the segmentation model(s), at 520, or manual identification/labeling of the spinal cord and/or thecal sac. FIGS. 9A-9E illustrate example axial images of the portion of the spine L5-S1 for a patient. The axial images include segmentation data that identifies the spinal cord 912a. Similarly, FIGS. 10A-10B illustrate example axial images of the portion of the spine L5-S1 and TH12-L1 respectively for a patient. The axial images include segmentation data with portions 1002 and 1022 in FIGS. 10A-10B representing the spinal cord. At 555b, the area of the spinal cord and/or the thecal sac can be determined based on the image data and/or segmentation data. For instance, the portions of the images representing the spinal cord and/or the thecal sac can be analyzed and the area of these portions can be measured. In some embodiments, the surface area can be represented as a number of pixels, while in other embodiments, the surface area can be represented in real metrics (e.g., mm$^2$). In some embodiments, where multiple axial scans of a portion of a spine are being used to analyze the spinal cord and/or thecal sac, a surface area of the cross-section of the spinal cord and/or thecal sac can be measured in each of the axial scans, and an average measure (e.g., a mean, median, etc.) can be used to approximate the spinal cord and/or thecal sac surface area for that portion of the spine. The area of the spinal cord and/or thecal sac can be indicative of the presence or absence and/or the severity of stenosis. For example, smaller surface areas associated with the spinal cord and/or thecal sac can be indicative of a greater severity of stenosis. While the surface area is described as being used to assess stenosis, it can be appreciated that other parameters associated with the spinal cord and/or thecal sac area can be used to assess stenosis, including, for example, a lateral dimension (e.g., width, height, etc.) of the thecal sac area, a shape of the thecal sac area, etc.

Additionally or alternatively, at 556a, nerve roots can be identified in the selected image data. The identification of the nerve roots can be based on the output of the segmentation model(s), at 520, or manual identification/labeling of the nerve in the image data. For instance, of the segmentation data can be used to identify the lateral nerve roots and the foraminal nerve roots. The image data can include one or more axial, sagittal, and/or coronal images that provide a view of the nerve through the opening between the adjacent vertebrae. At 556b, the nerve roots can be tracked from a first part of the nerve root to a second part of the nerve root (e.g., from the lateral recess to the vertebral foramen). Tracking of the nerve root can be an indicator of the presence or absence and/or the severity of stenosis. For example, discontinuities in the nerve roots, narrowed sections of the nerve roots, etc. can be indicative of spinal stenosis. In some embodiments, a location or a severity of the stenosis can be assessed, e.g., based on one or more regions including a discontinuity or narrowed section of a nerve root.

At 557, a spinal analysis model can be used to assess the presence and/or severity of stenosis. For example, the spinal analysis model can output a prediction of whether there is stenosis and/or the severity of the stenosis based on the anatomical feature analysis performed at 554. For example, threshold ranges and/or threshold values can be associated with different classes of stenosis (e.g., no stenosis, mild stenosis, severe stenosis) and/or grades of stenosis (Grade I - IV). As an example, if the surface area of the spinal cord and/or the thecal sac (e.g., determined at 555b) is below a first threshold value, then the spinal analysis model may classify the selected image and/or the ROI as severe stenosis. However, if the surface area of the spinal cord and/or the thecal sac (e.g., determined at 555b) is above the first threshold value but below a second threshold value, then the spinal analysis model may classify the selected image and/or the ROI as mild stenosis. Alternatively, if the surface area of the spinal cord and/or the thecal sac (e.g., determined at 555b) is above the second threshold value, then the spinal analysis model may classify the selected image and/or the ROI as no stenosis. As another example, if the tracked nerve roots (e.g., tracked at 556b) are indicative of pinched nerve roots (e.g., with one or more discontinuities in tracking the nerve roots and/or narrowing of the nerve roots to diameters that fall within certain predefined ranges), then the spinal analysis model may classify the selected image and/or the ROI as having spinal stenosis.

At 558, the stenosis assessment (e.g., output of spinal analysis model at 557) can be associated with one or more vertebral levels, e.g., based on the output of the level identification process 530 in FIG. 6A and/or manual identification of vertebral levels. As described above with reference to 530, a level identification model (e.g., trained CNN 300 in FIG. 3B) can assign a vertebral level or ordinal identifier (e.g., C1-S5, or C1-C7, TH1-TH12, L1-L5(L6), and/or S1-S5) to portions of the image data. The spinal analysis model (e.g., output of the spinal assessment process 540 in FIG. 6B and/or output of the spinal analysis model at 557) can assign a deformity assessment (e.g., no stenosis, mild stenosis, and/or severe stenosis) to the selected image and/or the ROI based on the anatomical feature analysis at 554. The spinal deformity assessment can be associated with the relevant vertebral levels, at 558. For example, in FIG. 9A, the ordinal identifier "L5-S1" is associated with deformity assessment "mild stenosis" (e.g., assessed based on the surface area of the spinal cord). The stenosis assessment can then end.

While not depicted as an iterative process, the stenosis assessment process 550 in FIG. 7A can iterate from 551-558 if further ROIs and/or anatomical parts need to be analyzed for stenosis.

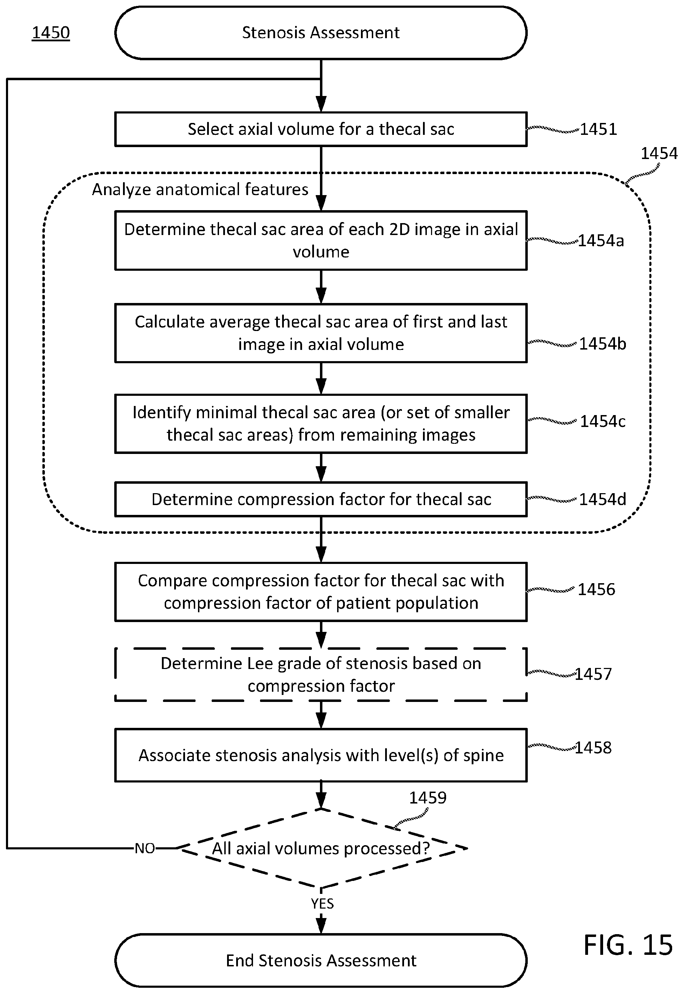

In some embodiments, quantitative evaluation of spinal stenosis can be performed using morphometric quantitative measurements, including, for example, the thecal sac compression factor. FIG. 15 is a flow chart of a stenosis assessment process 1450, according to some embodiments. The stenosis assessment process 1450 can be used in combination with or in lieu of the stenosis assessment process 550, described above with reference to FIG. 7A. As depicted in FIG. 15, the stenosis assessment process can include selecting an axial volume of a thecal sac (or intervertebral disc block including the thecal sac) of a patient for analysis, at 1451. The axial volume can be, for example, the axial volumes that are obtained via the level identification process 1130 described with reference to FIG. 12 above. At 1454, one or more anatomical features of the thecal sac and/or surrounding anatomy may be analyzed. In some embodiments, such analysis can include determining a compression factor for the thecal sac. To determine the compression factor, the thecal sac surface area of each 2D image in the axial volume can be determined, at 1454a, and the following calculation is performed:

$$c_f = \frac{A_{TS_{min}}}{\dfrac{A_{TS_1} + A_{TS_{-1}}}{2}} \quad (Eq.\,1)$$

where $c_f$ is the compression factor for the thecal sac, $A_{TS_{min}}$ is the minimum thecal sac surface area of the internal images of the axial volume (i.e., images of the axial volume that are not the first (top) and last (bottom) images), $A_{TS_1}$ is the thecal sac surface area for the first image in the axial volume, and $A_{TS_{-1}}$ is the thecal sac surface area for the last image in the axial volume. In some embodiments, the thecal sac surface area can be represented as a number of pixels, while in other embodiments, the surface area can be represented in real metrics (e.g., $mm^2$).

In particular, the process of determining the compression factor for the thecal sac can include calculating the average thecal sac surface area of the first and last images in the axial volume, at 1454b, identifying a minimal or smallest thecal sac surface area (or a set of smaller thecal sac surface areas) among the remaining images (i.e., those images in the middle of the axial volume), at 1454c, and then comparing the minimal thecal sac surface area to the average thecal sac surface area of the first and last images (e.g., by calculating a ratio of the two), at 1454d. Typically, compression of an thecal sac occurs in the middle of an intervertebral level, where the intervertebral disc with age and wear can bulge and create pressure on the thecal sac. Conversely, the boundary of the thecal sac, as captured by the boundary images (i.e., first and last images of the axial volume) are usually undamaged or les damaged, as they are taken on or close to the adjacent bony structures. Therefore, the average thecal sac surface area for the first and last images of the axial volume can provide a good approximation of the expected surface area for a more central portion of the thecal sac (as represented by the middle images of the axial scan). Accordingly, Eq. 1 as set forth above can be used to provide a quantitative measure of how much the smallest intervertebral level thecal sac surface area differs from the expected thecal sac surface area.

At 1456, the compression factor for the thecal sac can be compared with the compression factors of a patient population. In some embodiments, the patient population can be a general patient population. Alternatively, the patient population for comparison can be one that shares similar attributes to the patient being examined, e.g., by belonging to the same age group and/or having the same sex, height, weight, ethnicity, and/or other shared attributes. The comparison to the patient population can provide objective data to a clinician, while not providing a direct assessment of a classification of the stenosis condition.

Optionally, at 1457, a grade or classification can be assigned to the patient's spinal condition, e.g., based on the Lee grading system. For example, different thresholds can be set that correlate different ranges of the compression factor to the grades or classifications of the Lee grading system. In some embodiments, the different thresholds can be set based on analyzing the compression factors of thecal sacs that are associated with known Lee grades. For example, a training dataset can be obtained that includes axial volumes of multiple thecal sacs that have different degrees or severity of stenosis, and each of those thecal sacs can be associated with a known Lee grade. The compression factors of each of the axial volumes can be determined (e.g., by a compute device as described herein), and then based on the respective Lee grades of the axial volumes, enable a correlation (e.g., a linear or non-linear regression or other relationship) to be drawn between the compression factors and the Lee grades. Such correlation can then be used to set different thresholds or ranges that correspond to each of the Lee grades.

At 1458, the stenosis analysis, including the compression factor analysis and/or classification of the stenosis, can be associated with one or more levels of the spine. For example, the stenosis analysis can be associated with the ordinal identifiers of the two vertebrae that bound the intervertebral disc (e.g., L5-S1, L4-L5, L3-L4, L2-L3, L1-L2, T12-L1, etc.). While not depicted, such analysis can also be stored, e.g., in a memory.

If all the axial volumes have been processed (1459: YES), then the process can end. If additional axial volumes still need to be processed (1459: NO), then the process can iterate back through the steps with another axial volume for a different level. Once all of the axial volumes for the thecal sacs have been analyzed, the process can continue to visualizing the stenosis analysis. For example, in some embodiments, the process can continue to 580, as described above with reference to the high-level flow shown in FIG. 5.

b) Disc Degeneration Assessment

Disc degeneration can be caused by dehydration of annulus fibrosis and/or dehydration of nucleus which can lead to loss of structural and functional integrity of the intervertebral disc.

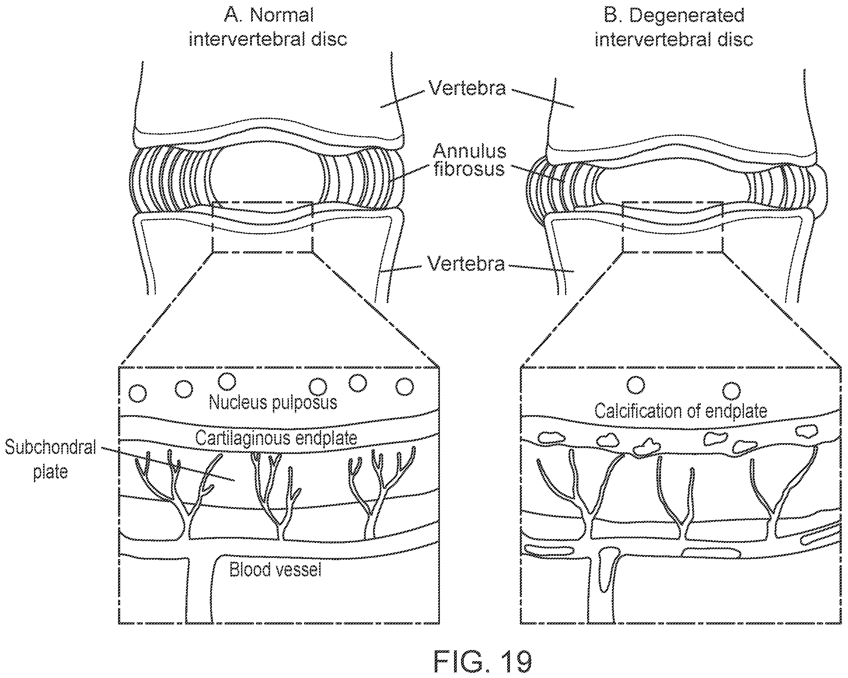

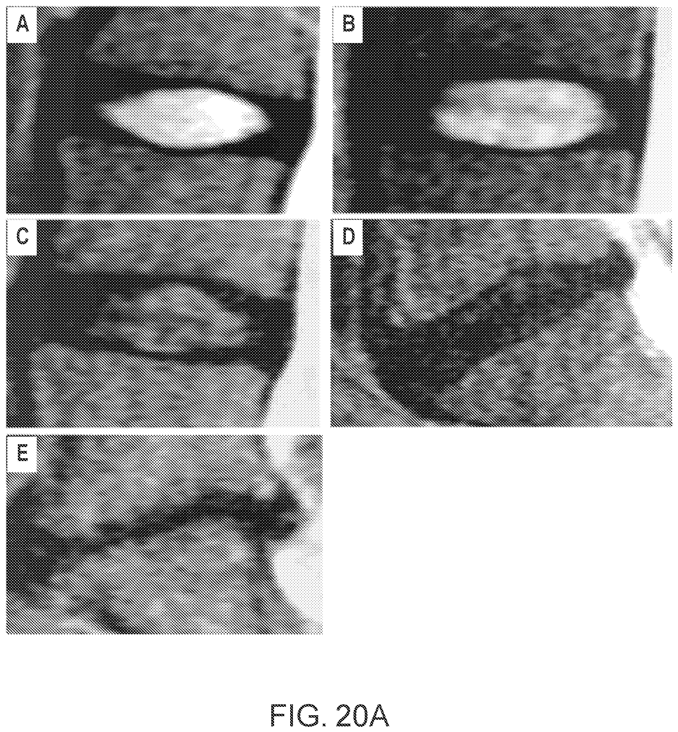

FIG. 19 provides an illustration of spinal anatomy, under normal conditions (left) and with degenerated disc disease (right). As shown, with degenerative disc disease, the nucleus dehydrates, which can lead to endplate calcification and a decrease in nutrients being transporting through the tissue. FIG. 20A depicts a grading system for classification of disc degeneration developed by Pfirrmann et al. ("Pfirrmann grading system"). The Pfirrmann grading system defines various qualitative metrics for classifying different cases of intervertebral disc degeneration. The classification involves five grades, which are based on the homogeneity of the intervertebral disc space and the brightness of the signal intensity in MRI images (e.g., T2-weighted images). Under the Pfirrmann grading system, the following qualitative characteristics are associated with each grade:

- Grade I (example A in FIG. 20A): The structure of the disc is homogeneous, with a bright hyperintense white signal intensity and a normal disc height.

- Grade II (example B in FIG. 20A): The structure of the disc is inhomogeneous, with a hyperintense white signal. The distinction between nucleus and annulus is clear, and the disc height is normal, with or without horizontal gray bands.

- Grade III (example C in FIG. 20A): The structure of the disc is inhomogeneous, with an intermediate gray signal intensity. The distinction between nucleus and annulus is unclear, and the disc height is normal or slightly decreased.

- Grade IV (example D in FIG. 20A): The structure of the disc is inhomogeneous, with an hypointense dark gray signal intensity. The distinction between nucleus and annulus is lost, and the disc height is normal or moderately decreased.

- Grade V (example E in FIG. 20A): The structure of the disc is inhomogeneous, with a hypointense black signal intensity. The distinction between the nucleus and annulus is lost, and the disc space is collapsed.

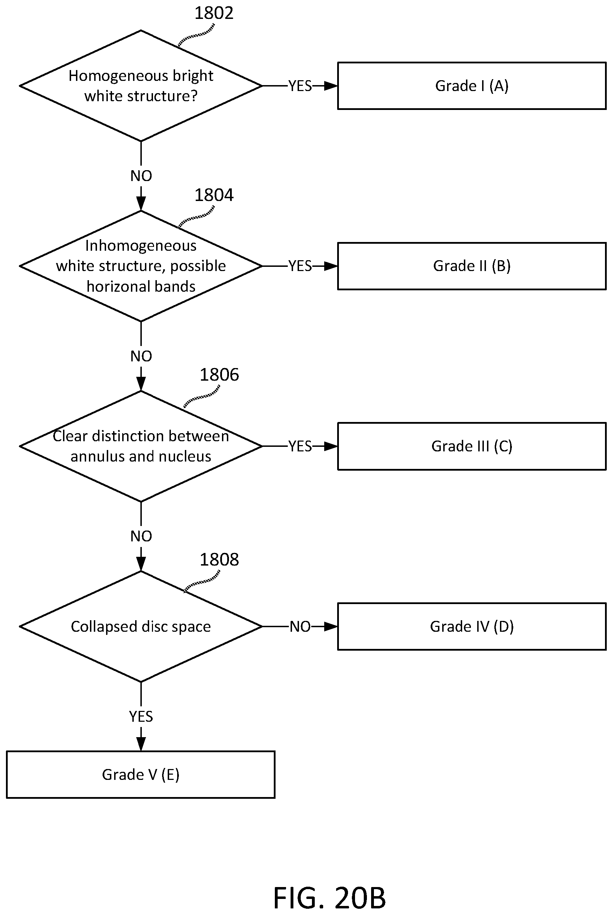

FIG. 20B depicts an example flow for assigning a Pfirrmann grade to an intervertebral disc, e.g., based on viewing a MRI scan of the intervertebral disc. At 1802, a physician can consider whether there is a homogeneous bright white structure. If yes, then the intervertebral disc can be classified as Grade I. If no, then at 1804, the physician can consider whether there is an inhomogeneous white structure, with or without one or more horizontal bands. If yes, then the intervertebral disc can be classified as Grade II. If no, then at 1806, the physician can consider whether there is a clear distinction between annulus and nucleus. If yes, then the intervertebral disc can be classified as Grade III. If no, then the physician can consider whether the disc space has collapsed. If no, then the intervertebral disc can be classified as Grade IV. If yes, then the intervertebral disc can be classified as Grade V.

Nevertheless, identification of disc degeneration by surgeons is subjective and can be inconsistent. Even with grading systems such as the Pfirrmann grading system, different surgeons viewing the same MRI scans and using the same grading system may arrive at different conclusions and therefore recommend different clinical interventions. As such, systems, devices, and methods described herein, by taking a more quantitative approach (e.g., in evaluating a ratio of degeneration and/or a distance between adjacent intervertebral discs), can provide a more reliable assessment of disc degeneration.

FIG. 7B is a flow chart of a disc degeneration assessment process 560, according to some embodiments. At 561, ROIs can be selected in the image data (e.g., similar to 531 in FIG. 6A and 541 in FIG. 6B). The ROIs can include one or more anatomical parts of interest, e.g., one or more vertebrae and surrounding intervertebral discs and/or other structures. At 562, 2D images associated with the ROIs can be selected (e.g., similar to 532 in FIG. 6A and 542 in FIG. 6B). These images can include axial views of the patient anatomy, sagittal view of the patient anatomy, coronal views of the patient anatomy, and/or a combination thereof. Optionally, at 563, these 2D images can be combined to generate 3D volumes or sub-volumes for further analysis (e.g., similar to 533 in FIG. 6A and 543 in FIG. 6B).

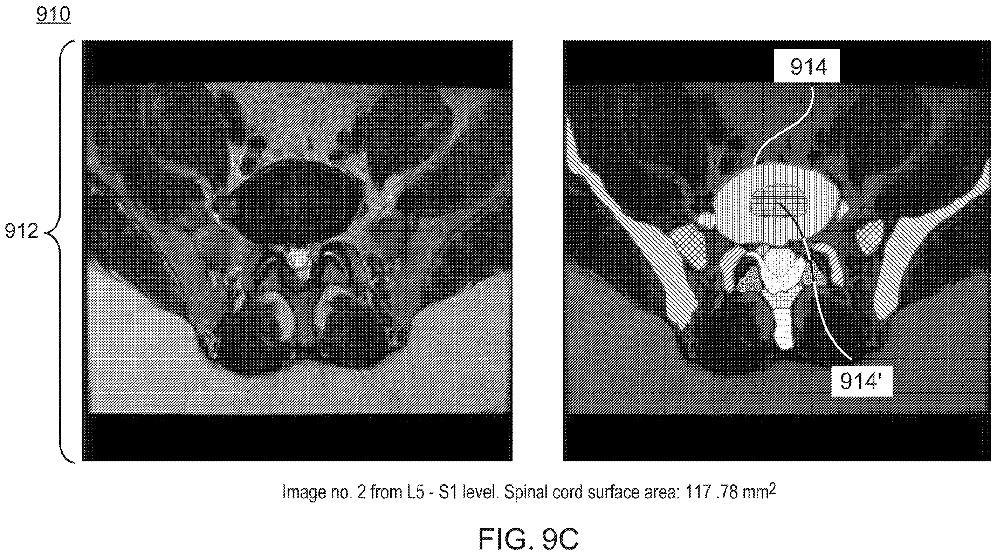

At 564, one or more anatomical features of a patient's anatomy in the selected image data can be analyzed (e.g., similar to 544 in FIG. 6B). In some embodiments, at 565a, an intervertebral disc can be identified in the selected image data, e.g., based on the output of the segmentation model(s), at 520, or manual identification/labeling of the intervertebral disc. As an example, the selected image data can be axial images of a portion of the spine (e.g., axial scans of C1-C2, ... C6-C7, C7-TH1, TH1-TH2, TH2-TH3, .... TH11-TH12, TH12-L1, L2-L3, L4-L5, L5-S1, ... S6-S7, etc.). Portions of the intervertebral disc (e.g., the annulus fibrosis and/or the nucleus) can be segmented in the axial images by the segmentation model. FIGS. 9A-9E illustrate example axial scans and/or axial images of the portion of the spine L5-S1 for a patient with the intervertebral disc segmented in the images. As depicted in FIG. 9C, 914 represents the intervertebral disc annulus and 914' represents the nucleus. At 565b, the shape, area, or other geometric properties of the intervertebral disc and/or components thereof (e.g., the intervertebral disc, the annulus fibrosis, and/or the nucleus) can be determined from the selected images. The shape and/or the area of the intervertebral disc can be indicative of the presence or absence and/or the severity of disc degeneration. For example, a narrower or smaller area of the nucleus (e.g., 914' in FIG. 9C) can be indicative of a more severe disc degeneration.

Additionally or alternatively, at 566a, upper vertebrae and lower vertebrae around an intervertebral disc can be identified in the selected image data, e.g., based on the output of the segmentation model(s), at 520, or manual identification/labeling of the vertebrae. At 566b, the distance between an upper (superior) vertebrae and a lower (inferior) vertebrae of an intervertebral disc (i.e., two adjacent vertebrae of the intervertebral disc) can be determined. This distance can be indicative of the presence or absence and/or the severity of disc degeneration. In particular, disc degeneration can lead to reduction in height of the intervertebral disc, thereby reducing the distance between the upper vertebrae and the lower vertebrae. Therefore, the distance between the upper vertebrae and the lower vertebrae can be indicative of the presence or absence and/or the severity of disc degeneration.

At 567, a spinal analysis model can be used to assess the presence and/or severity of disc degeneration. For example, the spinal analysis model can output a prediction of whether there is disc degeneration and/or a severity of the disc degeneration based on the anatomical feature analysis performed at 564. For example, threshold ranges and/or threshold values can be associated with the severity of disc degeneration. As an example, if the height of the intervertebral disc and/or the distance between two adjacent vertebrae is below a threshold value, then the spinal analysis model may classify the selected image and/or the ROI as having disc degeneration. Similarly, if the area of the nucleus of the intervertebral disc (e.g., 914' in FIG. 9C) is below a threshold value, then the spinal analysis model may classify the selected image and/or the ROI as having disc degeneration.

At 568, the disc degeneration assessment (e.g., output of spinal analysis model at 567) can be associated with one or more vertebral levels, e.g., based on the output of the level identification process 530 in FIG. 6A and/or manual identification of vertebral levels. As described above with reference to 530, a level identification model (e.g., trained CNN 300 in FIG. 3B) can assign a vertebral level or ordinal identifier (e.g., C1-S5, or C1-C7, TH1-TH12, L1-L5(L6), and/or S1-S5) to portions of the image data. The spinal analysis model (e.g., output of the spinal assessment process 540 in FIG. 6B and/or output of the spinal analysis model at 567) can assign a deformity assessment (e.g., no disc degeneration, mild disc degeneration, and/or severe disc degeneration) to the selected image and/or the ROI based on the anatomical feature analysis at 564. The spinal deformity assessment can be associated with the relevant vertebral levels, at 568. The disc degeneration assessment can then end.

While not depicted as an iterative process, the disc degeneration assessment process 560 in FIG. 7B can iterate from 561-568 if further ROIs and/or anatomical parts need to be analyzed for disc degeneration.

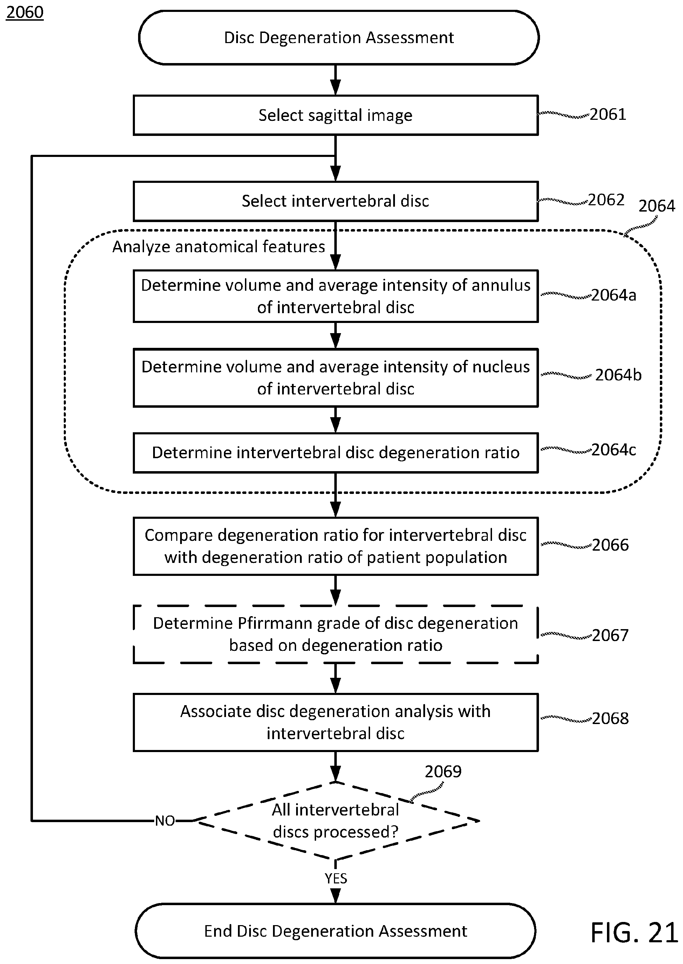

In some embodiments, quantitative evaluation of disc degeneration can be performed using morphometric quantitative measurements, including, for example, an intervertebral disc degeneration ratio. FIG. 21 is a flow chart of a disc degeneration assessment process 2060, according to embodiments. The disc degeneration assessment process 2060 can be used in combination with or in lieu of the disc degeneration process 560, described above with reference to FIG. 7B. As depicted in FIG. 21, the process 2060 can include selecting a sagittal image (or a set of sagittal images or sagittal volume) of a patient for analysis, at 2061. The sagittal image can show a range of intervertebral levels, e.g., suitable for evaluating different regions of the spine. For example, for evaluating the lower back, a sagittal image showing the range S1-T12 can be selected. At 2062, a first intervertebral disc shown in the sagittal image can be selected for analysis. In some embodiments, the segmentation data and/or level identification data for the sagittal image can be loaded, and such data can be used to identify each intervertebral level. The intervertebral levels can then be evaluated individually, e.g., by starting at one end of the intervertebral range (e.g., S1) and going to the other end of the intervertebral range (e.g., T12).

At 2064, one or more anatomical features of the intervertebral disc and/or surrounding anatomy may be analyzed. In some embodiments, such analysis can include determining an intervertebral disc degeneration ratio. To determine the intervertebral disc degeneration ratio, an average intensity and real volume of the annulus of the intervertebral disc can be determined, at 2064a, and an average intensity and real volume of the nucleus of the intervertebral disc can be determined, at 2064b, and then the following calculation can be performed, at 2064c:

$$IVD_{degen} = \frac{V_n \times I_n}{V_a \times I_a} \quad (Eq.\,2)$$

where $IVD_{degen}$ is the intervertebral disc degeneration ratio, $V_n$ is the intervertebral disc nucleus volume, $I_n$ is the intervertebral disc nucleus mean intensity, $V_a$ is the intervertebral disc anulus volume, and $I_a$ is the intervertebral disc annulus mean intensity.

As represented by Eq. 2, the intervertebral disc degeneration ratio or factor is based on the annulus and nucleus volumetric and intensity calculations. These calculations can be based on the sagittal image data and the segmentation data. For each intervertebral level, the volume and average intensity of the nucleus and annulus can be determined and used in Eq. 2 to determine the disc degeneration ratio.

Similar to the compression factor analysis described above with reference to FIG. 15, the intervertebral disc degeneration ratio can be compared with the intervertebral disc degeneration ratios of a patient population, at 2066. In some embodiments, the patient population can be a general patient population. Alternatively, the patient population for comparison can be one that shares similar attributes to the patient being examined, e.g., by belonging to the same age group and/or having the same sex, height, weight, ethnicity, and/or other shared attributes. The comparison to the patient population can provide objective data to a clinician, while not providing a direct assessment of a classification of the disc degeneration condition.

Optionally, at 2067, a grade or classification can be assigned to the patient's spinal condition, e.g., based on the Pfirrmann grading system. For example, different thresholds can be set that correlate different ranges of the intervertebral disc degeneration ratio to the grades or classifications of the Pfirrmann grading system. In some embodiments, the process for assigning such thresholds can be similar to the classification process described with respect to the spinal stenosis analysis above, at 1457 of process 1450 depicted in FIG. 15.

At 2068, the disc degeneration analysis, including the intervertebral disc degeneration ratio analysis and classification of the disc degeneration, can be associated with the intervertebral disc. For example, the disc degeneration analysis can be associated with the ordinal identifiers of the two vertebrae that bound the intervertebral disc (e.g., L5-S1, L4-L5, L3-L4, L2-L3, L1-L2, T12-L1, etc.). While not depicted, such analysis can also be stored, e.g., in a memory.

If all the intervertebral discs have been analyzed (2069: YES), then the process can end. If additional intervertebral discs still need to be analyzed (2069: NO), then the process can iterate back through the steps with another intervertebral disc. Once all of the intervertebral discs in the sagittal image or images have been analyzed, the process can continue to visualizing the disc degeneration analysis. For example, in some embodiments, the process can continue to 580, as described above with reference to the high-level flow shown in FIG. 5.

5. Visualization of Spinal Analysis

FIG. 8 illustrates an example report 800 generated by systems and methods described herein, according to some embodiments. The report 800 can include patient information 802 such as age, past spinal deformity information, etc. Spinal deformity diagnosis 804 can be generated based on level identification process (e.g., level identification process 530 in FIG. 6A), and spinal deformity assessment process (e.g., spinal deformity assessment process 540 in FIG. 6B, stenosis assessment process 550 in FIG. 7A, and/or disc degeneration assessment process 560 in FIG. 7B). For example, the level identification process can generate vertebral level or ordinal identifier (e.g., C1-S5, or C1-C7, TH1-TH12, L1-L5(L6), and/or S1-S5) such as first level 806a and second level 806b in the report 800. The spinal deformity assessment process can generate spinal deformity diagnosis (e.g., no stenosis, mild stenosis, moderate stenosis, severe stenosis, no disc degeneration, mild disc degeneration, moderate disc degeneration, severe disc degeneration, no deformity, mild deformity, and/or severe deformity) such as diagnosis 808a and diagnosis 808b in the report. The output of the level identification process can be associated with the output of the spinal deformity process. For example, first level 806a can be associated with diagnosis 808a, thereby indicating that the diagnosis was performed on the first level using the image data. Similarly, second level 806b can be associated with diagnosis 808b.

In some embodiments, the report 800 can include the output of anatomical feature analysis 810 (e.g., analysis 544 in FIG. 6B, analysis 554 in FIG. 7A, and analysis 564 in FIG. 7B). For example, the report 800 can include surface area of spinal cord and/or thecal sac, area of intervertebral disc, distance between adjacent vertebrae, etc. The anatomical feature analysis such as 812a and 812b in FIG. 8 can also be associated with the vertebral level or ordinal identifier such as 806a and 806b.

In some embodiments, the report 800 can include a virtual representation of the patient's anatomy 820. For example, the report 800 can include 3D views and/or 2D views of the patient's anatomy. In some embodiments, these 3D views and/or 2D views of the patient anatomy can be used to provide a virtual or augmented reality image for display by a computer-assisted surgical system. In such systems, a virtual 2D or 3D view of the patient's anatomy can be displayed over a real portion of the patient anatomy (e.g., a surgical site). In some embodiments, such virtual or augmented reality representation of the patient's anatomy can include information 822 relating to the spinal deformity diagnosis (e.g., diagnosis 808a and 808b) and/or anatomical feature analysis (e.g., analysis 812a and 812b).

FIGS. 9A-9E depict portions of an example report 900 generated for a patient with mild stenosis. While the portions of the example report are shown in separate figures, it can be appreciated that all portions (or one or more portions) of the report can be presented together, e.g., on a single page, screen, or other interface. The report includes the spinal deformity diagnosis 904 (e.g., similar to spinal deformity diagnosis 804). The vertebral level or ordinal identifier (e.g., similar to level 806a and 806b) "L5-S1" is associated with spinal deformity diagnosis (e.g., similar to diagnosis 808a and 808b) "Mild spinal stenosis." The report can include several images of the anatomical structure and/or the ROI. The anatomical feature analysis (e.g., similar to analysis 812a an 812b) can be performed on each image. The analysis for each image can be listed as 911. Additionally, the analysis (e.g., similar to analysis 812a an 812b) for each image can be associated with the vertebral level or ordinal identifier (e.g., similar to level 806a and 806b). For example, for image number 0, the analysis "Spinal cord surface area: 126.78 mm$^2$" 912a can be associated with the ordinal identifier "L5-S1." Although, in FIGS. 9A-9E, the anatomical feature analysis performed is area of the spinal cord, it should be readily understood that any suitable analysis can be performed to determine spinal deformity. For example, the compression factor, area of the intervertebral disc 914 and/or the area of the nucleus 914' can be determined to diagnose disc degeneration in the patient.

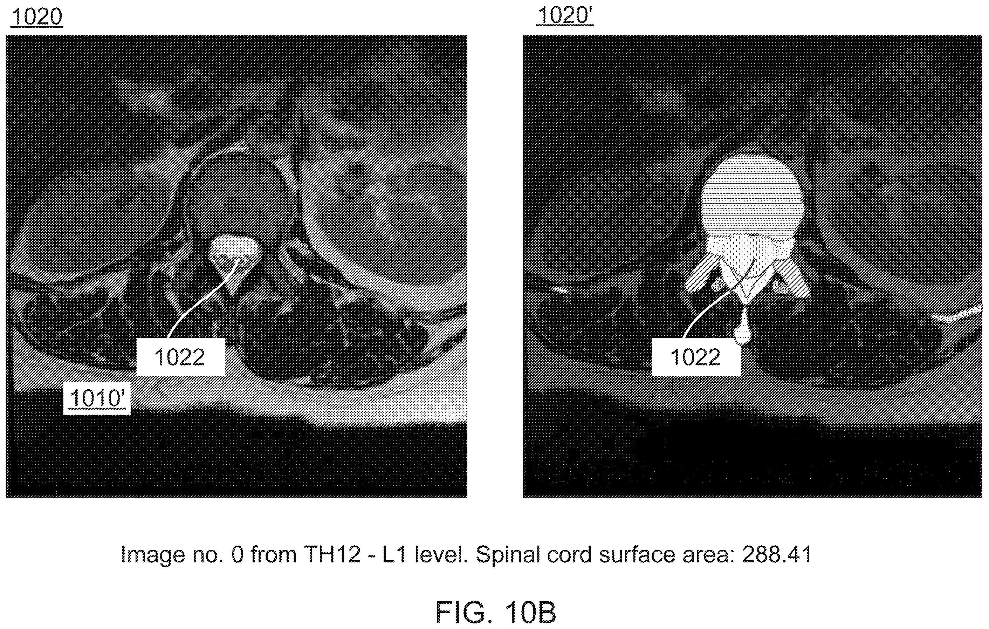

FIG. 10A illustrates an example diagnosis of severe stenosis in a patient. The left image 1000 depicts a scan 1010 of the patient anatomy, and the right image 1000' depicts the scan 1010 with segmentation data overlaying the scan. The anatomical feature analysis (e.g., similar to analysis 812a an 812b) for scan 1010 produces a spinal cord area of 56.25 mm$^2$. As discussed above, an extremely narrow spinal cord area can be indicative of severe stenosis. This analysis is associated with ordinal identifier (e.g., similar to level 806a and 806b) "L5-S1." FIG. 10B illustrates an example diagnosis of no stenosis in a patient. The left image 1020 depicts a scan 1010' of the patient anatomy, and the right image 1020' depicts the scan 1010' with segmentation data overlaying the scan. The anatomical feature analysis (e.g., similar to analysis 812a an 812b) for scan 1010' produces a spinal cord area of 288.41 mm$^2$. This area can be indicative of no stenosis since there spinal cord is not narrow. This analysis is associated with ordinal identifier (e.g., similar to level 806a and 806b) "TH12-L1."

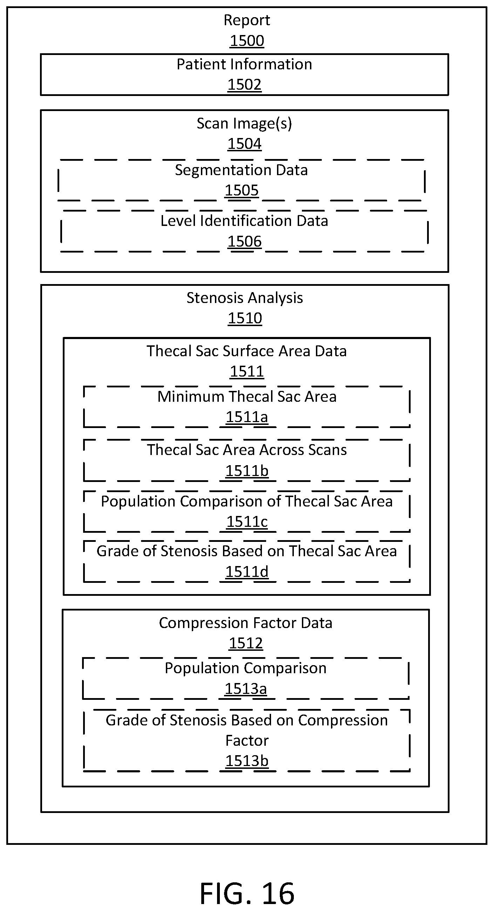

In some embodiments, systems and methods described herein can generate a stenosis report, including information associated with analyzing one or more axial volumes associated with one or more levels of the spine. FIG. 16 depicts an example stenosis report 1500, according to embodiments. The stenosis report 1500 can include elements that are similar to that of report 800. For example, the stenosis report 1500 can include patient information 1502. The stenosis report 1500 can also include scan image(s) 1504, such as, for example, 2D images or scans of a patient's anatomy. In some embodiments, the 2D images can optionally be depicted with segmentation data 1505 and/or level identification data 1506. For example, the 2D images can include segmentation data such as labels that identify different anatomical parts of interest and/or level identification data 1506 such as labels that identify different vertebral or intervertebral levels. In some embodiments, the 2D images can be specific images that are selected from 3D volumetric image data that show more severe cases of stenosis (e.g., Lee grade 2 of grade 3). The 2D images can be automatically selected by a compute device (e.g., compute device 110, 210) upon analyzing one or more image sets, or the 2D images can be manually selected by a physician after viewing the stenosis analysis. In some embodiments, a plurality of 2D images can be depicted in the stenosis report 1500, while in other embodiments, a single 2D image is depicted at a time. In some embodiments, a spatial sequence of 2D images can be depicted, such as, for example, all the images in an axial volume that has been determined to have or classified as having a more severe case of stenosis.

The stenosis report 1500 can also include stenosis analysis 1510. Stenosis analysis can include thecal sac surface area data 1511 and/or compression factor data 1512. The thecal sac surface area data 1511 can include, for example, a minimum thecal sac surface area 1511a (e.g., the smallest thecal sac surface area or a set of smaller thecal sac surface areas of one or more axial volumes including a thecal sac), a thecal sac surface area across a set of scans 1511b (e.g., the thecal sac surface area for all or a subset of scans within one or more axial volumes including a thecal sac, the average thecal sac surface area for all or a subset of scans within one or more axial volumes including a thecal sac, the range of thecal sac surface areas for all or a subset of scans within one or more axial volumes including a thecal sac, and/or other quantitative measures of thecal sac surface area across a plurality of scans), a comparison of the thecal sac surface area of the patient to the average values of a population 1511c (e.g., the percentile of the patient's thecal sac surface area for one or more levels of the spine compared to the population's thecal sac surface area), and/or a grade or classification of stenosis based on the thecal sac surface area 1511d (e.g., the Lee grade of one or more thecal sacs based on the thecal sac surface areas of their respective axial volumes).

The compression factor data 1512 can include a comparison of the compression factor of the patient to the values of a population 1513a (e.g., the percentile of the patient's compression factor for one or more thecal sacs compared to the population's compression factor), and/or a grade or classification of stenosis based on the compression factor 1513b (e.g., the Lee grade of one or more thecal sacs based on the compression factor of their respective axial volumes).

The quantitative metrics 1511a, 1511b, 1511c, 1511d, 1513a, 1513b and/or other metrics included in the report can be presented in textual format, graphical format (e.g., plots, bar graphs, etc.) and/or other suitable formats. In some embodiments, the stenosis report 1500 can be interactive, and a user (e.g., a physician) can select from a menu of options to update the report and/or reorganize the content within the report. For example, a user can select to hide one or more metrics while showing other metrics. Alternatively, a user can select to show a first set of 2D scans, and then select to show a second set of 2D scans. In some embodiments, a user can select to reduce or increase the size of certain information being presented. For example, the user can select to focus in on a portion of a 2D scan and/or zoom in or out on a graph or plot. The user can select what is presented using a keyboard, mouse, touchscreen, audio device, and/or other input device.

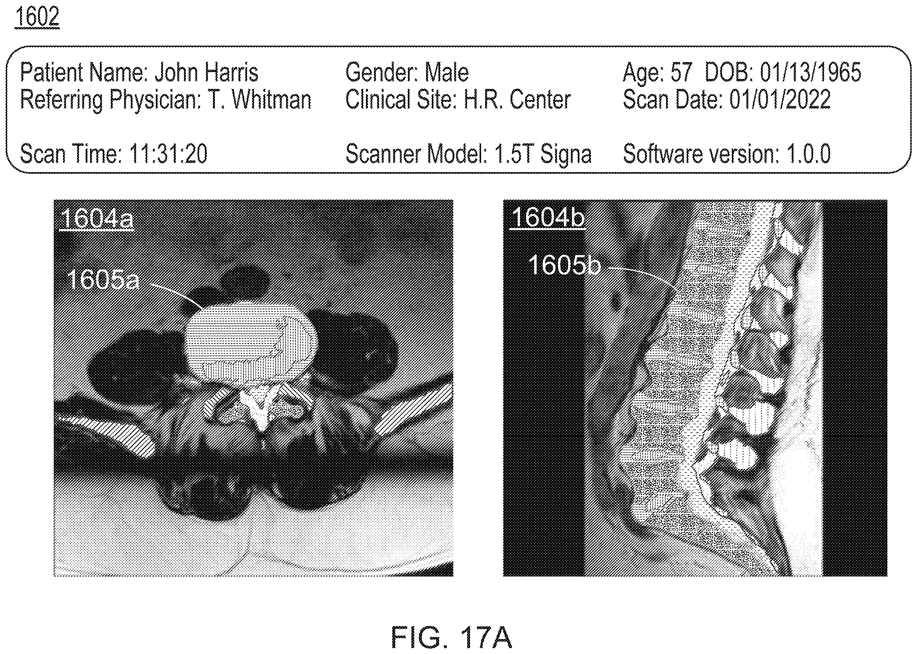

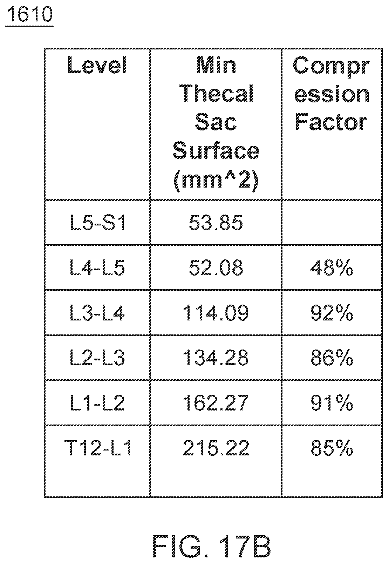

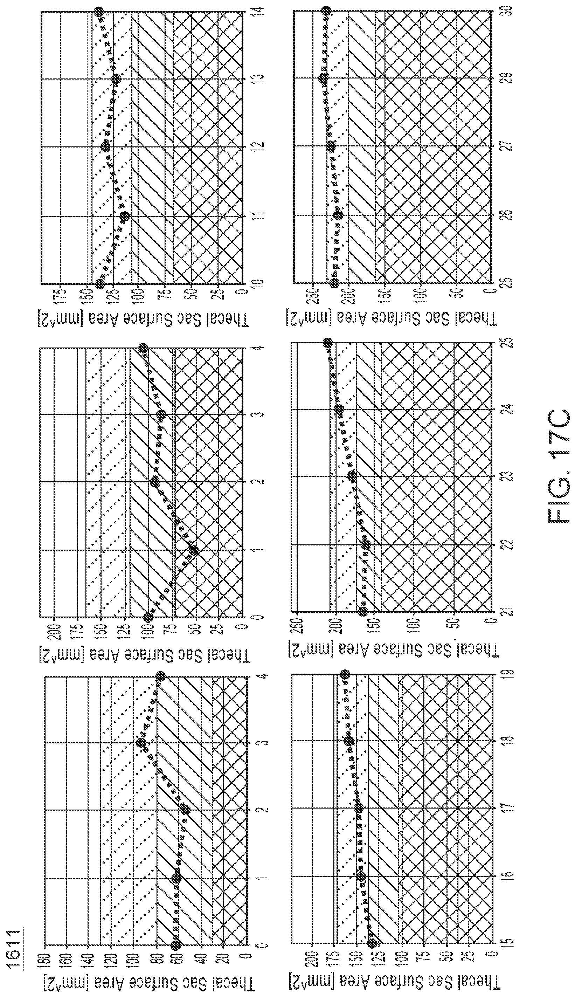

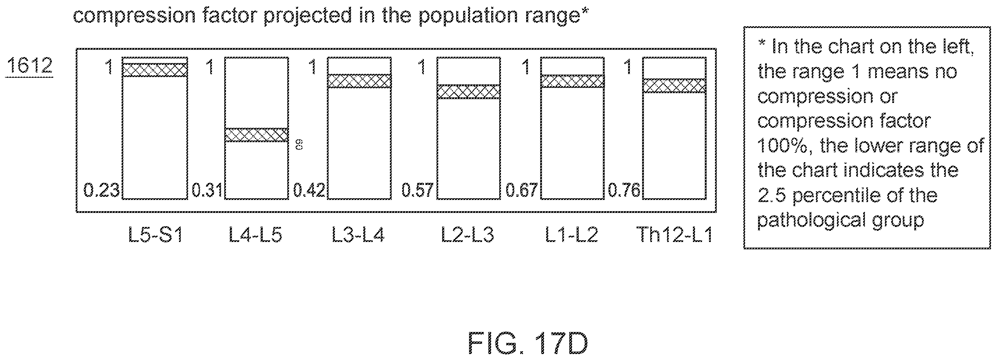

FIGS. 17A-17D depict portions of an example stenosis report, according to embodiments. While the portions of the stenosis report are shown in separate figures, it can be appreciated that all portions (or one or more portions) of the stenosis report can be presented together, e.g., on a single page, screen, or other interface. The stenosis report can include patient information 1602, such as, for example, a patient's name, gender, age, and date of birth, as well as information about the patient's physician and the patient's scans (e.g., the scan date, scan time, scanner model, etc.). The stenosis report can also include scan images 1604a, 1604b. The scan image 1604a can be an axial scan that shows the minimum thecal sac area, which corresponds to an area of high compression. The scan image 1604b can be a sagittal scan that shows the full region of interest of the spine (e.g., L5 to S1). Segmentation data 1605a, 1605b can also be shown in each scan image 1604a, 1604b. The stenosis report can also include a table 1610 that shows the quantitative metrics associated with a stenosis analysis. The table 1610 can identify the intervertebral levels being analyzed (e.g., L5-S1, L4-L5, etc.) and the corresponding minimum thecal sac surface area and compression factor for each level.

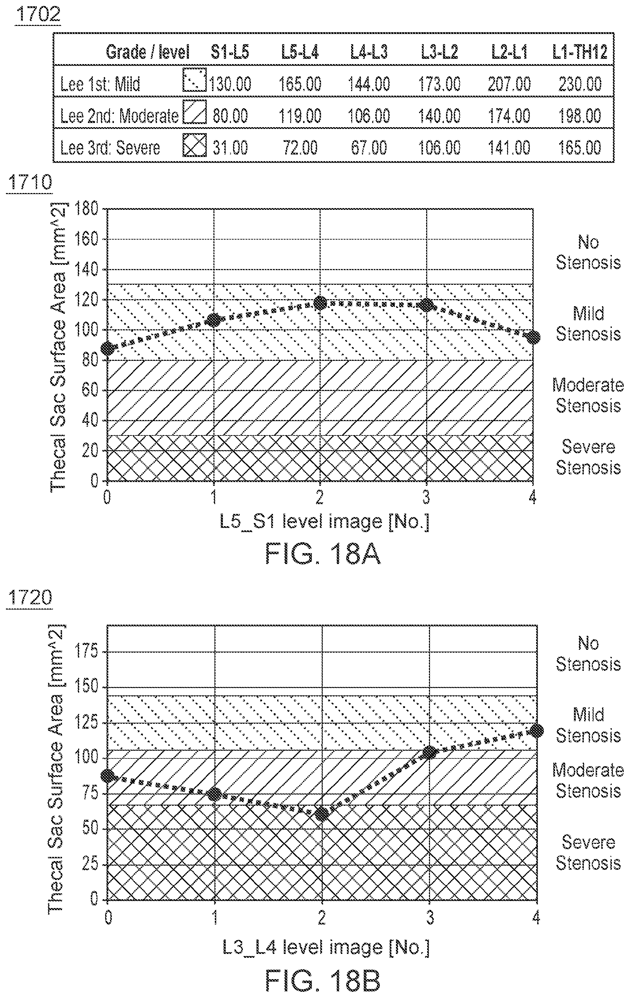

The stenosis report can also include comparison graphs 1611 that depict the thecal sac surface area for each of the intervertebral levels, across the scans of the axial volume for each intervertebral level. Each of the graphs 1611 can correspond to a different intervertebral level. In some embodiments, the graphs 1611 can include color coding that depicts, based on population metrics, when the thecal sac surface area for the patient falls within different classifications or grades of stenosis. For example, FIGS. 18A and 18B depict two detailed views of comparison graphs, according to embodiments. FIG. 18A depicts a comparison graph 1710 that shows the thecal sac surface area (in mm$^2$) across the axial scans for the L5-S1 intervertebral level, and FIG. 18B depicts a comparison graph 1720 that shows the thecal sac surface area (in mm$^2$) across the axial scans for the L3-L4 intervertebral level. For each intervertebral level, different ranges or thresholds can be defined that are associated with different classifications of stenosis. As described above under the stenosis assessment process, these ranges or thresholds can be set based on the thecal sac surface areas of a population with known stenosis grades or classifications. Table 1702 depicts example thresholds that can be set for each of the grades or classifications of stenosis. The thresholds for each intervertebral level are different, as shown in table 1702, as stenosis can present itself in different ways at each level, and therefore the population analysis must be performed at each level to obtain clinical thresholds for classifying the stenosis at each level. The graphs 1710, 1720 can then show the range for each grade or classification of stenosis in a different color, i.e., green for no stenosis, yellow for mild stenosis, orange for moderate stenosis, and red for severe stenosis.

Referring back to FIGS. 17A-17D, the stenosis report can also include a graphic 1612 for comparing the compression factor across the different intervertebral levels. As depicted, the graphic 1612 can include as an upper bound the value 1, which corresponds to no compression. The graphic 1612 can include as a lower bound the compression factor associated with the 2.5 percentile of the pathological group for each level. For example, for the L5-S1 level, the lower bound can be 0.23, which corresponds to the compression factor of the 2.5 percentile of the pathological group. Similarly, the level L4-L5 can include 0.31 as its lower bound, which corresponds to the compression factor of the 2.5 percentile of the pathological group. Each of the lower bounds represent significant compression, and therefore can provide an objective comparison for a physician to evaluate the severity of the spinal stenosis of the patient at each of his intervertebral levels. While the 2.5 percentile is used as the lower bound in this example, it can be appreciated that other lower bounds can be used, e.g., about 1 percentile, about 5 percentile, about 10 percentile, about 20 percentile, including all values and ranges therebetween.

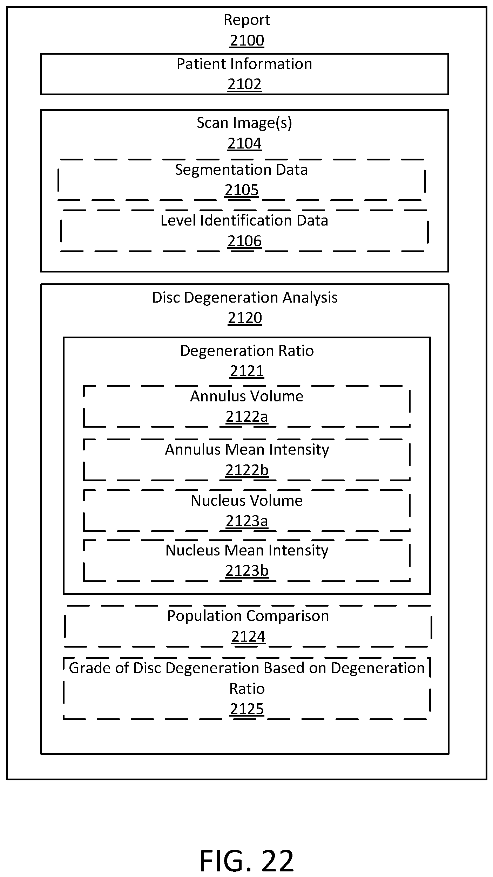

In some embodiments, systems and methods described herein can generate a disc degeneration report, including information associated with analyzing one or more intervertebral discs in a sagittal image or volume. FIG. 22 depicts an example disc degeneration report 2100, according to embodiments. The disc degeneration report 2100 can include elements that are similar to that of reports 800, 1500, described above. For example, the disc degeneration report 2100 can include patient information 2102. The disc degeneration report 2100 can also include scan image(s) 2104, such as, for example, 2D images or scans of a patient's anatomy. In some embodiments, the 2D images can include one or more sagittal images, or portions thereof. For example, systems and devices described herein can autonomously select and present the sagittal image that provides the best or a better view of the intervertebral discs of interest. In some embodiments, a close-up view of a portion of the sagittal image that depicts one or more intervertebral levels having more severe disc degeneration can be shown. Additionally or alternatively, the display of the scan image(s) can be interactive, and a physician can select to zoom in or focus on a portion of a sagittal image that shows one or more intervertebral levels. In some embodiments, the 2D images or scans can optionally be depicted with segmentation data 2105 and/or level identification data 2106. For example, the 2D images can include segmentation data such as labels that identify different anatomical parts of interest and/or level identification data 1506 such as labels that identify different vertebral or intervertebral levels.

The disc degeneration report 2100 can also include disc degeneration analysis 2120. Disc degeneration analysis can include degeneration ratio data 2121 for one or more intervertebral discs, as well as data of the annulus volume 2122b, annulus mean intensity 2122b, nucleus volume 2123a, and nucleus mean intensity 2123b used to determine the degeneration ratio for the one or more intervertebral discs. The disc degeneration report 2100 can also include a comparison of the disc degeneration ratio and/or other metrics (e.g., a distance between adjacent vertebrae) of the patient to the disc degeneration ratios of a population 2124. In some embodiments, such comparison can be a visual that shows the percentile of the patient's disc degeneration ratio within the larger population data. Alternatively or additionally, the numerical percentile of the patient's disc degeneration ratio relative to the population data can be provided.

The quantitative metrics 2121, 2122a, 2122b, 2123a, 2123b, and/or other metrics included in the report can be presented in textual format, graphical format (e.g., plots, bar graphs, etc.) and/or other suitable formats. Similar to the report 1500, the disc degeneration report 2100 can be interactive, e.g., by allowing a user to adapt or update information being presented in the report.

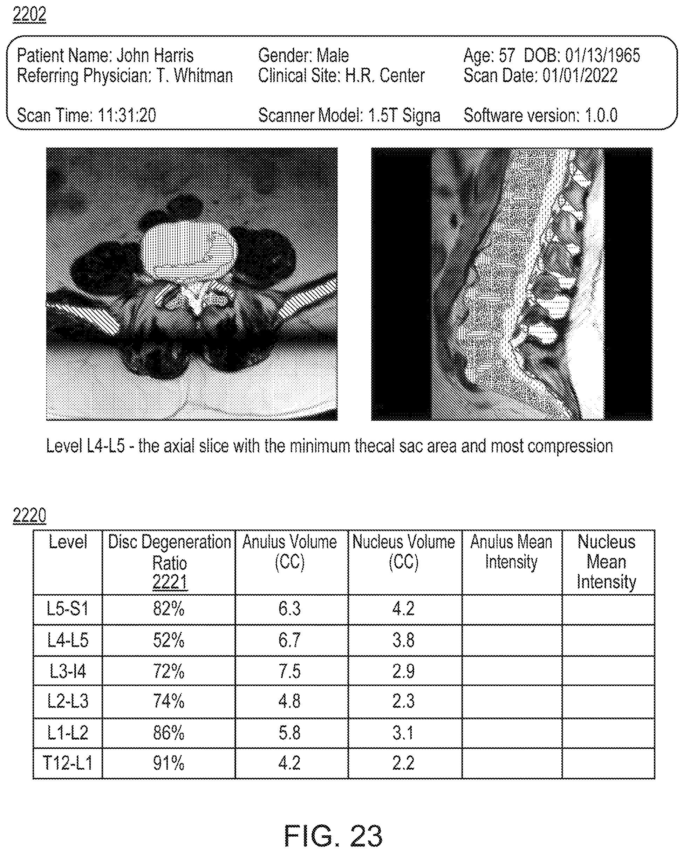

FIG. 23 depicts a specific example of a disc degeneration report, according to embodiments. The disc degeneration report can include patient information 2202, such as, for example, a patient's name, gender, age, and date of birth, as well as information about the patient's physician and the patient's scans (e.g., the scan date, scan time, scanner model, etc.). The disc degeneration report can also include scan images and segmentation data, e.g., similar to that described in FIGS. 17A-17D above. The disc degeneration report can also include a table 2220 that includes certain disc degeneration analysis data, including, for example, the annulus volume in CC, the nucleus volume in CC, and the disc degeneration ratio 2221.

While separate spinal stenosis and disc degeneration reports are described, it can be appreciated that different elements of each can be combined into a single report. In some embodiments, a physician can also interactively update a report, e.g., to show spinal stenosis analysis information and then disc degeneration analysis information, and/or to show different information of each alongside one another.

While various inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto; inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

Also, various inventive concepts may be embodied as one or more methods, of which examples have been provided. The acts performed as part of the methods may be ordered in any suitable way. Accordingly, embodiments may be constructed in which acts are performed in an order different than illustrated, which may include performing some acts simultaneously, even though shown as sequential acts in illustrative embodiments.

As used herein, the terms "about" and/or "approximately" when used in conjunction with numerical values and/or ranges generally refer to those numerical values and/or ranges near to a recited numerical value and/or range. In some instances, the terms "about" and "approximately" may mean within ± 10% of the recited value. For example, in some instances, "about 100 [units]" may mean within ± 10% of 100 (e.g., from 90 to 110). The terms "about" and "approximately" may be used interchangeably.

Any and all references to publications or other documents, including but not limited to, patents, patent applications, articles, webpages, books, etc., presented anywhere in the present application, are herein incorporated by reference in their entirety. Moreover, all definitions, as defined and used herein, should be understood to control over dictionary definitions, definitions in documents incorporated by reference, and/or ordinary meanings of the defined terms.

Some embodiments and/or methods described herein can be performed by a different software (executed on hardware), hardware, or a combination thereof. Hardware modules may include, for example, a general-purpose processor, a field programmable gate array (FPGA), and/or an application specific integrated circuit (ASIC). Software modules (executed on hardware) can be expressed in a variety of software languages (e.g., computer code), including C, C++, Java™, Python, Ruby, Visual Basic™, and/or other object-oriented, procedural, or other programming language and development tools. Examples of computer code include, but are not limited to, micro-code or micro-instructions, machine instructions, such as produced by a compiler, code used to produce a web service, and files containing higher-level instructions that are executed by a computer using an interpreter. For example, embodiments may be implemented using imperative programming languages (e.g., C, Fortran, etc.), functional programming languages (Haskell, Erlang, etc.), logical programming languages (e.g., Prolog), object-oriented programming languages (e.g., Java, C++, etc.) or other suitable programming languages and/or development tools. Additional examples of computer code include, but are not limited to, control signals, encrypted code, and compressed code.

CLAUSES RELATED TO INVENTION(S) DESCRIBED HEREIN

1. A method, comprising:

selecting a region of interest (ROI) in a three-dimensional (3D) volume of image data of a plurality of vertebra of a spine, the ROI including image data of one or more vertebra from the plurality of vertebra and tissue structures surrounding the one or more vertebra;

identifying a spinal cord or thecal sac in the ROI;

determining one or more parameters associated with the spinal cord or thecal sac in the ROI; and

determining a severity of spinal stenosis in the ROI based on the one or more parameters associated with the spinal cord or thecal sac.

2. The method of claim 1, wherein identifying the spinal cord or thecal sac includes:
processing, using a convolutional neural network (CNN) trained to segment the plurality of vertebra and tissue structures surrounding the plurality of vertebra, the image data of the ROI to obtain segmentation data identifying the spinal cord or thecal sac.

3. The method of claim 1, wherein the one or more parameters includes a cross-sectional surface area of the spinal cord or thecal sac.

4. The method of claim 1, wherein the image data of the ROI includes a plurality of axial scans each including a cross-section of the spinal cord or thecal sac, and
determining the one or more parameters of the spinal cord or thecal sac includes determining a surface area of the cross-section of the spinal cord or thecal sac in each axial scan from the plurality of axial scans.

5. The method of claim 4, further comprising:
determining an average measure of the surface areas determined for the cross-sections in the plurality of axial scans.

6. The method of claim 4, further comprising:
generating a plot of the surface areas determined for the cross-sections in the plurality of axial scans.

7. The method of claim 6, wherein the plot includes one or more indicators marking ranges of cross-sectional surface areas of the spinal cord or thecal sac associated with a plurality of different grades of stenosis.

8. The method of claim 7, wherein the plurality of different grades of stenosis includes no stenosis, mild stenosis, moderate stenosis, and severe stenosis.

9. The method of claim 1, wherein the image data of the ROI includes a plurality of axial scans of a vertebrae from the plurality of vertebrae, each axial scan from the plurality of axial scans including a cross-section of the spinal cord or thecal sac adjacent to the vertebrae, and
determining the one or more parameters of the spinal cord or thecal sac includes:

determining a surface area of the cross-section of the spinal cord or thecal sac in each axial scan from the plurality of axial scans;

determining an average surface area for the spinal cord or thecal sac based on (1) the surface area of the cross-section determined for a first axial scan from the plurality of axial scans corresponding to a most superior scan of the vertebrae and (2) the surface area of the cross-section determined for a second axial scan from the plurality of axial scans corresponding to a most inferior scan of the vertebrae;

identifying one or more surface areas of the cross-sections determined for the plurality of axial scans that are lower than the remaining surface areas of the cross-sections determined for the plurality of axial scans; and

determining a compression factor based on the one or more surface areas that are lower than the remaining surface areas and the average surface area.

10. The method of claim 9, wherein determining the compression factor includes determining a ratio of (1) an average of the one or more surface areas that are lower than the remaining surface areas and (2) the average surface area.

11. The method of any one of claims 9-10, wherein determining the severity of spinal stenosis in the ROI includes determining a grade of spinal stenosis based on the compression factor,

the grade being associated with one of: no stenosis, mild stenosis, moderate stenosis, or severe stenosis.

12. An apparatus, comprising:

a memory; and

a processor operatively coupled to the memory, the processor configured to:

select a region of interest (ROI) in a three-dimensional (3D) volume of image data of a plurality of vertebra of a spine, the ROI including image data of one or more vertebra from the plurality of vertebra and tissue structures surrounding the one or more vertebra;

identify a spinal cord or thecal sac in the ROI;

determine one or more parameters associated with the spinal cord or thecal sac in the ROI; and

determine a severity of spinal stenosis in the ROI based on the one or more parameters associated with the spinal cord or thecal sac.

13. The apparatus of claim 12, wherein the processor is configured to identify the spinal cord or thecal sac by:
processing, using a convolutional neural network (CNN) trained to segment the plurality of vertebra and tissue structures surrounding the plurality of vertebra, the image data of the ROI to obtain segmentation data identifying the spinal cord or thecal sac.

14. The apparatus of claim 12, wherein the one or more parameters includes a cross-sectional surface area of the spinal cord or thecal sac.

15. The apparatus of claim 12, wherein the image data of the **ROI** includes a plurality of axial scans each including a cross-section of the spinal cord or thecal sac, and
the processor is configured to determine the one or more parameters of the spinal cord or thecal sac by determining a surface area of the cross-section of the spinal cord or thecal sac in each axial scan from the plurality of axial scans.

16. The apparatus of claim 15, wherein the processor is further configured to:
generate a plot of the surface areas determined for the cross-sections in the plurality of axial scans.

17. The apparatus of claim 12, wherein the image data of the **ROI** includes a plurality of axial scans of a vertebrae from the plurality of vertebrae, each axial scan from the plurality of axial scans including a cross-section of the spinal cord or thecal sac adjacent to the vertebrae, and
the processor is configured to determine the one or more parameters of the spinal cord or thecal sac by:

determining a surface area of the cross-section of the spinal cord or thecal sac in each axial scan from the plurality of axial scans;

determining an average surface area for the spinal cord or thecal sac based on (1) the surface area of the cross-section determined for a first axial scan from the plurality of axial scans corresponding to a most superior scan of the vertebrae and (2) the surface area of the cross-section determined for a second axial scan from the plurality of axial scans corresponding to a most inferior scan of the vertebrae;

identifying one or more surface areas of the cross-sections determined for the plurality of axial scans that are lower than the remaining surface areas of the cross-sections determined for the plurality of axial scans; and

determining a compression factor based on the one or more surface areas that are lower than the remaining surface areas and the average surface area.

18. A non-transitory processor-readable medium storing code representing instructions to be executed by a processor, the code comprising code to cause the processor to:

select a region of interest (ROI) in a three-dimensional (3D) volume of image data of a plurality of vertebra of a spine, the ROI including image data of one or more vertebra from the plurality of vertebra and tissue structures surrounding the one or more vertebra;

identify a spinal cord or thecal sac in the ROI;

determine one or more parameters associated with the spinal cord or thecal sac in the ROI; and

determine a severity of spinal stenosis in the ROI based on the one or more parameters associated with the spinal cord or thecal sac.

19. The non-transitory processor-readable medium of claim 18, wherein the code to cause the processor to identify the spinal cord or thecal sac includes code to cause the processor to:
process, using a convolutional neural network (CNN) trained to segment the plurality of vertebra and tissue structures surrounding the plurality of vertebra, the image data of the ROI to obtain segmentation data identifying the spinal cord or thecal sac.

20. The non-transitory processor-readable medium of claim 18, wherein the one or more parameters includes a cross-sectional surface area of the spinal cord or thecal sac.

21. The non-transitory processor-readable medium of claim 18, wherein the image data of the ROI includes a plurality of axial scans each including a cross-section of the spinal cord or thecal sac, and the code to cause the processor to determine the one or more parameters of the spinal cord or thecal sac includes code to cause the processing to determine a surface area of the cross-section of the spinal cord or thecal sac in each axial scan from the plurality of axial scans.

22. The non-transitory processor-readable medium of claim 21, further comprising code to cause the processor to:
generate a plot of the surface areas determined for the cross-sections in the plurality of axial scans.

23. The non-transitory processor-readable medium of claim 18, wherein the image data of the ROI includes a plurality of axial scans of a vertebrae from the plurality of vertebrae, each axial scan from the plurality of axial scans including a cross-section of the spinal cord or thecal sac adjacent to the vertebrae, and
the code to cause the processor to determine the one or more parameters of the spinal cord or thecal sac includes code to cause the processor to:

determine a surface area of the cross-section of the spinal cord or thecal sac in each axial scan from the plurality of axial scans;

determine an average surface area for the spinal cord or thecal sac based on (1) the surface area of the cross-section determined for a first axial scan from the plurality of axial scans corresponding to a most superior scan of the vertebrae and (2) the surface area of the cross-section determined for a second axial scan from the plurality of axial scans corresponding to a most inferior scan of the vertebrae;

identify one or more surface areas of the cross-sections determined for the plurality of axial scans that are lower than the remaining surface areas of the cross-sections determined for the plurality of axial scans; and

determine a compression factor based on the one or more surface areas that are lower than the remaining surface areas and the average surface area.

24. A method, comprising:

selecting a region of interest (ROI) in a three-dimensional (3D) volume of image data of a plurality of vertebra of a spine, the ROI including image data of one or more vertebra from the plurality of vertebra and tissue structures surrounding the one or more vertebra;

identifying one or more nerve roots in the ROI;

tracking the one or more nerve roots from a lateral recess to a vertebral foramen of the one or more vertebra to identify one or more regions including a discontinuity in a nerve root or a narrowed section of a nerve root; and

determining a severity or a location of spinal stenosis in the ROI based on the one or more regions.

25. The method of claim 24, wherein identifying the one or more nerve roots includes:
processing, using a convolutional neural network (CNN) trained to segment the plurality of vertebra and tissue structures surrounding the plurality of vertebra, the image data of the ROI to obtain segmentation data identifying the one or more nerve roots.

26. The method of claim 24, wherein the image data of the ROI includes a plurality of axial scans each including a cross-section of the spinal cord or thecal sac, and
tracking the one or more nerve roots includes tracking the one or more nerve roots across the plurality of axial scans.

27. An apparatus, comprising:

a memory; and

a processor operatively coupled to the memory, the process configured to:

select a region of interest (ROI) in a three-dimensional (3D) volume of image data of a plurality of vertebra of a spine, the ROI including image data of one or more vertebra from the plurality of vertebra and tissue structures surrounding the one or more vertebra;

identify one or more nerve roots in the ROI;

track the one or more nerve roots from a lateral recess to a vertebral foramen of the one or more vertebra to identify one or more regions including a discontinuity in a nerve root or a narrowed section of a nerve root; and

determine a severity or a location of spinal stenosis in the ROI based on the one or more regions.

28. A non-transitory processor-readable medium storing code representing instructions to be executed by a processor, the code comprising code to cause the processor to:

select a region of interest (ROI) in a three-dimensional (3D) volume of image data of a plurality of vertebra of a spine, the ROI including image data of one or more vertebra from the plurality of vertebra and tissue structures surrounding the one or more vertebra;

identify one or more nerve roots in the ROI;

track the one or more nerve roots from a lateral recess to a vertebral foramen of the one or more vertebra to identify one or more regions including a discontinuity in a nerve root or a narrowed section of a nerve root; and

determine a severity or a location of spinal stenosis in the ROI based on the one or more regions.

29. A method, comprising:

selecting a region of interest (ROI) in a three-dimensional (3D) volume of image data of a plurality of vertebra of a spine, the ROI including image data of one or more vertebra from the plurality of vertebra and tissue structures surrounding the one or more vertebra;

identifying an intervertebral disc in the ROI;

determining one or more parameters of an annulus and a nucleus of the intervertebral disc; and

determining a disc degeneration ratio based on the one or more parameters of the annulus and the nucleus of the intervertebral disc.

30. The method of claim 29, wherein the one or more parameters of the annulus and the nucleus includes: an average intensity of the nucleus, a real volume of the nucleus, an average intensity of the annulus, and a real volume of the annulus.

31. The method of claim 30, wherein determining the disc degeneration ratio includes calculating:

$$\frac{V_n \times I_n}{V_a \times I_a} \ ,$$

where $V_n$ is the real volume of the nucleus, $I_n$ is the average intensity of the nucleus, $V_a$ is the real volume of the annulus, and $I_a$ is the average intensity of the annulus.

32. The method of any one of claims 29-31, wherein the image data of the ROI includes a sagittal scan of the plurality of vertebra.

33. The method of any one of claims 29-32, wherein identifying the intervertebral disc includes:
processing, using a convolutional neural network (CNN) trained to segment the plurality of vertebra and tissue structures surrounding the plurality of vertebra, the image data of the ROI to obtain segmentation data identifying the intervertebral disc.

34. The method of any one of claims 29-33, further comprising:

comparing the disc degeneration ratio with a degeneration ratio associated with a patient population; and
determining a severity of disc degeneration based on the comparison.

35. An apparatus, comprising:

a memory; and
a processor operatively coupled to the memory, the process configured to:

select a region of interest (ROI) in a three-dimensional (3D) volume of image data of a plurality of vertebra of a spine, the ROI including image data of one or more vertebra from the plurality of vertebra and tissue structures surrounding the one or more vertebra;
identify an intervertebral disc in the ROI;
determine one or more parameters of an annulus and a nucleus of the intervertebral disc; and
determine a disc degeneration ratio based on the one or more parameters of the annulus and the nucleus of the intervertebral disc.

36. The apparatus of claim 35, wherein the one or more parameters of the annulus and the nucleus includes: an average intensity of the nucleus, a real volume of the nucleus, an average intensity of the annulus, and a real volume of the annulus.

37. The apparatus of claim 36, wherein the processor is configured to determine the disc degeneration ratio by calculating:

$$\frac{V_n \times I_n}{V_a \times I_a} \, ,$$

where $V_n$ is the real volume of the nucleus, $I_n$ is the average intensity of the nucleus, $V_a$ is the real volume of the annulus, and $I_a$ is the average intensity of the annulus.

38. A non-transitory processor-readable medium storing code representing instructions to be executed by a processor, the code comprising code to cause the processor to:

select a region of interest (ROI) in a three-dimensional (3D) volume of image data of a plurality of vertebra of a spine, the ROI including image data of one or more vertebra from the plurality of vertebra and tissue structures surrounding the one or more vertebra;

identify an intervertebral disc in the ROI;

determine one or more parameters of an annulus and a nucleus of the intervertebral disc; and

determine a disc degeneration ratio based on the one or more parameters of the annulus and the nucleus of the intervertebral disc.

39. The non-transitory processor-readable medium of claim 38, wherein the one or more parameters of the annulus and the nucleus includes: an average intensity of the nucleus, a real volume of the nucleus, an average intensity of the annulus, and a real volume of the annulus.

40. The non-transitory processor-readable medium of claim 39, wherein the code to cause the processor to determine the disc degeneration ratio includes code to cause the processor to calculate:

$$\frac{V_n \times I_n}{V_a \times I_a} \, ,$$

where $V_n$ is the real volume of the nucleus, $I_n$ is the average intensity of the nucleus, $V_a$ is the real volume of the annulus, and $I_a$ is the average intensity of the annulus.

## Claims

1. A method, comprising:

   selecting a region of interest (ROI) in a three-dimensional (3D) volume of image data of a plurality of vertebra of a spine, the ROI including image data of one or more vertebra from the plurality of vertebra and tissue structures surrounding the one or more vertebra;
   identifying an intervertebral disc in the ROI;
   determining one or more parameters of an annulus and a nucleus of the intervertebral disc; and
   determining a disc degeneration ratio based on the one or more parameters of the annulus and the nucleus of the intervertebral disc.

2. The method of claim 1, wherein the one or more parameters of the annulus and the nucleus includes: an average intensity of the nucleus, a real volume of the nucleus, an average intensity of the annulus, and a real volume of the annulus.

3. The method of claim 2, wherein determining the disc degeneration ratio includes calculating:

$$\frac{V_n \times I_n}{V_a \times I_a},$$

where $V_n$ is the real volume of the nucleus, $I_n$ is the average intensity of the nucleus, $V_a$ is the real volume of the annulus, and $I_a$ is the average intensity of the annulus.

4. The method of any one of claims 1-3, wherein the image data of the ROI includes a sagittal scan of the plurality of vertebra.

5. The method of any one of claims 1-4, wherein identifying the intervertebral disc includes:
   processing, using a convolutional neural network (CNN) trained to segment the plurality of vertebra and tissue structures surrounding the plurality of vertebra, the image data of the ROI to obtain segmentation data identifying the intervertebral disc.

**6.** The method of any one of claims 1-5, further comprising:

comparing the disc degeneration ratio with a degeneration ratio associated with a patient population; and
determining a severity of disc degeneration based on the comparison.

**7.** An apparatus, comprising:

a memory; and
a processor operatively coupled to the memory, the process configured to:

select a region of interest (ROI) in a three-dimensional (3D) volume of image data of a plurality of vertebra of a spine, the ROI including image data of one or more vertebra from the plurality of vertebra and tissue structures surrounding the one or more vertebra;
identify an intervertebral disc in the ROI;
determine one or more parameters of an annulus and a nucleus of the intervertebral disc; and
determine a disc degeneration ratio based on the one or more parameters of the annulus and the nucleus of the intervertebral disc.

**8.** The apparatus of claim 7, wherein the one or more parameters of the annulus and the nucleus includes: an average intensity of the nucleus, a real volume of the nucleus, an average intensity of the annulus, and a real volume of the annulus.

**9.** The apparatus of claim 8, wherein the processor is configured to determine the disc degeneration ratio by calculating:

$$\frac{V_n \times I_n}{V_a \times I_a},$$

where $V_n$ is the real volume of the nucleus, $I_n$ is the average intensity of the nucleus, $V_a$ is the real volume of the annulus, and $I_a$ is the average intensity of the annulus.

**10.** A non-transitory processor-readable medium storing code representing instructions to be executed by a processor, the code comprising code to cause the processor to:

select a region of interest (ROI) in a three-dimensional (3D) volume of image data of a plurality of vertebra of a spine, the ROI including image data of one or more vertebra from the plurality of vertebra and tissue structures surrounding the one or more vertebra;
identify an intervertebral disc in the ROI;
determine one or more parameters of an annulus and a nucleus of the intervertebral disc; and
determine a disc degeneration ratio based on the one or more parameters of the annulus and the nucleus of the intervertebral disc.

**11.** The non-transitory processor-readable medium of claim 10, wherein the one or more parameters of the annulus and the nucleus includes: an average intensity of the nucleus, a real volume of the nucleus, an average intensity of the annulus, and a real volume of the annulus.

**12.** The non-transitory processor-readable medium of claim 11, wherein the code to cause the processor to determine the disc degeneration ratio includes code to cause the processor to calculate:

$$\frac{V_n \times I_n}{V_a \times I_a},$$

where $V_n$ is the real volume of the nucleus, $I_n$ is the average intensity of the nucleus, $V_a$ is the real volume of the annulus, and $I_a$ is the average intensity of the annulus.

100

Compute Device
110

Network
150

Imaging Device(s)
160

180

Surgical Navigation
System(s)
170

FIG. 1

FIG. 2

350

360

362   370   372   380

input image

output image

information merging across encoder and decoder stages

# FIG. 3A

FIG. 3B

400

Read input data from training set
(e.g., 2D image) — 410

Combine 2D image data to 3D volume — 420

Select 3D region of interest (ROI) — 430

Train model using selected image data
(optionally, 3D ROI) — 440

Run validation set through trained
spinal analysis model — 450

Calculate performance metrics on
validation set — 460

470
Model
performance sufficient (or #
iterations reached)?

NO

YES

Store model — 480

FIG. 4A

440

Train model using selected image data

Input training set to neural network ⟍ 441

Calculate results of forward pass through a neural network ⟍ 442

Calculate loss function and gradients ⟍ 444

Upgrade neural network weights ⟍ 446

448

—NO— Epoch complete?

YES

Complete training iteration

# FIG. 4B

500

| Read patient image data | 510 |

| Perform segmentation | 520 |

| Perform level identification | 530 |

540

**Spinal Deformity Assessment**

550

| Stenosis Assessment |

560

| Disc Degeneration Assessment |

570

| Other Deformity Assessment |

| Present information associated with spinal deformity assessment | 580 |

| Generate and present virtual representation of patient anatomy with information associated with spinal deformity assessment | 582 |

| Store spinal deformity assessment | 590 |

## FIG. 5

FIG. 6A

<u>540</u>

FIG. 6B

Stenosis Assessment

550

Select region of interest (ROI)
(e.g., entire volume or sub-volume of 3D image data
including one or more vertebrae) ~ 551

Select 2D image(s) associated with ROI ~ 552

Combine 2D images into 3D ROI ~ 553

554

Analyze anatomical features

Identify spinal cord or thecal sac area based on segmentation data ~ 555a

Identify lateral and foraminal nerve roots based on segmentation data ~ 556a

Determine surface area of spinal cord or thecal sac area ~ 555b

Track nerve roots ~ 556b

Use spinal analysis model to assess stenosis based on determined surface area and/or nerve tracking data ~ 557

Associate stenosis assessment with vertebral level(s) based on level identification data ~ 558

End Stenosis Assessment

FIG. 7A

FIG. 7B

**Report**
**800**

**Patient Information**
**802**

**Spinal Deformity Diagnosis**
**804**

| First Level 806a | ---- | Diagnosis 808a |
| Second Level 806b | ---- | Diagnosis 808b |

.
.
.

**Anatomical Feature Analysis**
**810**

| First Level 806a | | Analysis 812a |
| Second Level 806b | | Analysis 812b |

.
.
.

**Virtual Representation of Patient Anatomy**
**820**

Information Associated with Spinal Deformity Diagnosis and/or Anatomical Feature Analysis
**822**

## FIG. 8

**904** L5 - S1: MILD Spinal Stenosis

| 911 | Image | Spinal Cord Surface Area |
|---|---|---|
| | 0 | 126.78 mm$^2$ |
| | 1 | 102.98 mm$^2$ |
| | 2 | 117.78 mm$^2$ |
| | 3 | 136.4 mm$^2$ |
| | 4 | 135.63 mm$^2$ |

**910**

**912** {

**FIG. 9A**   Image no. 0 from L5 - S1 level. Spinal cord surface area: 126 .78 mm$^2$

EP 4 759 230 A2

Image no. 1 from L5 - S1 level. Spinal cord surface area: 102.98 mm$^2$

## FIG. 9B

Image no. 2 from L5 - S1 level. Spinal cord surface area: 117 .78 mm²

FIG. 9C

Image no. 3 from L5 - S1 level. Spinal cord surface area: 136.4 mm$^2$

FIG. 9D

Image no. 4 from L5 - S1 level. Spinal cord surface area: 135.63 mm$^2$

FIG. 9E

FIG. 10A

Image no. 0 from L5-S1 level. Spinal cord surface area: 56.25 mm²

Image no. 0 from TH12 – L1 level. Spinal cord surface area: 288.41

FIG. 10B

EP 4 759 230 A2

Normal

Degenerated
Disc

Bulging
Disc

Herniated
Disc

Thinning
Disc

Disc Degeneration
with Osteophyte
Formation

FIG. 11

1130

```
Perform level identification
```

Select sagittal image(s) of region of interest (ROI) (e.g., L5-S1) ⟋1131

Analyze segmentation results of sagittal image(s) to identify intervertebral discs in sagittal image(s) ⟋1132

Assign level identifiers to intervertebral discs (e.g., by counting from bottom or top) ⟋1133

Separate axial images into separate volumes based on position and/or angulation associated with axial images ⟋1134

Map location of intervertebral discs (obtained from level identification of sagittal image(s)) onto axial volumes ⟋1135

More than one disc in an axial volume? 1136a — NO

Over-scanning of disc area? 1136b — NO

YES 1137a

Separate axial volume into multiple volumes

YES 1137b

Select axial scans in location of disc (and discarding others)

Associate axial volumes with sagittal disc levels and store level identification data ⟋1138

```
End level identification
```

FIG. 12

FIG. 13A

FIG. 13B

Normal _1302_

Spinal stenosis _1304_

Normal spinal canal

Degenerative changes and bone spurs

Compressed spinal nerve

FIG. 14A

EP 4 759 230 A2

<u>1312</u> **Lee classification**
Grade 0: no stenosis

<u>1314</u> **Lee classification**
Grade 1: mild stenosis

<u>1316</u> **Lee classification**
Grade 2: moderate stenosis

<u>1318</u> **Lee classification**
Grade 3: severe stenosis

# FIG. 14B

1450 **Stenosis Assessment**

Select axial volume for a thecal sac ———1451

— 1454

Analyze anatomical features

Determine thecal sac area of each 2D image in axial volume ———1454a

Calculate average thecal sac area of first and last image in axial volume ———1454b

Identify minimal thecal sac area (or set of smaller thecal sac areas) from remaining images ———1454c

Determine compression factor for thecal sac ———1454d

Compare compression factor for thecal sac with compression factor of patient population ———1456

Determine Lee grade of stenosis based on compression factor ———1457

Associate stenosis analysis with level(s) of spine ———1458

1459

NO— All axial volumes processed?

YES

End Stenosis Assessment

FIG. 15

Report
1500

Patient Information
1502

Scan Image(s)
1504

Segmentation Data
1505

Level Identification Data
1506

Stenosis Analysis
1510

Thecal Sac Surface Area Data
1511

Minimum Thecal Sac Area
1511a

Thecal Sac Area Across Scans
1511b

Population Comparison of Thecal Sac Area
1511c

Grade of Stenosis Based on Thecal Sac Area
1511d

Compression Factor Data
1512

Population Comparison
1513a

Grade of Stenosis Based on Compression
Factor
1513b

FIG. 16

1602

Patient Name: John Harris          Gender: Male                    Age: 57  DOB: 01/13/1965
Referring Physician: T. Whitman    Clinical Site: H.R. Center       Scan Date: 01/01/2022

Scan Time: 11:31:20                Scanner Model: 1.5T Signa        Software version: 1.0.0

1604a

1605a

1604b

1605b

FIG. 17A

1610

| Level | Min Thecal Sac Surface (mm^2) | Compression Factor |
|-------|-------------------------------|--------------------|
| L5-S1 | 53.85 | |
| L4-L5 | 52.08 | 48% |
| L3-L4 | 114.09 | 92% |
| L2-L3 | 134.28 | 86% |
| L1-L2 | 162.27 | 91% |
| T12-L1 | 215.22 | 85% |

FIG. 17B

EP 4 759 230 A2

1611

FIG. 17C

compression factor projected in the population range*

1612

* In the chart on the left, the range 1 means no compression or compression factor 100%, the lower range of the chart indicates the 2.5 percentile of the pathological group

| L5-S1 | L4-L5 | L3-L4 | L2-L3 | L1-L2 | Th12-L1 |

1 0.23 | 1 0.31 | 1 0.42 | 1 0.57 | 1 0.67 | 1 0.76

FIG. 17D

EP 4 759 230 A2

1702

| Grade / level | | S1-L5 | L5-L4 | L4-L3 | L3-L2 | L2-L1 | L1-TH12 |
|---|---|---|---|---|---|---|---|
| Lee 1st: Mild | | 130.00 | 165.00 | 144.00 | 173.00 | 207.00 | 230.00 |
| Lee 2nd: Moderate | | 80.00 | 119.00 | 106.00 | 140.00 | 174.00 | 198.00 |
| Lee 3rd: Severe | | 31.00 | 72.00 | 67.00 | 106.00 | 141.00 | 165.00 |

1710

FIG. 18A

1720

FIG. 18B

A. Normal
intervertebral disc

B. Degenerated
intervertebral disc

Vertebra

Annulus
fibrosus

Vertebra

Nucleus pulposus

Cartilaginous endplate

Calcification of endplate

Subchondral
plate

Blood vessel

FIG. 19

EP 4 759 230 A2

FIG. 20A

1802

Homogeneous bright
white structure?  ──YES──▶  Grade I (A)

│
NO
▼

1804

Inhomogeneous
white structure, possible  ──YES──▶  Grade II (B)
horizonal bands

│
NO
▼

1806

Clear distinction between  ──YES──▶  Grade III (C)
annulus and nucleus

│
NO
▼

1808

Collapsed disc space  ──NO──▶  Grade IV (D)

│
YES
▼

Grade V (E)

FIG. 20B

2060

FIG. 21

Report
2100

Patient Information
2102

Scan Image(s)
2104

Segmentation Data
2105

Level Identification Data
2106

Disc Degeneration Analysis
2120

Degeneration Ratio
2121

Annulus Volume
2122a

Annulus Mean Intensity
2122b

Nucleus Volume
2123a

Nucleus Mean Intensity
2123b

Population Comparison
2124

Grade of Disc Degeneration Based on Degeneration Ratio
2125

FIG. 22

2202

Patient Name: John Harris — Gender: Male — Age: 57  DOB: 01/13/1965
Referring Physician: T. Whitman — Clinical Site: H.R. Center — Scan Date: 01/01/2022

Scan Time: 11:31:20 — Scanner Model: 1.5T Signa — Software version: 1.0.0

Level L4-L5 - the axial slice with the minimum thecal sac area and most compression

2220

| Level | Disc Degeneration Ratio 2221 | Anulus Volume (CC) | Nucleus Volume (CC) | Anulus Mean Intensity | Nucleus Mean Intensity |
|---|---|---|---|---|---|
| L5-S1 | 82% | 6.3 | 4.2 | | |
| L4-L5 | 52% | 6.7 | 3.8 | | |
| L3-I4 | 72% | 7.5 | 2.9 | | |
| L2-L3 | 74% | 4.8 | 2.3 | | |
| L1-L2 | 86% | 5.8 | 3.1 | | |
| T12-L1 | 91% | 4.2 | 2.2 | | |

## FIG. 23

**REFERENCES CITED IN THE DESCRIPTION**

**Patent documents cited in the description**

- US 63312678
- US 20190105009
- US 20200151507
- US 20200410687
- US 63187777
- US 2229000 W
- US 20200327721 A
- US 63256306
- US 20190053851